# EUROPEAN PATENT SPECIFICATION

(11) **EP 4 230 626 B1**
(45) Date of publication and mention of the grant of the patent: **21.01.2026**
(21) Application number: 21880299.9
(22) Date of filing: 25.08.2021
(51) Int. Cl.: C07D 405/14, C07D 409/14, H10K 85/30, H10K 85/60, C09K 11/06, H10K 101/10

(54) **HETEROCYCLIC COMPOUND AND ORGANIC LIGHT-EMITTING DEVICE COMPRISING SAME**
HETEROCYCLISCHE VERBINDUNG UND ORGANISCHE LICHTEMITTIERENDE VORRICHTUNG DAMIT
COMPOSÉ HÉTÉROCYCLIQUE ET DISPOSITIF ÉLECTROLUMINESCENT ORGANIQUE LE COMPRENANT

(30) Priority: 16.10.2020 KR 20200134081
(43) Date of publication of application: 23.08.2023
(73) Proprietor: LT Materials Co., Ltd., Yongin-si, Gyeonggi-do 17118 (KR)
(72) Inventor: LEE, Min-Su, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/011357
(87) International publication number: WO 2022/080651

(56) References cited:
- WO-A1-2017/023021
- WO-A1-2020/157204
- WO-A1-2020/209602
- KR-A- 20190 058 748
- KR-A- 20190 127 536
- KR-A- 20200 052 239
- KR-A- 20200 110 230

## Description

### [Technical Field]

The present specification relates to a heterocyclic compound, and an organic light emitting device including the same.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a hostdopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

WO 2017/023021 A1 relates to an organic light emitting element having high efficiency, wherein an amine compound is used in the light-emitting layer and a specific compound is included in the electron density control layer.

KR 2019-0058748 A discloses an organic light-emitting compound and an organic electroluminescent device using the same, which includes a specific compound suitable for organic light-emitting applications.

### [Disclosure]

### [Technical Problem]

The present specification is directed to providing a heterocyclic compound, and an organic light emitting device including the same.

### [Technical Solution]

One aspect of the present specification provides a heterocyclic compound of the following Chemical Formula 1. 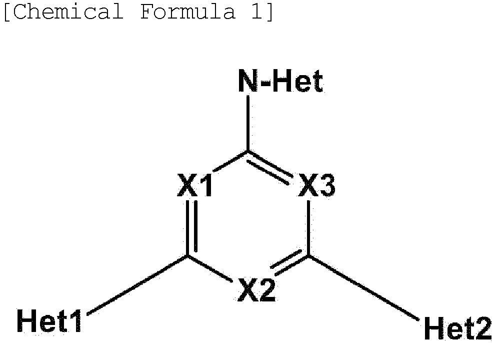 in Chemical Formula 1,
X1 to X3 are N,
N-Het is represented by the following Chemical Formula N-1 or N-2, 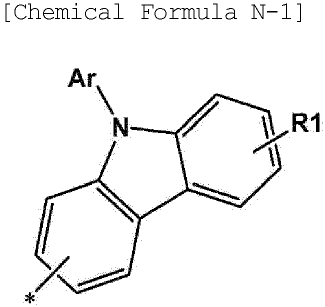 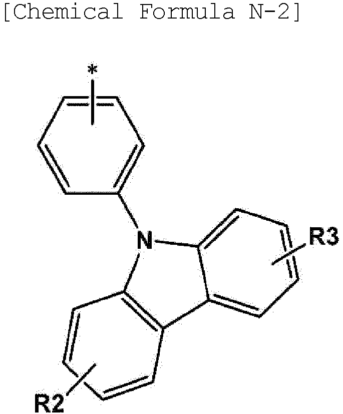
in Chemical Formulae N-1 and N-2,
R1 is hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted bicyclic or lower aryl group; a substituted or unsubstituted terphenyl group; a substituted or unsubstituted fluorenyl group; a substituted or unsubstituted tetracyclic or higher aryl group; or a substituted or unsubstituted C2 to C60 heteroaryl group,
R2 and R3 are each independently hydrogen; deuterium; a substituted or unsubstituted phenyl group; a substituted or unsubstituted biphenyl group; or a substituted or unsubstituted terphenyl group,
Ar is a substituted or unsubstituted C6 to C60 aryl group; or a substituted or unsubstituted C2 to C60 heteroaryl group, and
Het1 is represented by the following Chemical Formula H-1, and Het2 is represented by the following Chemical Formula H-2, 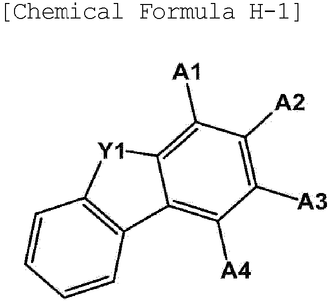 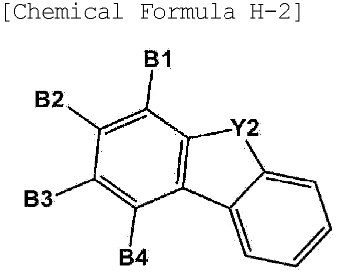
in Chemical Formulae H-1 and H-2,
Y1 and Y2 are each independently O or S,
A1 to A4 and B1 to B4 each independently bond to Chemical Formula 1, or are hydrogen; or deuterium, and
Chemical Formula 1 bonds to any one of A1 to A4 of Chemical Formula H-1 and any one of B1 to B4 of Chemical Formula H-2, which is represented by Am-Bn, m and n are each 1, 2, 3 or 4, and m and n are different.

Another aspect of the present specification provides an organic light emitting device including a first electrode; a second electrode provided opposite to the first electrode; and an organic material layer provided between the first electrode and the second electrode, wherein the organic material layer includes the heterocyclic compound of Chemical Formula 1.

Another aspect of the present specification provides a composition for forming an organic material layer, the composition including the heterocyclic compound of Chemical Formula 1; and a compound of the following Chemical Formula 3. 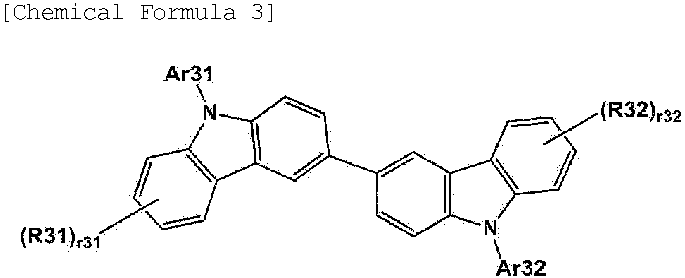 in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a substituted or unsubstituted C2 to C60 heteroaryl group,
r31 is an integer of 0 to 4, and when 2 or greater, R31s are the same as or different from each other, and
r32 is an integer of 0 to 4, and when 2 or greater, R32s are the same as or different from each other.

Further embodiments are disclosed in the dependent claims.

### [Advantageous Effects]

A compound described in the present specification can be used as a material of an organic material layer of an organic light emitting device. The compound is capable of acting as a hole injection material, a hole transfer material, a light emitting material, an electron transfer material, an electron injection material, a charge generation material or the like. Particularly, the compound can be used as a material of a light emitting layer of an organic light emitting device.

Using the heterocyclic compound of Chemical Formula 1 and the compound of Chemical Formula 3 together as a material of a light emitting layer of an organic light emitting device is capable of lowering a driving voltage, enhancing light emission efficiency and enhancing lifetime properties in the device.

### [Description of Drawings]

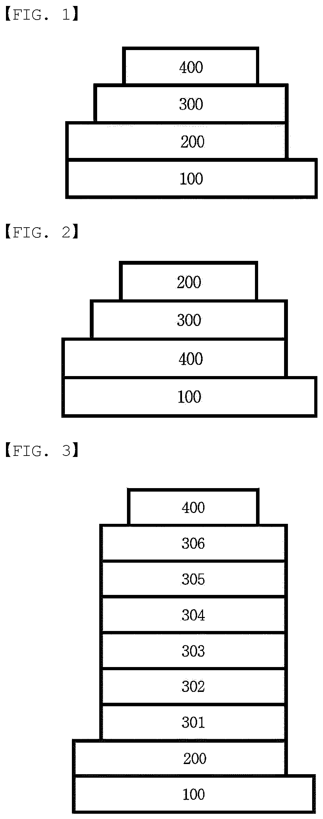

FIG. 1 to FIG. 3 are diagrams each illustrating a lamination structure of an organic light emitting device according to one aspect of the present specification.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

A term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; halogen; a cyano group; a C1 to C60 alkyl group; a C2 to C60 alkenyl group; a C2 to C60 alkynyl group; a C3 to C60 cycloalkyl group; a C2 to C60 heterocycloalkyl group; a C6 to C60 aryl group; a C2 to C60 heteroaryl group; a silyl group; a phosphine oxide group; and an amine group, or being unsubstituted, or being substituted with a substituent linking two or more substituents selected from among the substituents illustrated above, or being unsubstituted.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one aspect of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one aspect of the present application, in a "case of a substituent being not indicated in a chemical formula or compound structure", hydrogen and deuterium may be mixed in compounds when deuterium is not explicitly excluded such as a deuterium content being 0%, a hydrogen content being 100% or substituents being all hydrogen.

In one aspect of the present application, deuterium is one of isotopes of hydrogen, is an element having deuteron formed with one proton and one neutron as a nucleus, and may be expressed as hydrogen-2, and the elemental symbol may also be written as D or ²H.

In one aspect of the present application, an isotope means an atom with the same atomic number (Z) but with a different mass number (A), and may also be interpreted as an element with the same number of protons but with a different number of neutrons.

In one aspect of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 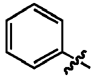 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

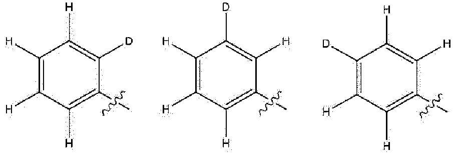

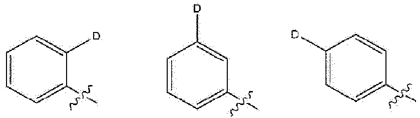

In addition, in one aspect of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched, and may be further substituted with other substituents. The number of carbon atoms of the alkynyl group may be from 2 to 60, specifically from 2 to 40 and more specifically from 2 to 20.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. When the aryl group is dicyclic or higher, the number of carbon atoms may be from 8 to 60, from 8 to 40 or from 8 to 30. Specific examples of the aryl group may include a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the terphenyl group may be selected from among the following structures.

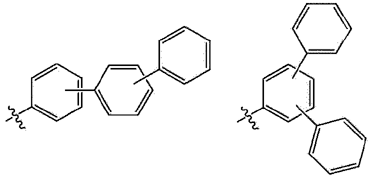

In the present specification, the fluorenyl group may be substituted, and adjacent substituents may bond to each other to form a ring.

When the fluorenyl group is substituted, 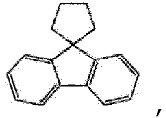 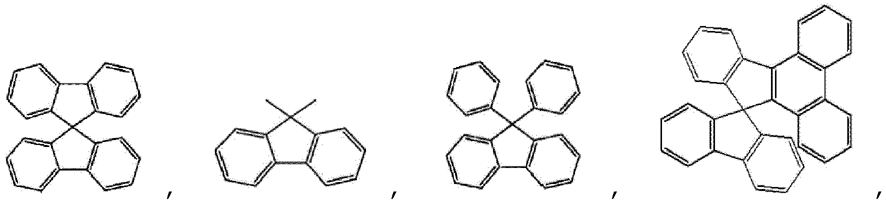 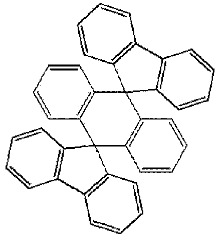 and the like may be included, however, the structure is not limited thereto.

In the present specification, the heteroaryl group includes O, S, SO₂, Se, N or Si as a heteroatom, includes monocyclic or polycyclic, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. When the heteroaryl group is dicyclic or higher, the number of carbon atoms may be from 4 to 60, from 4 to 40 or from 4 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a benzofuro[2,3-d]pyrimidyl group; a benzothieno[2,3-d]pyrimidyl group; a benzofuro[2,3-a]carbazolyl group, a benzothieno[2,3-a]carbazolyl group, a 1,3-dihydroindolo[2,3-a]carbazolyl group, a benzofuro[3,2-a]carbazolyl group, a benzothieno[3,2-a]carbazolyl group, a 1,3-dihydroindolo[3,2-a]carbazolyl group, a benzofuro[2,3-b]carbazolyl group, a benzothieno[2,3-b]carbazolyl group, a 1,3-dihydroindolo[2,3-b]carbazolyl group, a benzofuro[3,2-b]carbazolyl group, a benzothieno[3,2-b]carbazolyl group, a 1,3-dihydroindolo[3,2-b]carbazolyl group, a benzofuro[2,3-c]carbazolyl group, a benzothieno[2,3-c]carbazolyl group, a 1,3-dihydroindolo[2,3-c]carbazolyl group, a benzofuro[3,2-c]carbazolyl group, a benzothieno[3,2-c]carbazolyl group, a 1,3-dihydroindolo[3,2-c]carbazolyl group, a 1,3-dihydroindeno[2,1-b]carbazolyl group, a 5,11-dihydroindeno[1,2-b]carbazolyl group, a 5,12-dihydroindeno[1,2-c]carbazolyl group, a 5,8-dihydroindeno[2,1-c]carbazolyl group, a 7,12-dihydroindeno[1,2-a]carbazolyl group, a 11,12-dihydroindeno[2,1-a]carbazolyl group and the like, but are not limited thereto.

In the present specification, the silyl group is a substituent including Si, having the Si atom directly linked as a radical, and is represented by -Si(R101) (R102) (R103). R101 to R103 are the same as or different from each other, and may be each independently a substituent formed with at least one of hydrogen; deuterium; a halogen group; an alkyl group; an alkenyl group; an alkoxy group; a cycloalkyl group; an aryl group; and a heteroaryl group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the phosphine oxide group is represented by -P(=O) (R104) (R105), and R104 and R105 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. Specifically, the phosphine oxide group may be substituted with an alkyl group or an aryl group, and as the alkyl group and the aryl group, the examples described above may be applied. Examples of the phosphine oxide group may include a dimethylphosphine oxide group, a diphenylphosphine oxide group, a dinaphthylphosphine oxide group and the like, but are not limited thereto.

In the present specification, the amine group is represented by -N(R106) (R107), and R106 and R107 are the same as or different from each other and may be each independently a substituent formed with at least one of hydrogen; deuterium; a halogen group; an alkyl group; an alkenyl group; an alkoxy group; a cycloalkyl group; an aryl group; and a heteroaryl group. The amine group may be selected from the group consisting of -NH₂; a monoalkylamine group; a monoarylamine group; a monoheteroarylamine group; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the examples of the aryl group described above may be applied to the arylene group except that the arylene group is a divalent group.

In the present specification, the examples of the heteroaryl group described above may be applied to the heteroarylene group except that the heteroarylene group is a divalent group.

In the present specification, X1 to X3 are N.

In one aspect of the present specification, N-Het of Chemical Formula 1 may be represented by the following Chemical Formula N-1. 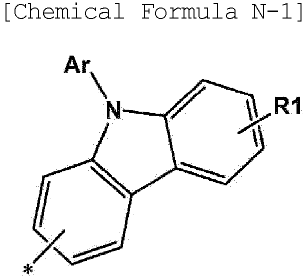 in Chemical Formula N-1,
R1 is hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted bicyclic or lower aryl group; a substituted or unsubstituted terphenyl group; a substituted or unsubstituted fluorenyl group; a substituted or unsubstituted tetracyclic or higher aryl group; or a substituted or unsubstituted C2 to C60 heteroaryl group, and
Ar is a substituted or unsubstituted C6 to C60 aryl group; or a substituted or unsubstituted C2 to C60 heteroaryl group.

In one aspect of the present specification, R1 is hydrogen; a substituted or unsubstituted bicyclic or lower aryl group; a substituted or unsubstituted terphenyl group; a substituted or unsubstituted fluorenyl group; a substituted or unsubstituted tetracyclic or higher aryl group; or a substituted or unsubstituted C2 to C60 heteroaryl group.

In one aspect of the present specification, R1 is hydrogen; a substituted or unsubstituted bicyclic or lower aryl group; a substituted or unsubstituted terphenyl group; a substituted or unsubstituted fluorenyl group; or a substituted or unsubstituted tetracyclic or higher aryl group.

In one aspect of the present specification, R1 may be hydrogen; or a substituted or unsubstituted bicyclic or lower aryl group.

In one aspect of the present specification, R1 may be hydrogen; a substituted or unsubstituted phenyl group; or a substituted or unsubstituted biphenyl group.

In one aspect of the present specification, R1 may be hydrogen; a phenyl group; or a biphenyl group.

In one aspect of the present specification, Ar is a substituted or unsubstituted C6 to C40 aryl group; or a substituted or unsubstituted C2 to C40 heteroaryl group.

In one aspect of the present specification, Ar is a substituted or unsubstituted C6 to C60 aryl group.

In one aspect of the present specification, Ar is a substituted or unsubstituted C6 to C40 aryl group.

In one aspect of the present specification, Ar is a substituted or unsubstituted C6 to C20 aryl group.

In one aspect of the present specification, Ar may be a substituted or unsubstituted phenyl group; a substituted or unsubstituted biphenyl group; a substituted or unsubstituted terphenyl group; a substituted or unsubstituted naphthyl group; a substituted or unsubstituted phenanthrenyl group; a substituted or unsubstituted triphenylenyl group; or a substituted or unsubstituted pyrenyl group.

In one aspect of the present specification, Ar may be an unsubstituted phenyl group; a phenyl group substituted with deuterium; a phenyl group substituted with a C1 to C10 alkyl group; a phenyl group substituted with a C1 to C10 alkyl group substituted with deuterium; a biphenyl group unsubstituted or substituted with deuterium; a terphenyl group; a naphthyl group; a phenanthrenyl group; a triphenylenyl group; or a pyrenyl group.

In one aspect of the present specification, Ar may be a phenyl group unsubstituted or substituted with deuterium or t-Bu (tert-butyl); a phenyl group substituted with a C1 to C5 alkyl group substituted with deuterium; a biphenyl group unsubstituted or substituted with deuterium; a terphenyl group; a naphthyl group; a phenanthrenyl group; a triphenylenyl group; or a pyrenyl group.

When Chemical Formula N-1 includes a linker instead of directly bonding to Chemical Formula 1, the band gap decreases as the conjugation length increases, which may cause a problem of reducing efficiency.

In one aspect of the present specification, N-Het of Chemical Formula 1 may be represented by the following Chemical Formula N-2. 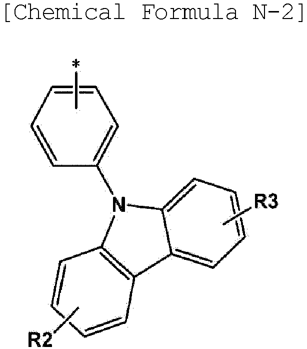 in Chemical Formula N-2,
In the present specification, R2 and R3 are each independently hydrogen; deuterium; a substituted or unsubstituted phenyl group; a substituted or unsubstituted biphenyl group; or a substituted or unsubstituted terphenyl group.

In one aspect of the present specification, R2 is hydrogen; deuterium; a phenyl group unsubstituted or substituted with deuterium or a C1 to C10 alkyl group; a biphenyl group; or a terphenyl group.

In one aspect of the present specification, R3 is hydrogen; deuterium; or a substituted or unsubstituted phenyl group.

In one aspect of the present specification, R3 may be hydrogen; deuterium; or a phenyl group unsubstituted or substituted with deuterium or a C1 to C10 alkyl group.

When nitrogen of the carbazole directly bonds to Chemical Formula 1 instead of bonding as the structure of Chemical Formula N-2, the band gap decreases by enhancing charge transfer between the donor and the acceptor, and a weak BDE (bond dissociation energy) value is obtained, which cause a problem of reducing the lifetime.

In one aspect of the present specification, Het1 of Chemical Formula 1 is represented by the following Chemical Formula H-1, and Het2 of Chemical Formula 1 is represented by the following Chemical Formula H-2. 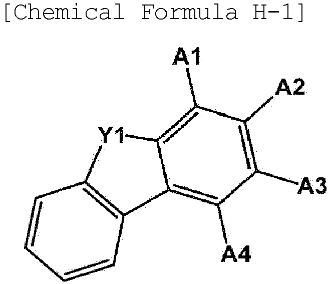 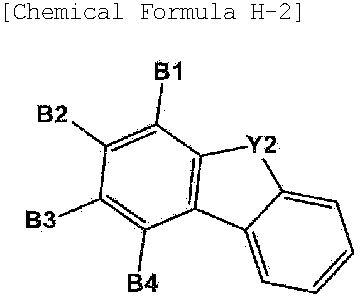 in Chemical Formulae H-1 and H-2,
Y1 and Y2 are each independently O or S,
A1 to A4 and B1 to B4 each independently bond to Chemical Formula 1, or are hydrogen; or deuterium,
Chemical Formula 1 bonds to any one of A1 to A4 of Chemical Formula H-1 and any one of B1 to B4 of Chemical Formula H-2, which is represented by Am-Bn, m and n are each 1, 2, 3 or 4, and m and n are different.

In one aspect of the present specification, Y1 and Y2 may be O.

In one aspect of the present specification, Y1 and Y2 may be S.

In one aspect of the present specification, any one of Y1 and Y2 is O, and the other one may be S.

In one aspect of the present specification, A1 to A4 of Chemical Formula H-1 and B1 to B4 of Chemical Formula H-2 each independently bond to Chemical Formula 1, or are hydrogen; or deuterium.

In one aspect of the present specification, the rest of A1 to A4 and B1 to B4 not bonding to Chemical Formula 1 are hydrogen; or deuterium.

In one aspect of the present specification, Chemical Formula 1 bonds to any one of A1 to A4 of Chemical Formula H-1 and any one of B1 to B4 of Chemical Formula H-2, which is represented by Am-Bn, and m and n are different.

In one aspect of the present specification, when Chemical Formula 1 bonds to A1 of Chemical Formula H-1 and bonds to B2 of Chemical Formula H-2, it is represented as A1-B2.

In one aspect of the present specification, when Chemical Formula 1 bonds to A1 of Chemical Formula H-1, Chemical Formula 1 may bond to B2, B3 or B4 of Chemical Formula H-2, which may be represented respectively by A1-B2, A1-B3 or A1-B4. In other words, when Chemical Formula 1 bonds to A1 of Chemical Formula H-1, Chemical Formula 1 is not able to bond to B1 of Chemical Formula H-2.

In one aspect of the present specification, when Chemical Formula 1 bonds to A2 of Chemical Formula H-1, Chemical Formula 1 may bond to B1, B3 or B4 of Chemical Formula H-2, which may be respectively represented by A2-B1, A2-B3 or A2-B4. In other words, when Chemical Formula 1 bonds to A2 of Chemical Formula H-1, Chemical Formula 1 is not able to bond to B2 of Chemical Formula H-2.

In one aspect of the present specification, when Chemical Formula 1 bonds to A3 of Chemical Formula H-1, Chemical Formula 1 may bond to B1, B2 or B4 of Chemical Formula H-2, which may be respectively represented by A3-B1, A3-B2 or A3-B4. In other words, when Chemical Formula 1 bonds to A3 of Chemical Formula H-1, Chemical Formula 1 is not able to bond to B3 of Chemical Formula H-2.

In one aspect of the present specification, when Chemical Formula 1 bonds to A4 of Chemical Formula H-1, Chemical Formula 1 may bond to B1, B2 or B3 of Chemical Formula H-2, which may be respectively represented by A4-B1, A4-B2 or A4-B3. In other words, when Chemical Formula 1 bonds to A4 of Chemical Formula H-1, Chemical Formula 1 is not able to bond to B4 of Chemical Formula H-2.

When dibenzofuran or dibenzothiophene bonds to Chemical Formula 1 at the same position, the LUMO orbital is widely delocalized reducing electron mobility, and efficiency may be reduced. In addition, deposition may occur in a flat molecular structure, and crystallinity is obtained therefrom, which leads to a disadvantage of being vulnerable in terms of a lifetime.

In one aspect of the present specification, Chemical Formula 1 may be represented by any one of the following Chemical Formulae 2-1 to 2-4. 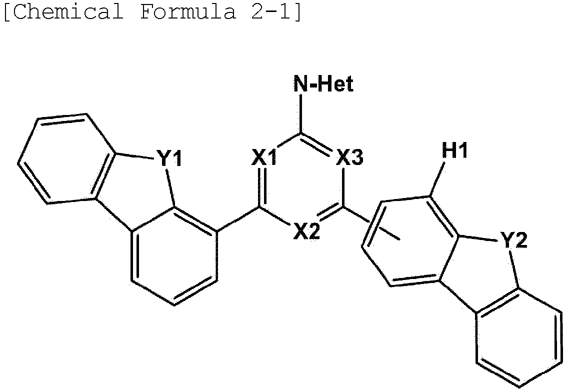 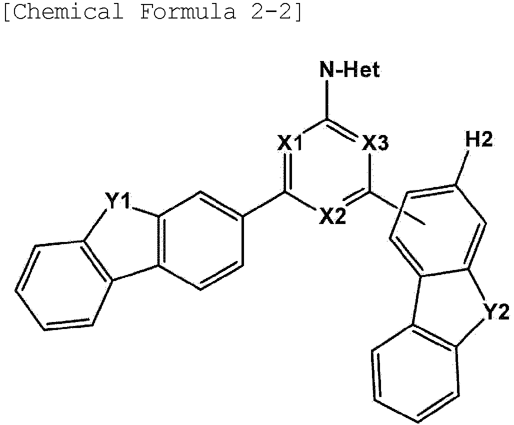 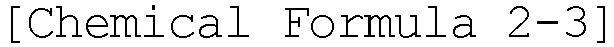 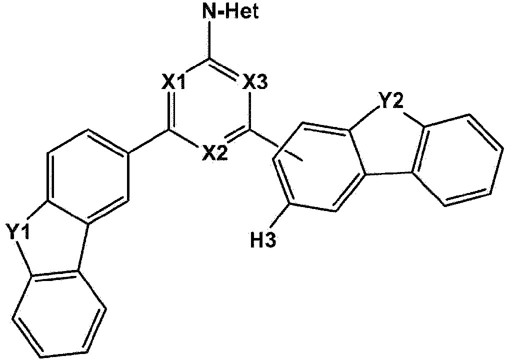 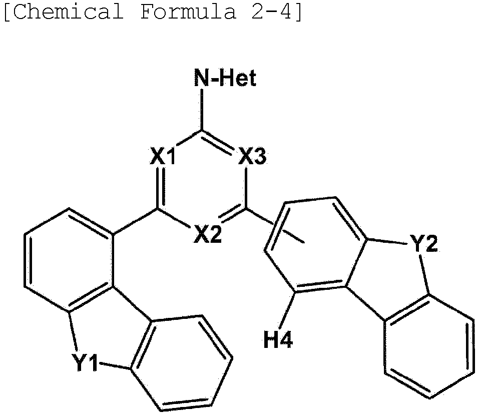 in Chemical Formulae 2-1 to 2-4,
H1 to H4 are each independently hydrogen; or deuterium, and
the rest of the substituents have the same definitions as in Chemical Formula 1.

In one aspect of the present specification, Chemical Formula 1 may be represented by any one of the following compounds, but is not limited thereto.

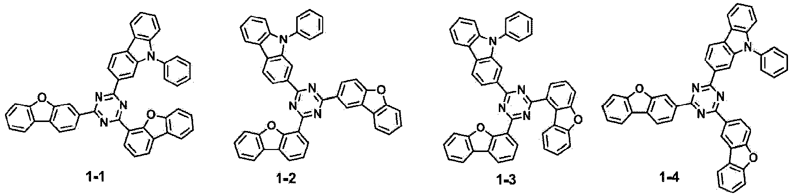

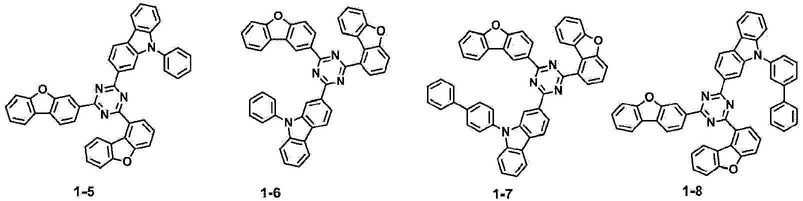

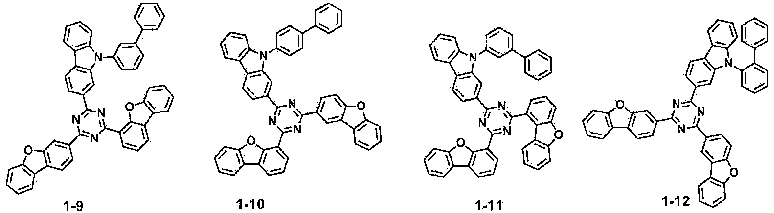

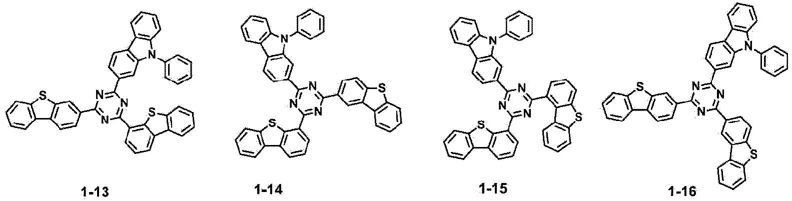

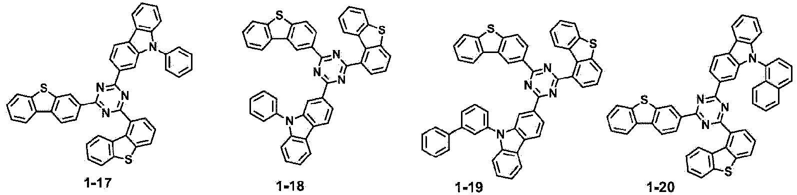

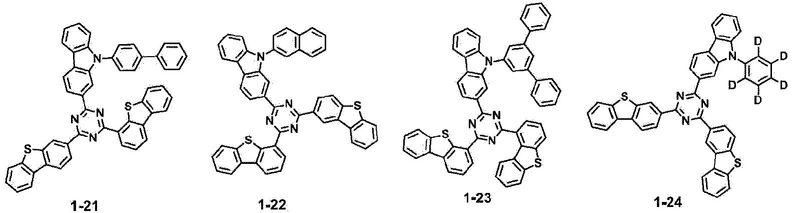

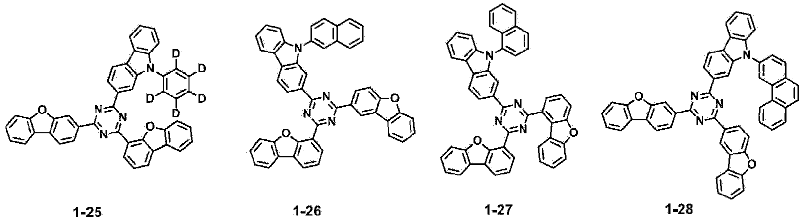

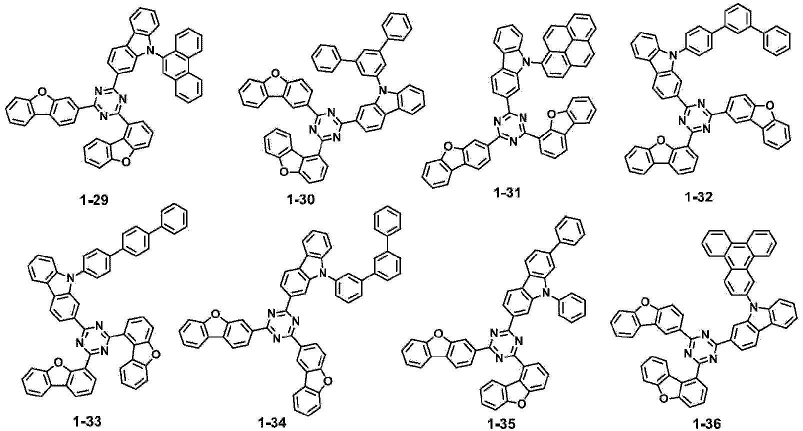

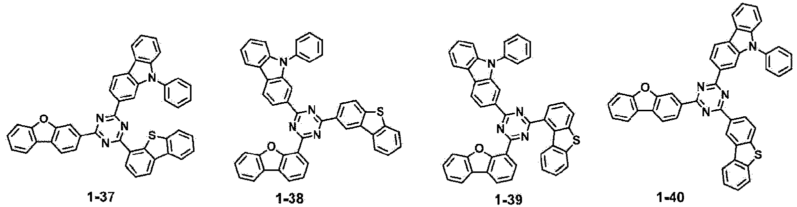

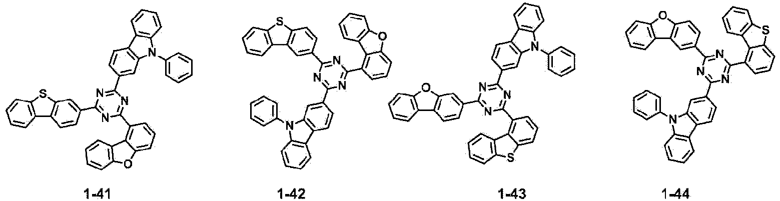

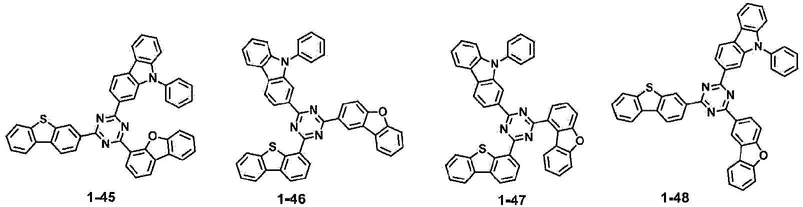

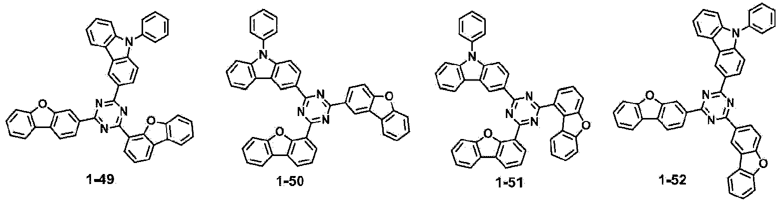

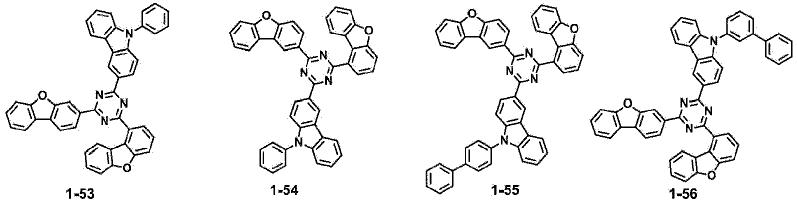

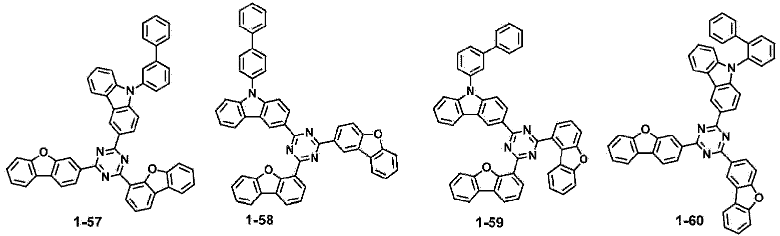

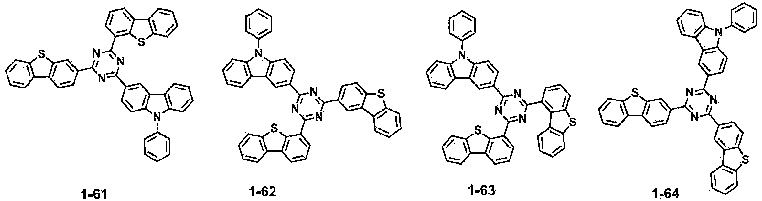

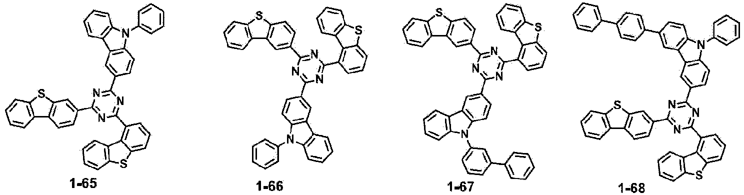

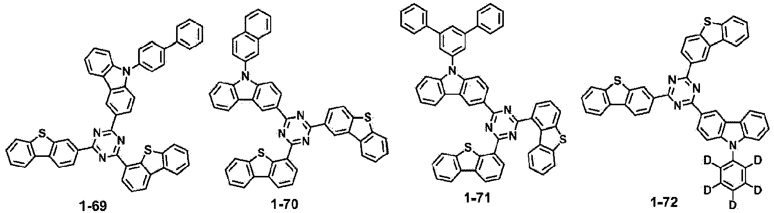

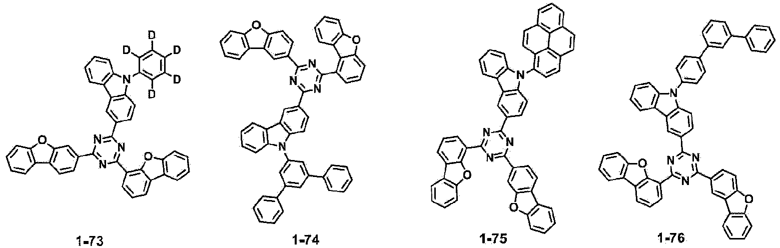

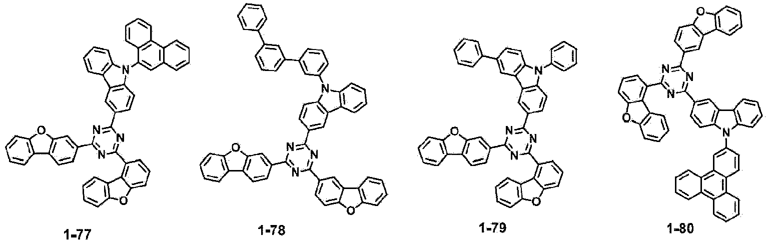

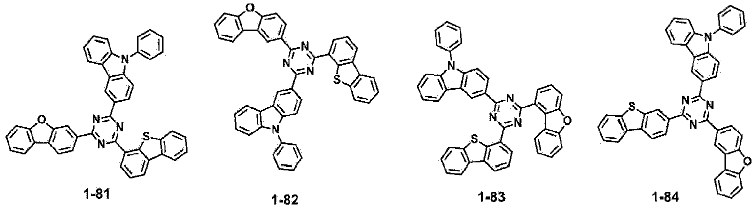

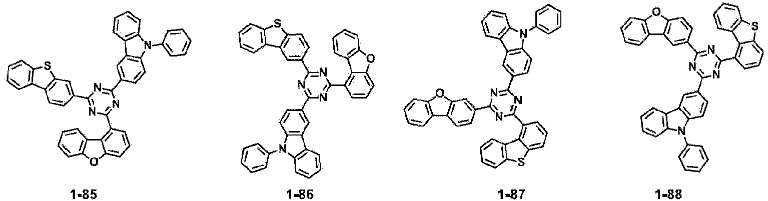

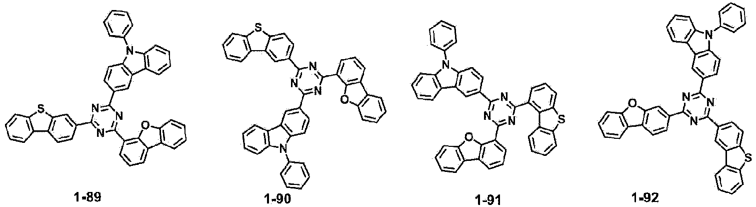

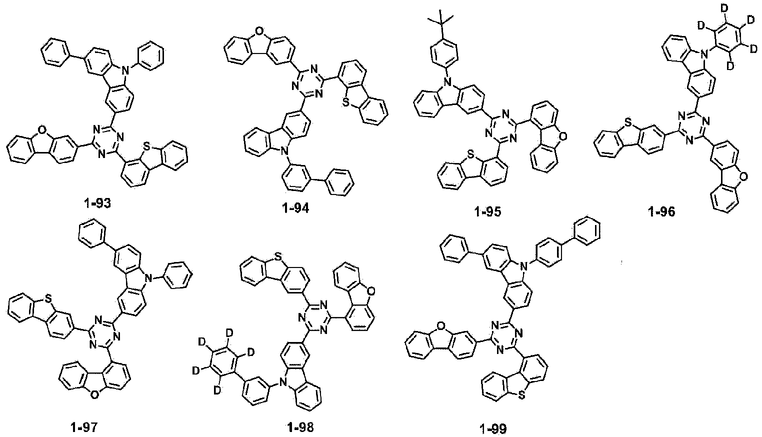

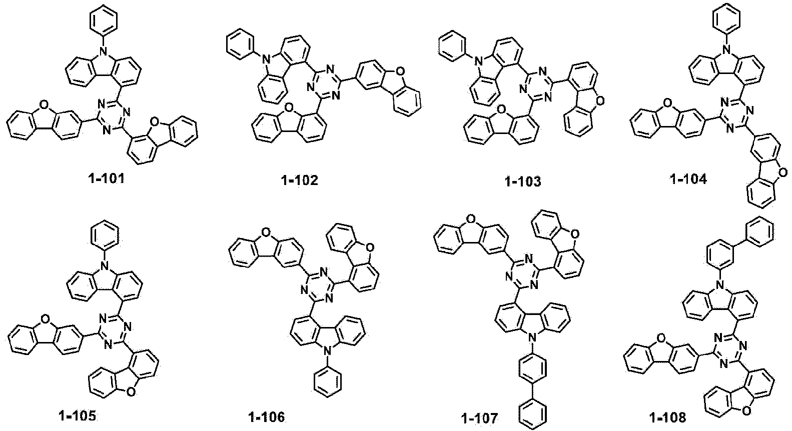

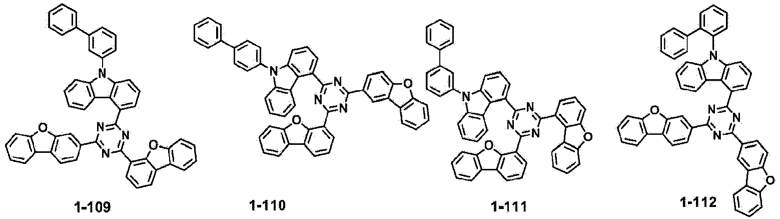

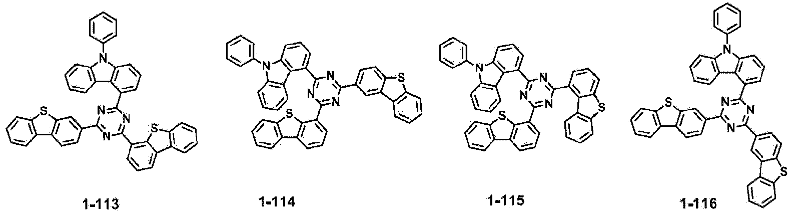

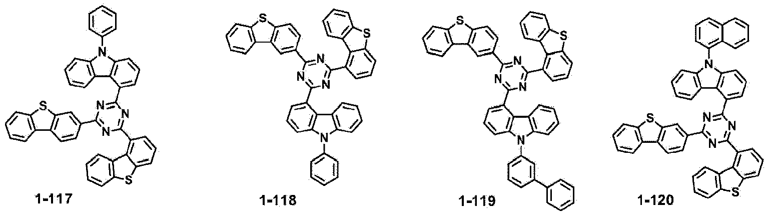

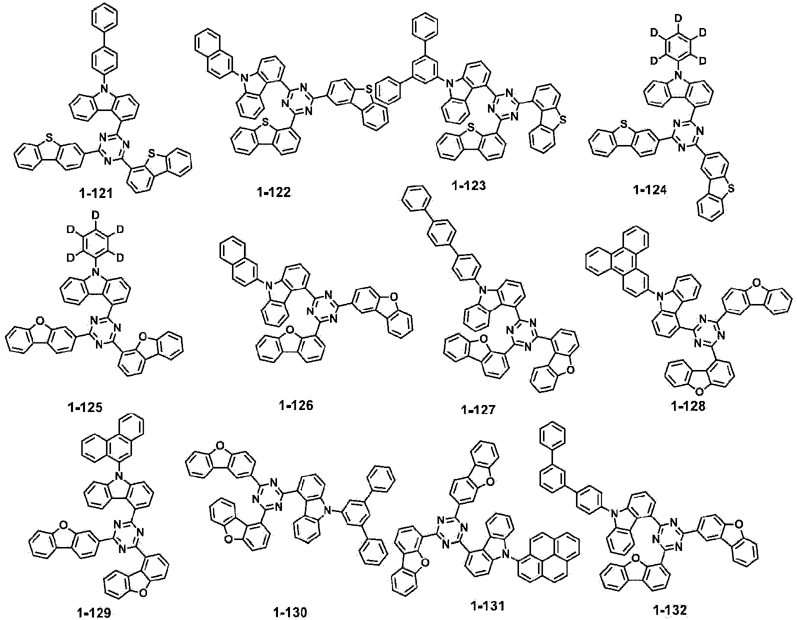

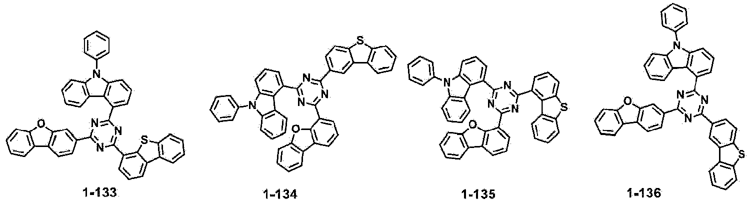

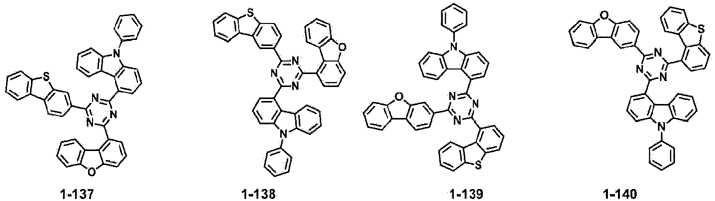

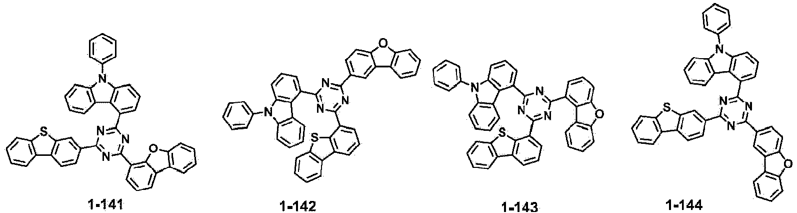

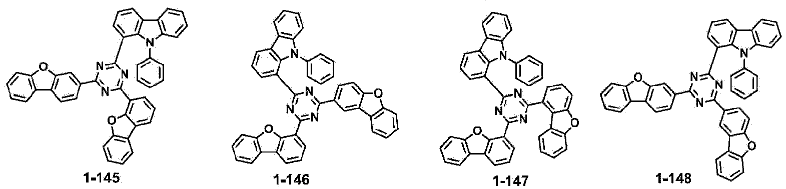

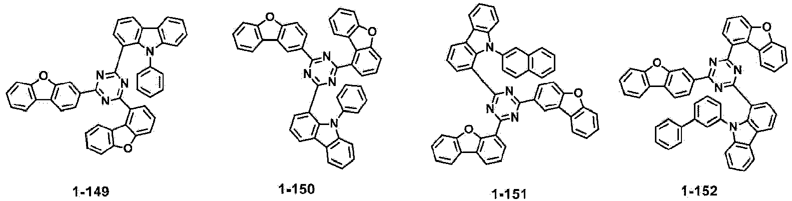

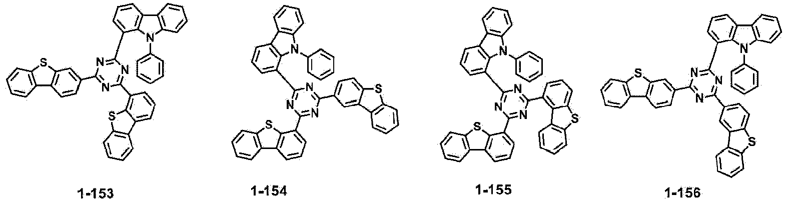

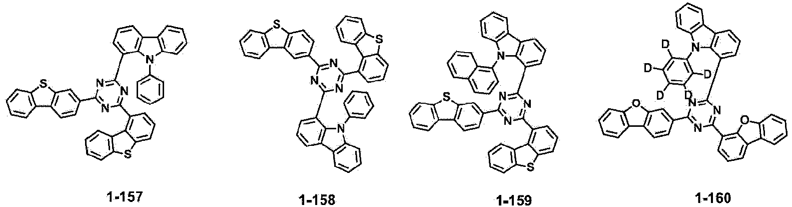

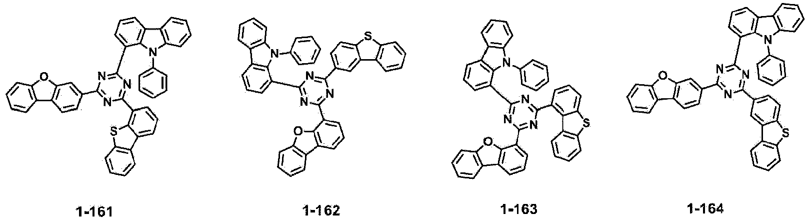

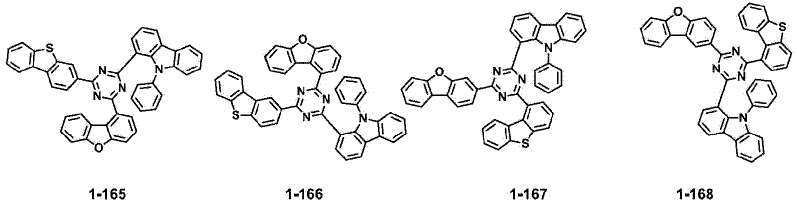

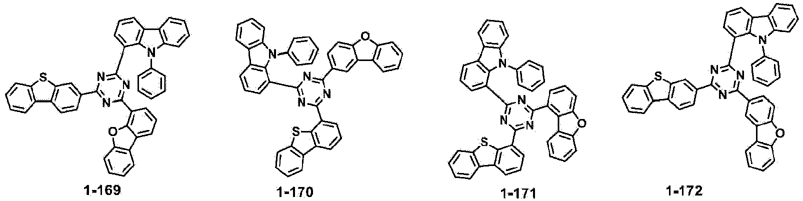

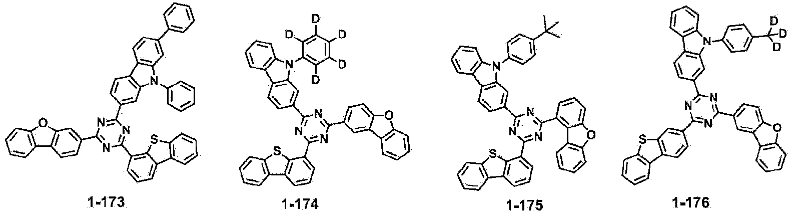

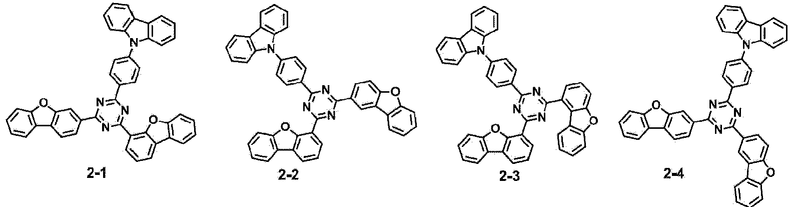

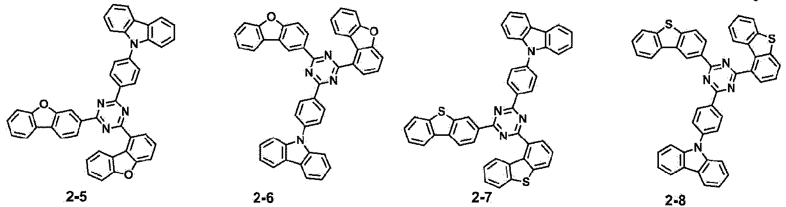

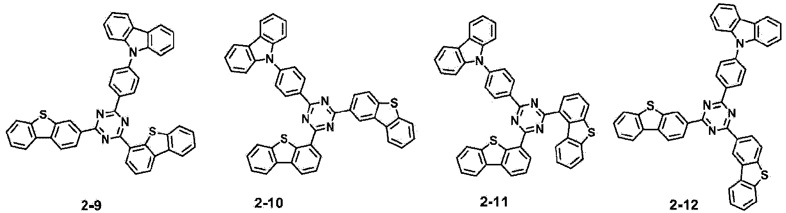

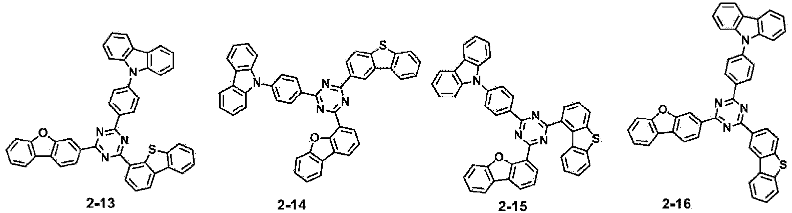

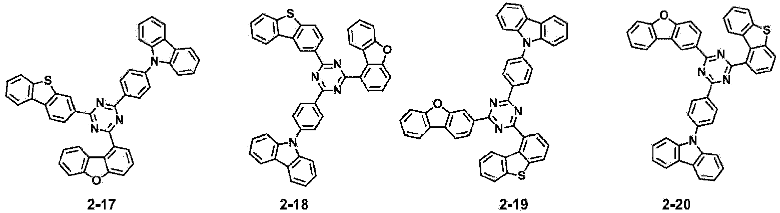

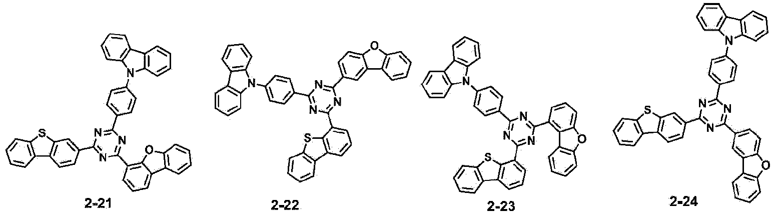

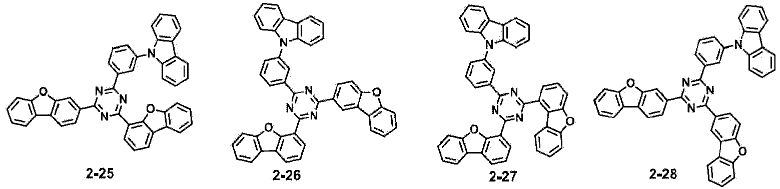

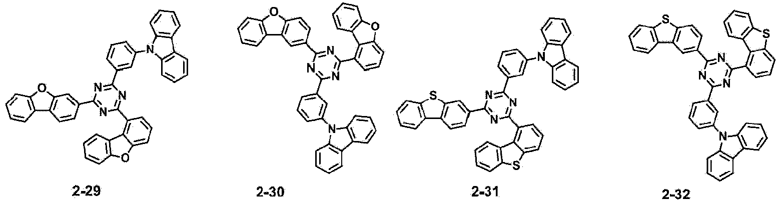

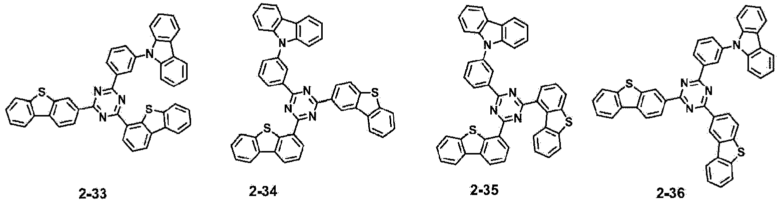

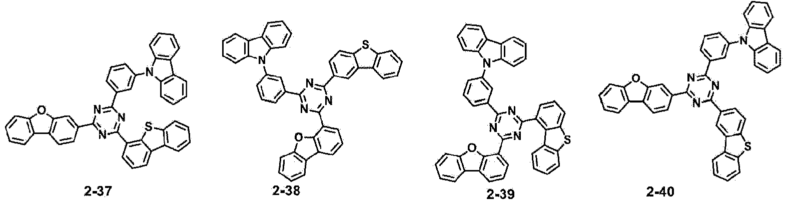

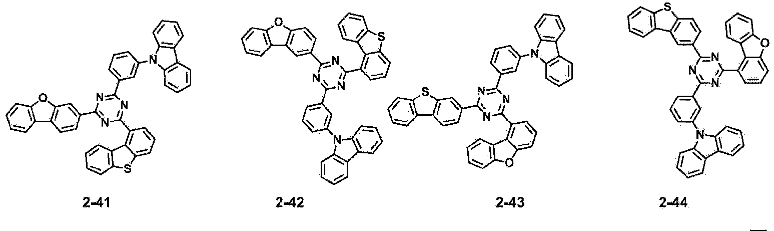

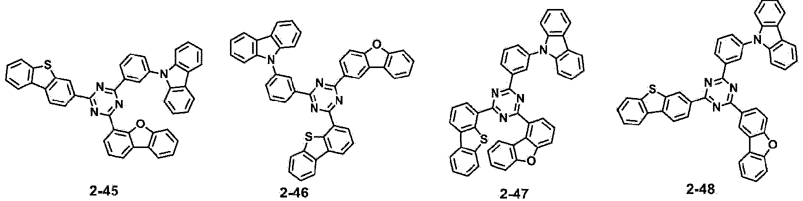

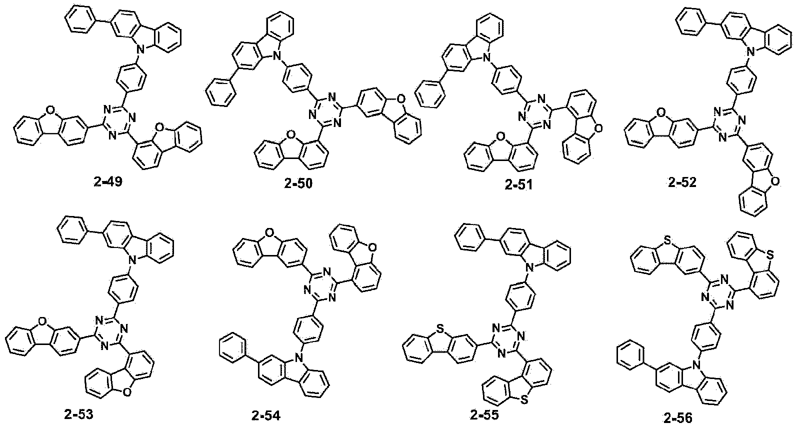

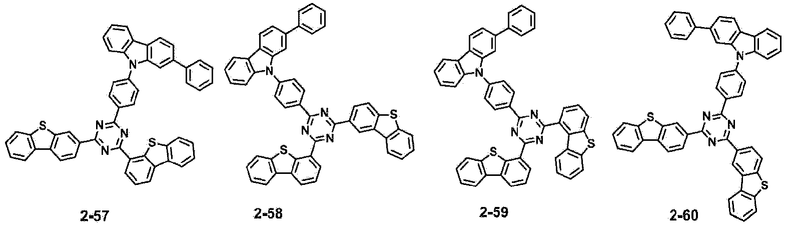

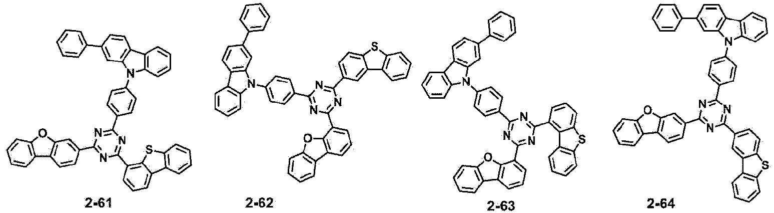

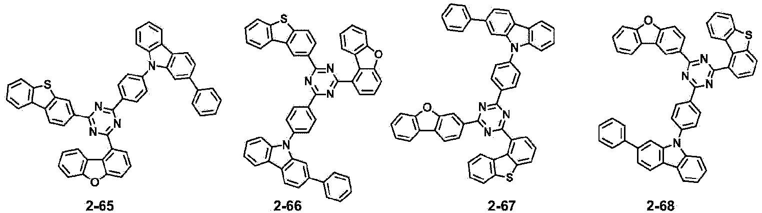

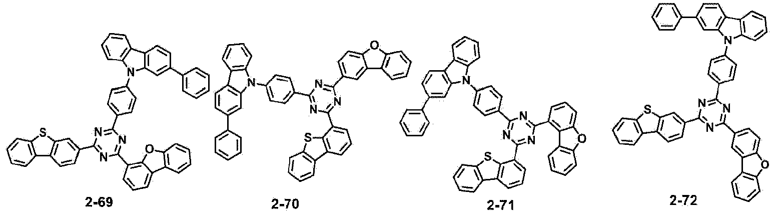

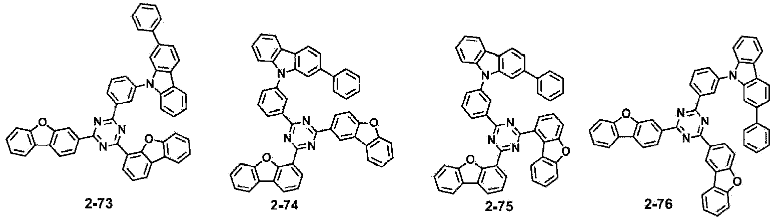

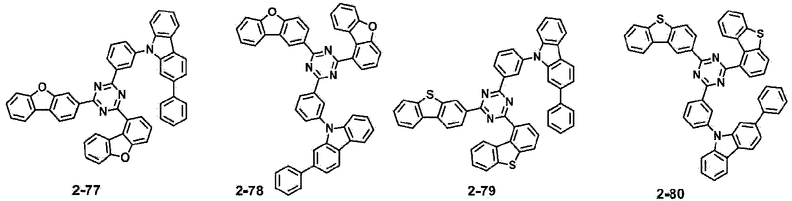

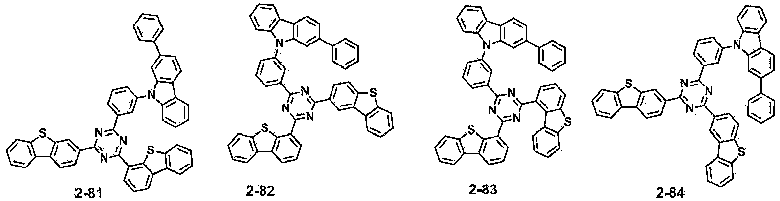

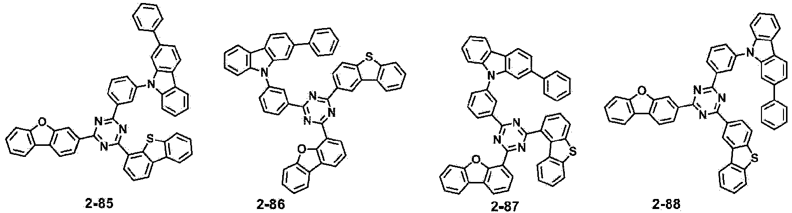

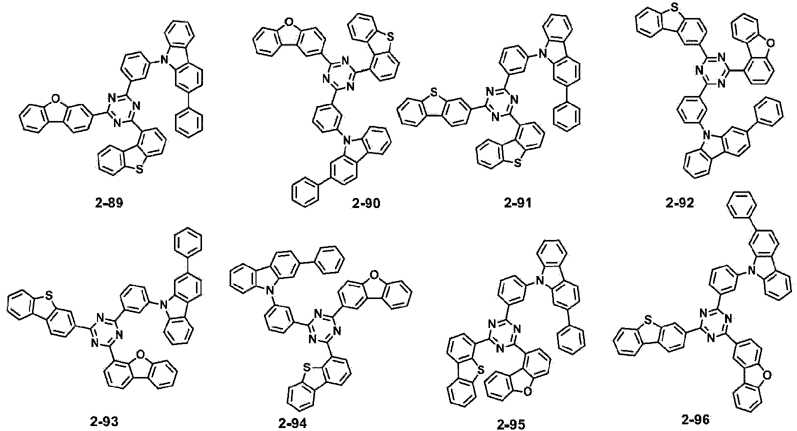

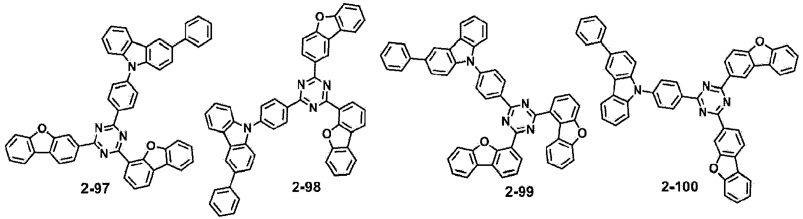

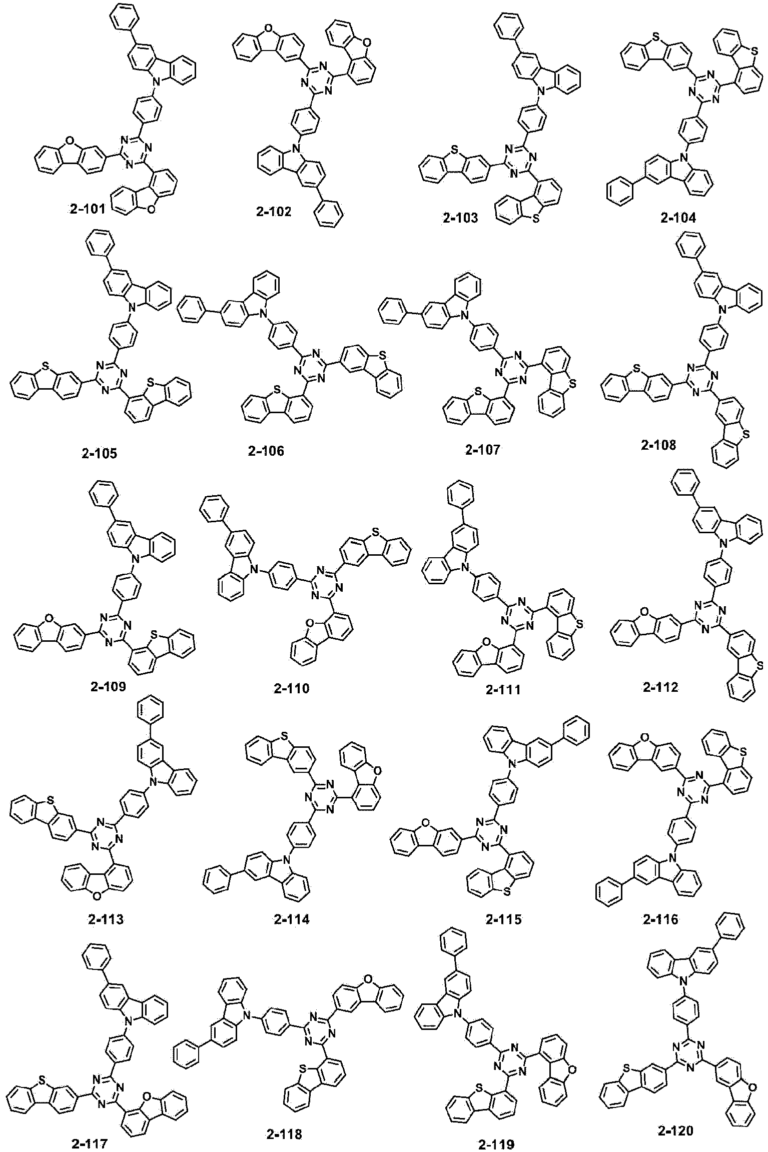

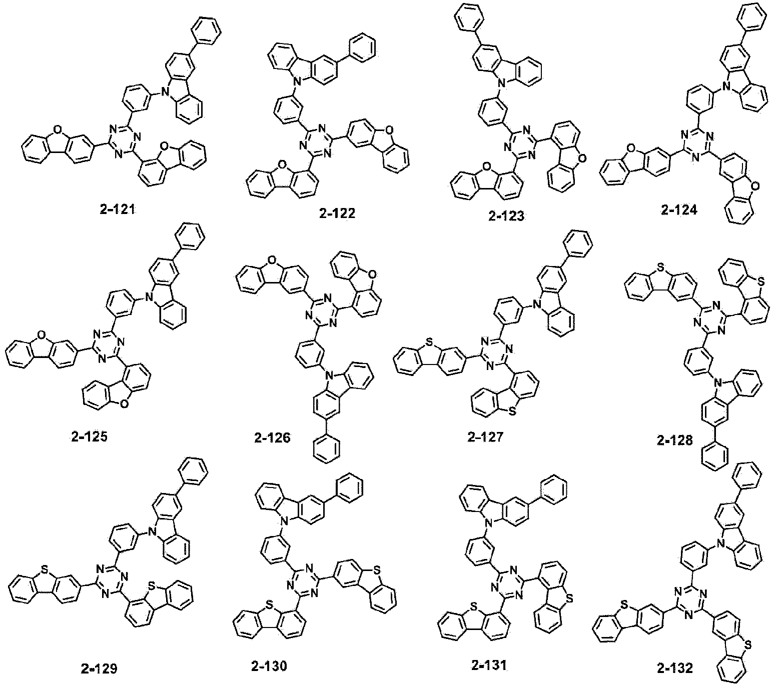

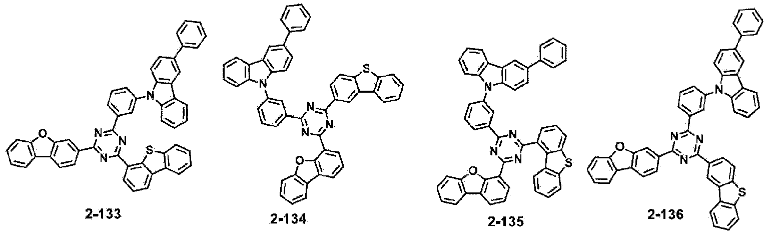

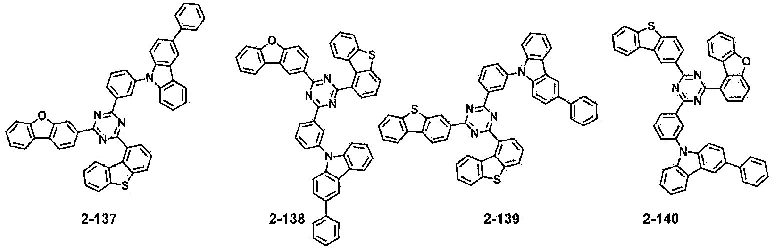

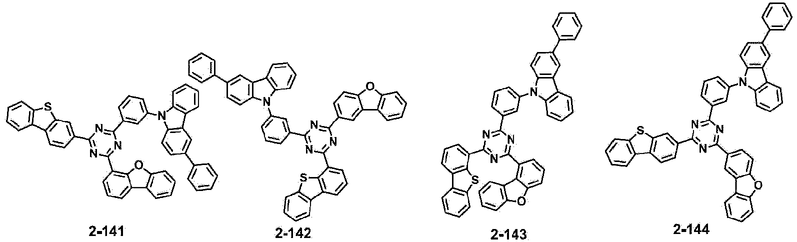

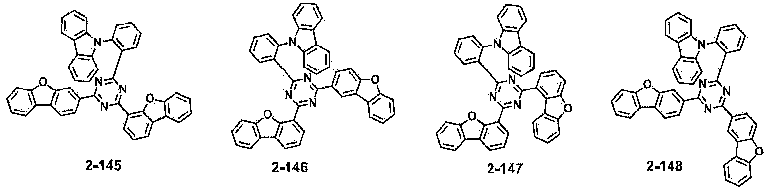

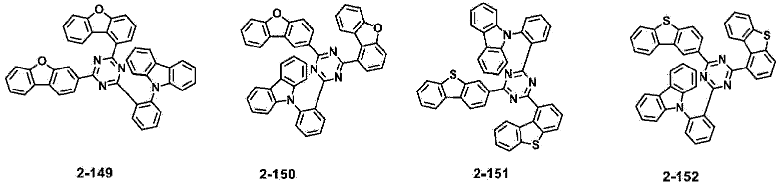

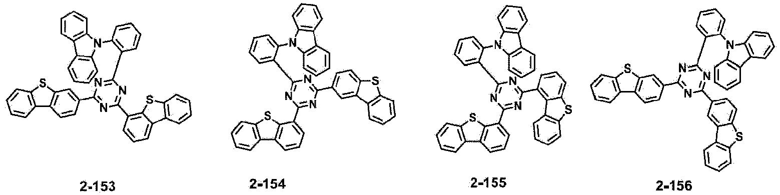

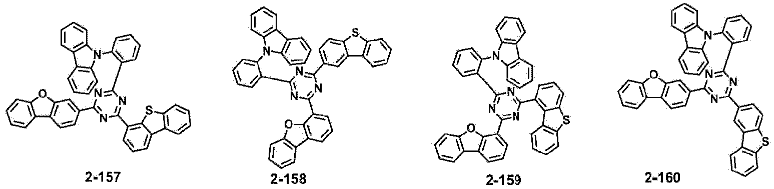

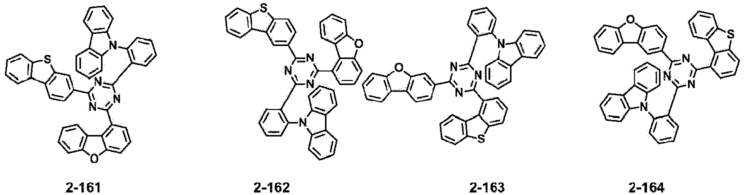

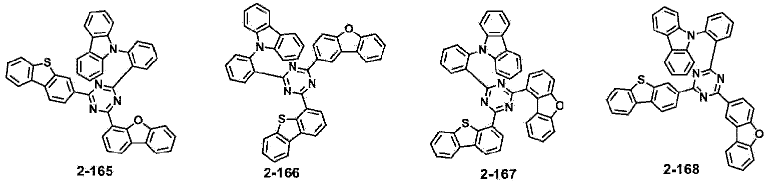

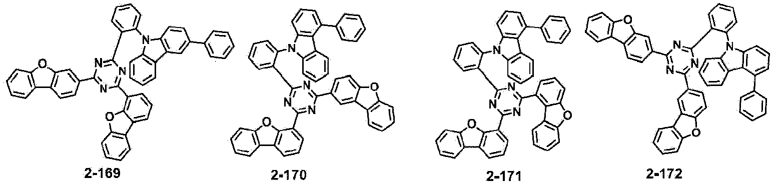

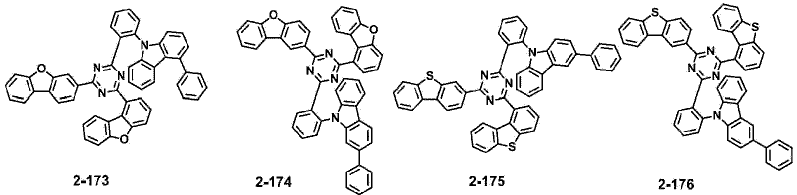

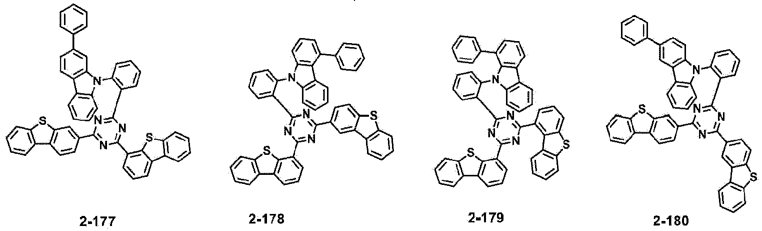

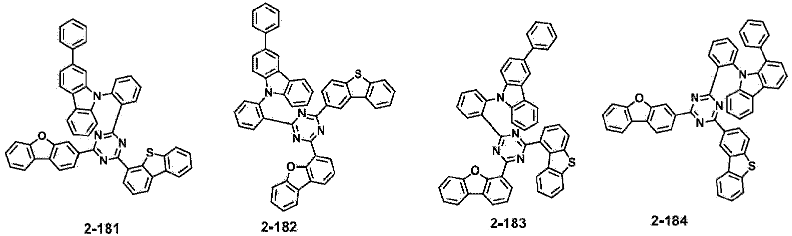

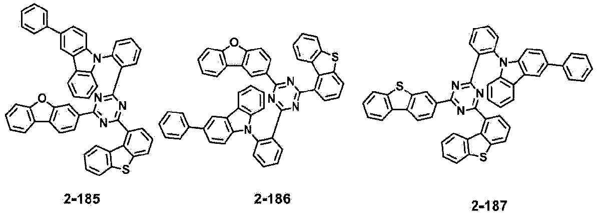

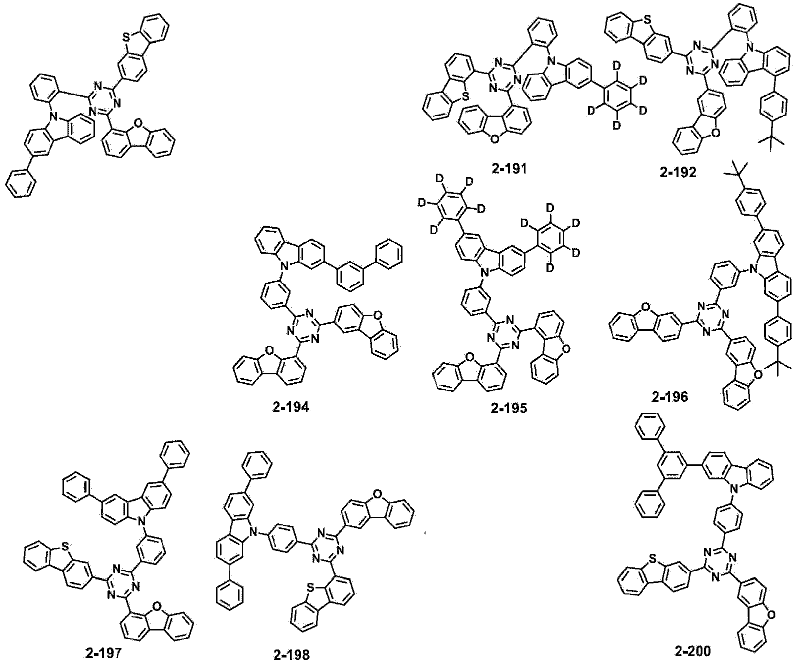

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

One aspect of the present specification provides an organic light emitting device including a first electrode; a second electrode; and one or more organic material layers provided between the first electrode and the second electrode, wherein one or more layers of the organic material layers include the heterocyclic compound of Chemical Formula 1.

In one aspect of the present specification, the first electrode may be an anode, and the second electrode may be a cathode.

In another aspect of the present specification, the first electrode may be a cathode, and the second electrode may be an anode.

In one aspect of the present specification, the organic light emitting device may be a blue organic light emitting device, and the heterocyclic compound of Chemical Formula 1 may be used as a material of the blue organic light emitting device. For example, the heterocyclic compound of Chemical Formula 1 may be included in a light emitting layer of the blue organic light emitting device.

In one aspect of the present specification, the organic light emitting device may be a green organic light emitting device, and the heterocyclic compound of Chemical Formula 1 may be used as a material of the green organic light emitting device. For example, the heterocyclic compound of Chemical Formula 1 may be included in a light emitting layer of the green organic light emitting device.

In one aspect of the present specification, the organic light emitting device may be a red organic light emitting device, and the heterocyclic compound of Chemical Formula 1 may be used as a material of the red organic light emitting device. For example, the heterocyclic compound of Chemical Formula 1 may be included in a light emitting layer of the red organic light emitting device.

The organic light emitting device of the present specification may be manufactured using common organic light emitting device manufacturing methods and materials except that one or more organic material layers are formed using the compound described above.

The compound may be formed into an organic material layer using a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present specification may be formed in a single layer structure, but may be formed in a multilayer structure in which two or more organic material layers are laminated. For example, the organic light emitting device of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present specification, the organic material layer includes a light emitting layer, and the light emitting layer may include the heterocyclic compound of Chemical Formula 1.

In the organic light emitting device of the present specification, the organic material layer includes a light emitting layer, the light emitting layer includes a host, and the host may include the heterocyclic compound of Chemical Formula 1.

In the organic light emitting device of the present specification, the organic material layer includes a light emitting layer, and the light emitting layer may include the heterocyclic compound of Chemical Formula 1 and a compound of the following Chemical Formula 3. 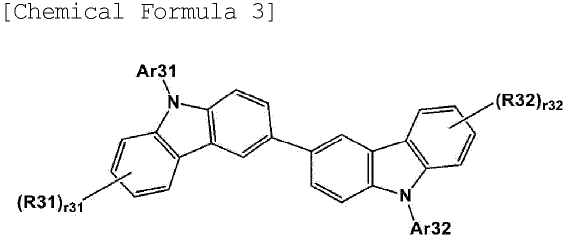 in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a substituted or unsubstituted C2 to C60 heteroaryl group,
r31 is an integer of 0 to 4, and when 2 or greater, R31s are the same as or different from each other, and
r32 is an integer of 0 to 4, and when 2 or greater, R32s are the same as or different from each other.

In one aspect of the present specification, R31 and R32 of Chemical Formula 3 are each independently hydrogen; deuterium; a substituted or unsubstituted C6 to C60 aryl group; or a substituted or unsubstituted C2 to C60 heteroaryl group.

In one aspect of the present specification, R31 and R32 are each independently hydrogen; deuterium; a substituted or unsubstituted C6 to C30 aryl group; or a substituted or unsubstituted C2 to C30 heteroaryl group.

In one aspect of the present specification, R31 and R32 are each independently hydrogen; or a substituted or unsubstituted C6 to C30 aryl group.

In one aspect of the present specification, R31 and R32 are each independently hydrogen; or deuterium.

In one aspect of the present specification, Ar31 and Ar32 of Chemical Formula 3 are each independently a substituted or unsubstituted C6 to C40 aryl group; or a substituted or unsubstituted C2 to C40 heteroaryl group.

In one aspect of the present specification, Ar31 and Ar32 are each independently a substituted or unsubstituted C6 to C30 aryl group; or a substituted or unsubstituted C2 to C30 heteroaryl group.

In one aspect of the present specification, Ar31 and Ar32 are each independently a substituted or unsubstituted C6 to C30 aryl group; or a C2 to C30 heteroaryl group substituted or unsubstituted and including S.

In one aspect of the present specification, Ar31 and Ar32 are each independently a substituted or unsubstituted C6 to C30 aryl group; or a substituted or unsubstituted dibenzothiophene group.

In one aspect of the present specification, Ar31 and Ar32 are each independently a substituted or unsubstituted phenyl group; a substituted or unsubstituted biphenyl group; a substituted or unsubstituted terphenyl group; a substituted or unsubstituted naphthyl group; a substituted or unsubstituted fluorenyl group; a substituted or unsubstituted triphenylene group; or a substituted or unsubstituted dibenzothiophene group.

In one aspect of the present specification, Ar31 and Ar32 are each independently a phenyl group unsubstituted or substituted with a cyano group, a silyl group or an aryl group; a biphenyl group; a terphenyl group; a naphthyl group; a fluorenyl group unsubstituted or substituted with an alkyl group or an aryl group; 9,9'-spirobi[fluorene]; a triphenylene group; or a dibenzothiophene group unsubstituted or substituted with an aryl group unsubstituted or substituted with an alkyl group, or a heteroaryl group.

In one aspect of the present specification, Ar31 and Ar32 may be each independently a phenyl group unsubstituted or substituted with a cyano group, a triphenylsilyl group or an aryl group; a biphenyl group; a terphenyl group; a naphthyl group; a fluorenyl group unsubstituted or substituted with an alkyl group or an aryl group; 9,9'-spirobi[fluorene]; or a triphenylene group.

In one aspect of the present specification, any one of Ar31 and Ar32 is a substituted or unsubstituted dibenzothiophene group, and the other one may be a substituted or unsubstituted C6 to C30 aryl group.

In one aspect of the present specification, Chemical Formula 3 may be represented by the following Chemical Formula 4. 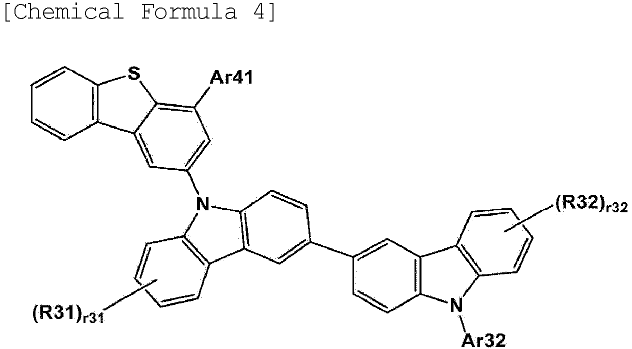 in Chemical Formula 4,
Ar41 is a C6 to C60 aryl group unsubstituted or substituted with an alkyl group; or a C2 to C60 heteroaryl group, and
the rest of the substituents have the same definitions as in Chemical Formula 3.

In one aspect of the present specification, Ar41 of Chemical Formula 3 is a C6 to C40 aryl group; or a C2 to C40 heteroaryl group.

In one aspect of the present specification, Ar41 of Chemical Formula 3 is a C6 to C40 aryl group; or a C2 to C40 heteroaryl group including O or S.

In one aspect of the present specification, Ar41 of Chemical Formula 3 is a C6 to C20 aryl group; or a C2 to C20 heteroaryl group including O or S.

In one aspect of the present specification, Ar41 of Chemical Formula 3 is a phenyl group; a biphenyl group; a naphthyl group; a fluorenyl group unsubstituted or substituted with an alkyl group; a dibenzothiophene group; or a dibenzofuran group.

In one aspect of the present specification, Chemical Formula 3 may be represented by any one of the following compounds, but is not limited thereto.

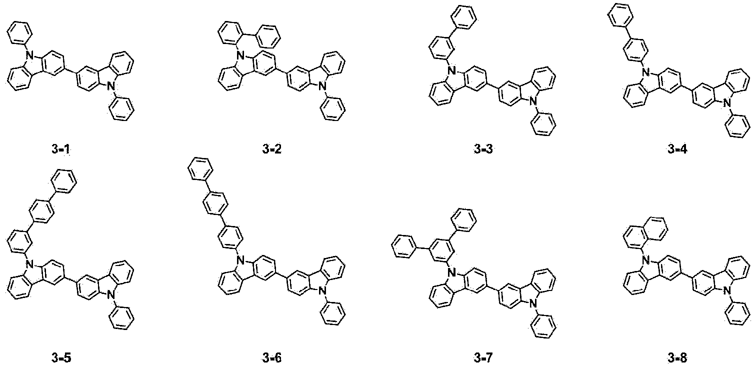

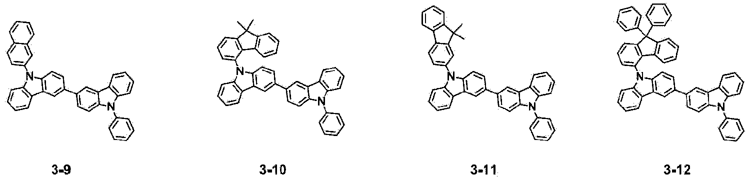

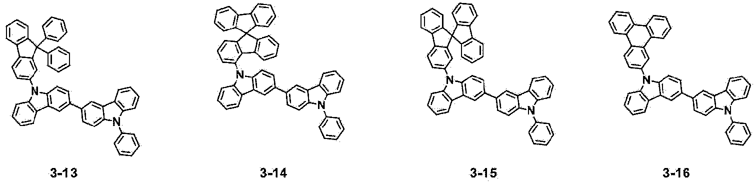

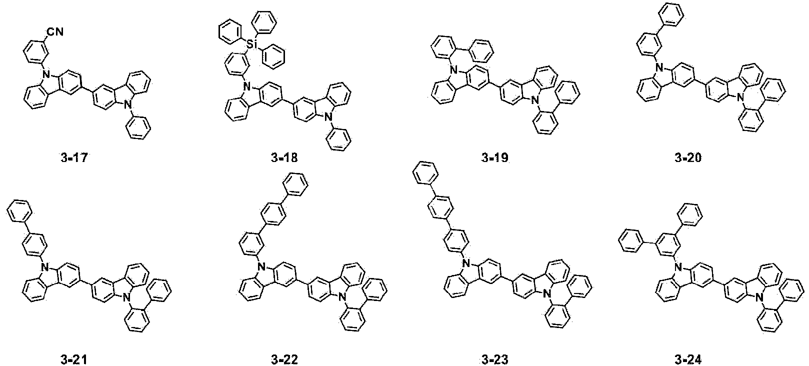

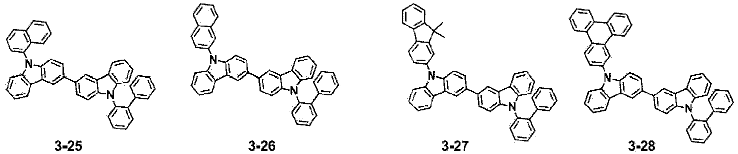

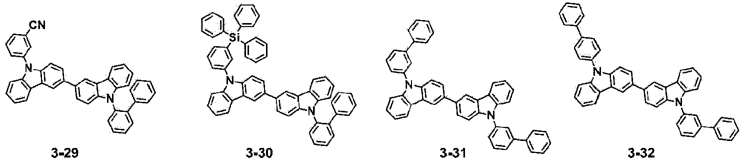

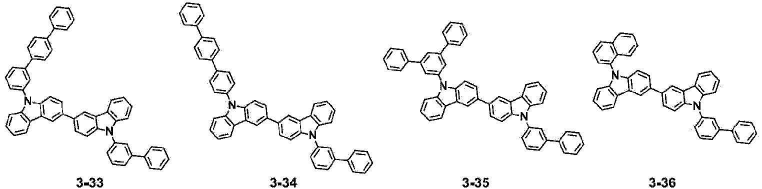

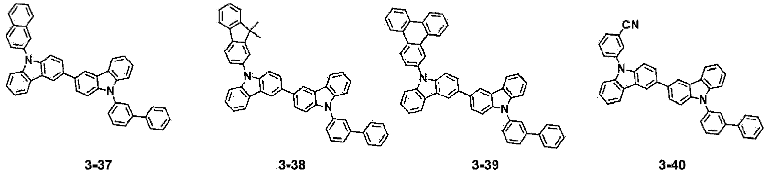

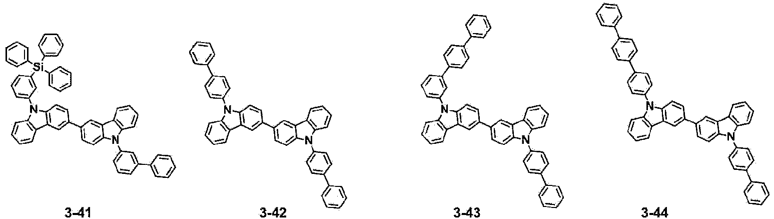

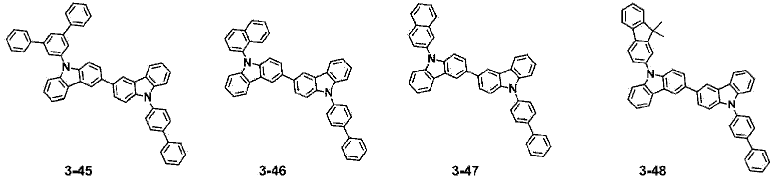

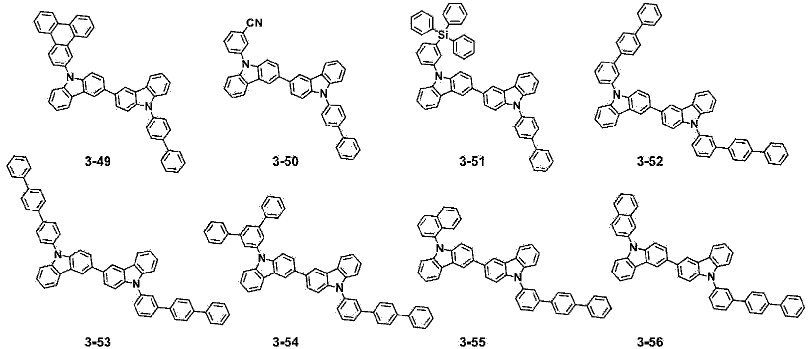

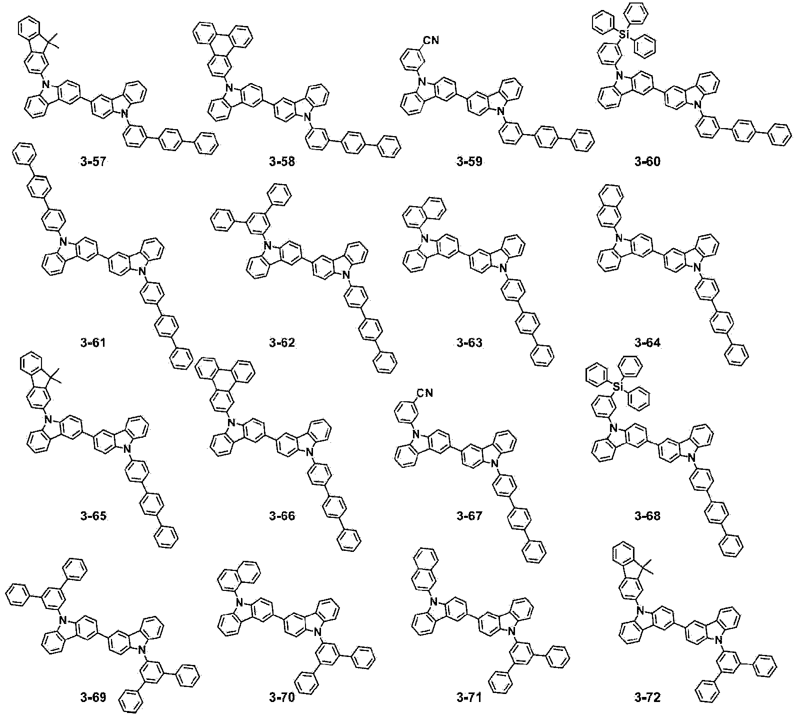

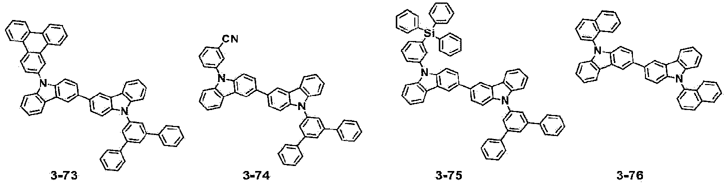

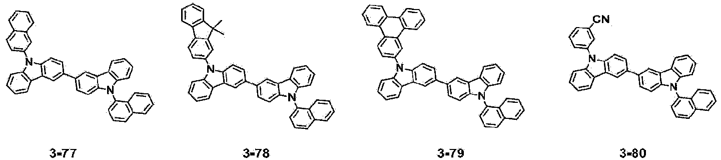

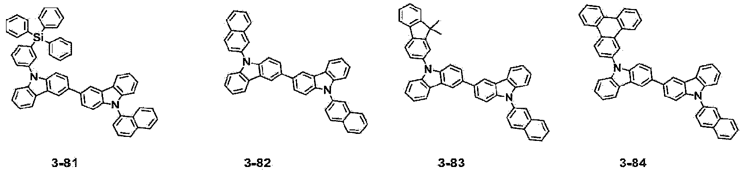

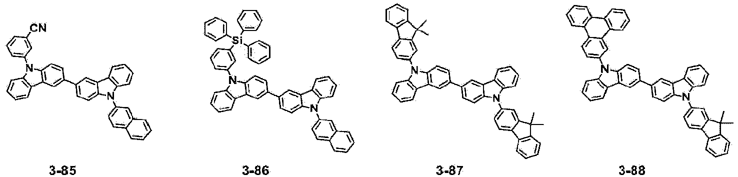

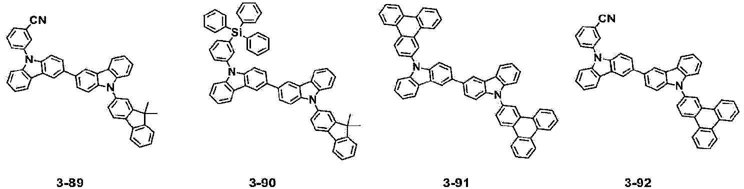

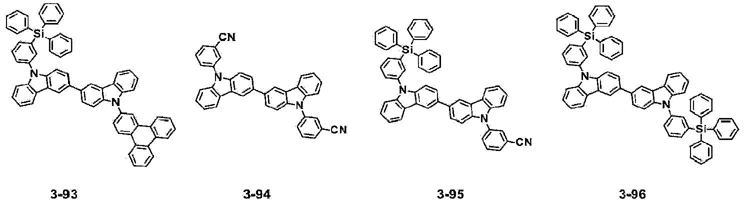

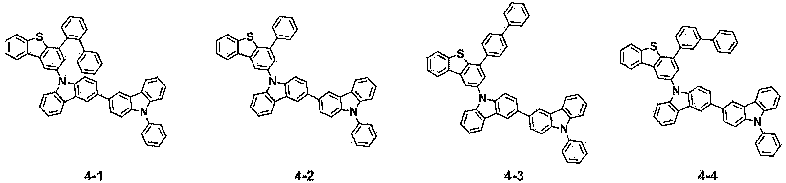

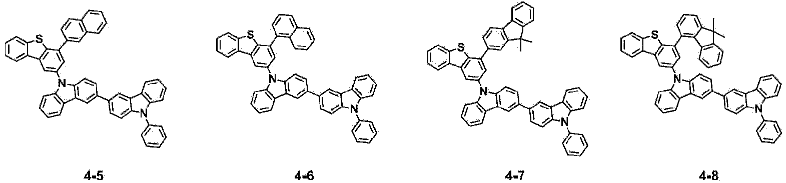

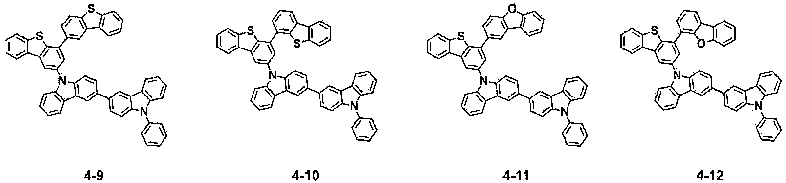

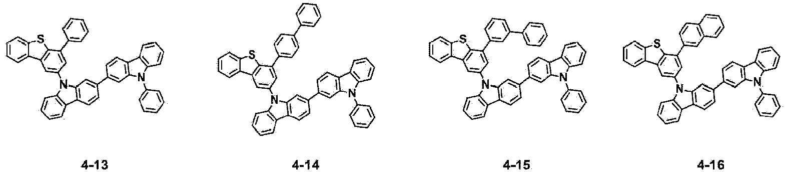

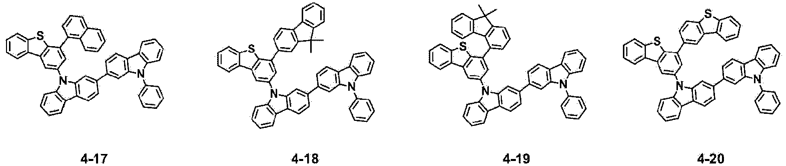

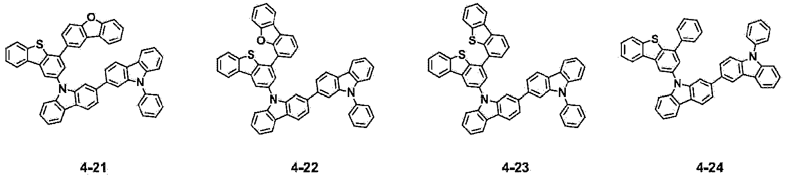

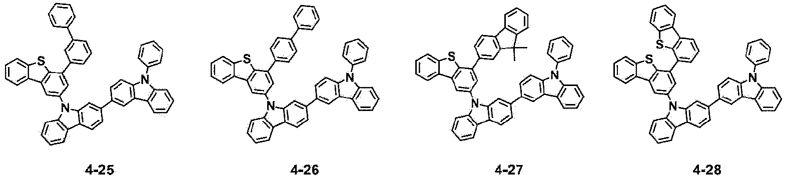

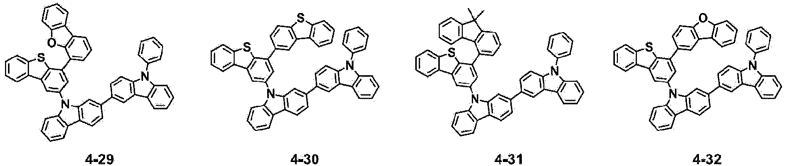

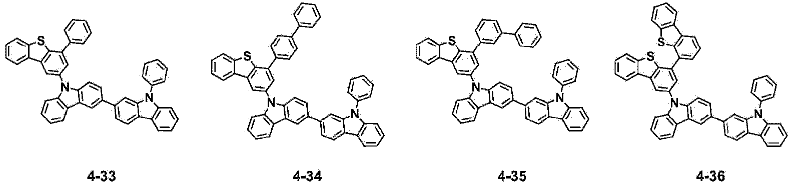

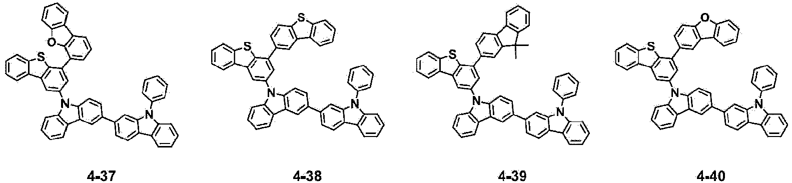

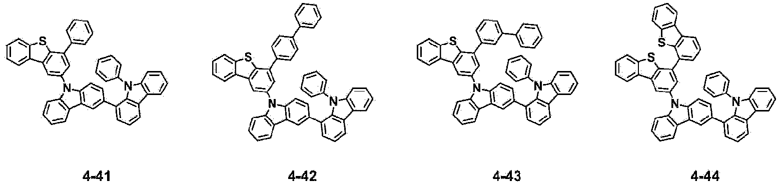

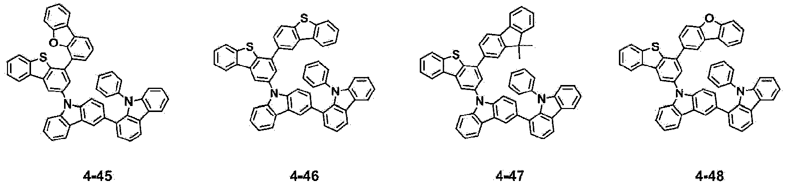

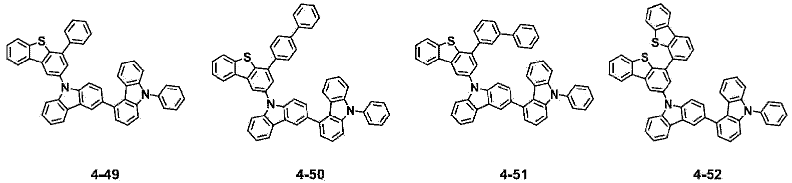

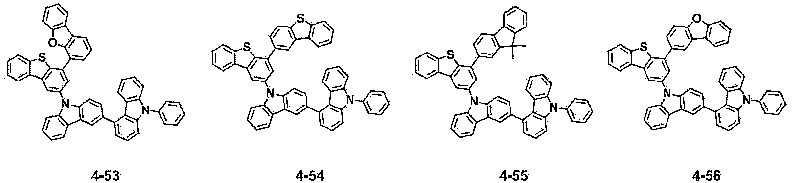

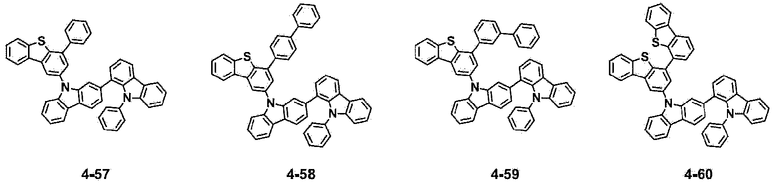

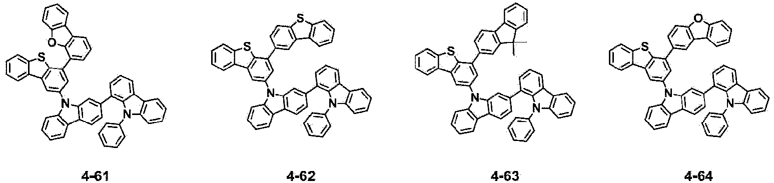

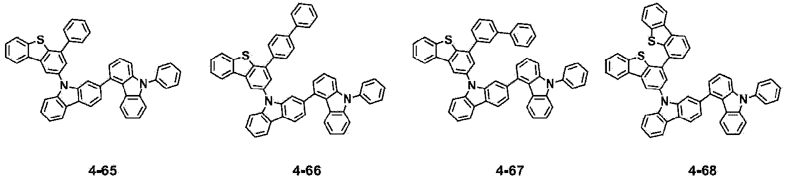

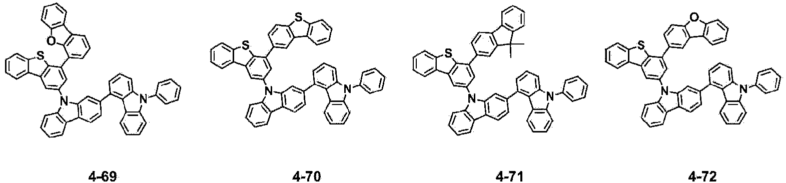

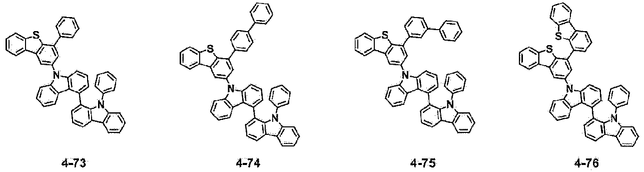

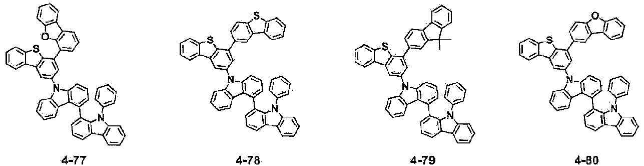

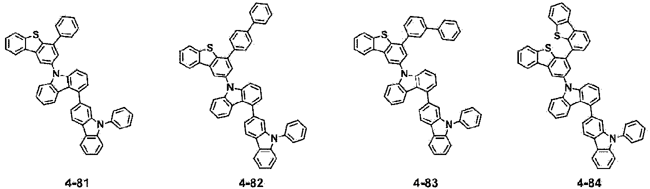

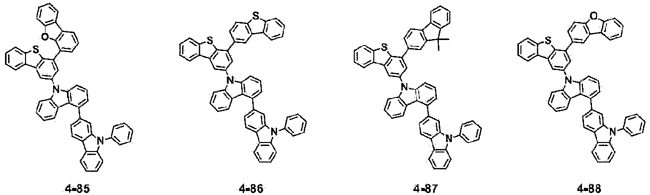

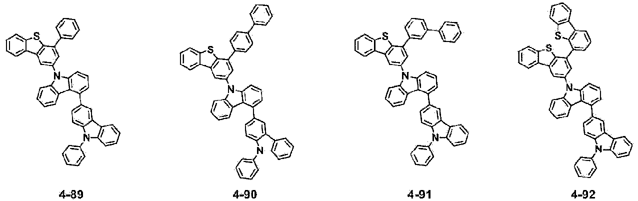

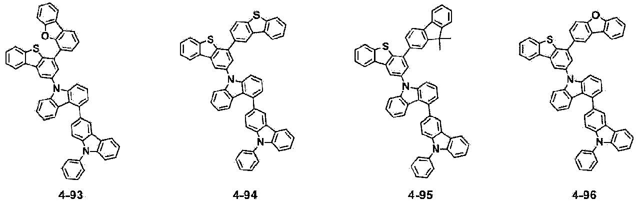

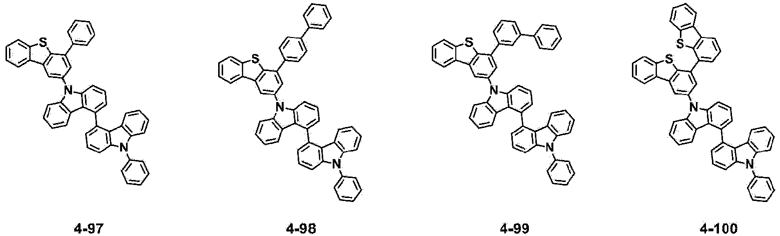

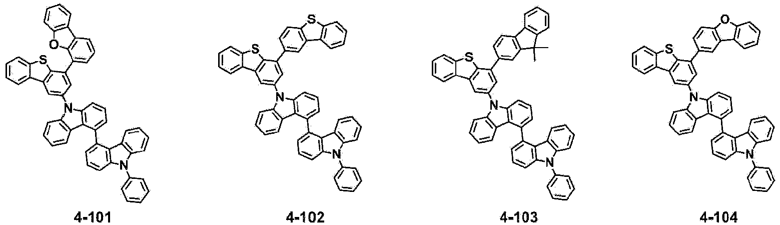

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of the organic light emitting device according to one aspect of the present specification. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer including the heterocyclic compound of Chemical Formula 1 may further include other materials as necessary.

In the organic light emitting device according to one aspect of the present specification, materials other than the heterocyclic compound of Chemical Formula 1 are illustrated below, however, these are for illustrative purposes only and not for limiting the scope of the present application, and these materials may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris (4-carbazoyl-9-ylphenyl) amine (TCTA), 4,4',4"-tri[phenyl (m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrenesulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be additionally used in addition to the compound of Chemical Formula 1 and the compound of Chemical Formula 3, and as necessary, two or more light emitting materials may be mixed and used. Herein, the two or more light emitting materials may be deposited with individual sources of supply or premixed and deposited with one source of supply when used. In addition, fluorescent materials may also be used as the light emitting material, however, phosphorescent materials may also be used. As the light emitting material, materials emitting light by bonding holes and electrons injected from an anode and a cathode, respectively, may be used alone, however, materials having a host material and a dopant material involving together in light emission may also be used.

In one aspect of the present specification, a phosphorescent dopant may be used as the dopant material.

In one aspect of the present specification, Ir(ppy)₃, Ir(ppy)₂(acac), Ir(mppy)₃, Ir(ppy)₂(bpmp), Ir(ppy)₂(m-bppy) and the like may be used as the phosphorescent dopant, and the like, however, the phosphorescent dopant is not limited thereto.

In one aspect of the present specification, Ir(ppy)₃ may be used as the phosphorescent dopant.

When mixing light emitting material hosts, same series hosts may be mixed, or different series hosts may be mixed. For example, any two or more types of materials among N-type host materials or P-type host materials may be selected and used as a host material of a light emitting layer.

In one aspect of the present specification, the compound of Chemical Formula 1 and the compound of Chemical Formula 3 may be mixed and used as the light emitting material host. Herein, the compound of Chemical Formula 1 may be used as the N-type host material, and the compound of Chemical Formula 3 may be used as the P-type host material.

The organic light emitting device according to one aspect of the present specification may be a top-emission type, a bottom-emission type or a dual-emission type depending on the materials used.

The compound according to one aspect of the present specification may also be used in an organic electronic device including an organic solar cell, an organic photo conductor, an organic transistor and the like under a similar principle used in the organic light emitting device.

One aspect of the present specification provides a composition for forming an organic material layer, the composition including the heterocyclic compound of Chemical Formula 1; and the compound of Chemical Formula 3.

The composition for forming an organic material layer according to one aspect of the present specification includes the heterocyclic compound of Chemical Formula 1 and the compound of Chemical Formula 3 in a weight ratio of 1:10 to 10:1, a weight ratio of 1:8 to 8:1, a weight ratio of 1:5 to 5:1, or a weight ratio of 1:2 to 2:1.

When the heterocyclic compound of Chemical Formula 1 and the compound of Chemical Formula 3 are included in the weight ratio of the above-mentioned range, an organic light emitting device having a low driving voltage and excellent light emission efficiency and lifetime may be provided. Particularly, when included in a weight ratio of 1:2 to 2:1, the organic light emitting device has significantly enhanced driving voltage, light emission efficiency and lifetime properties.

The composition for forming an organic material layer according to one aspect of the present specification may be used as a light emitting layer material of an organic light emitting device.

Hereinafter, the present specification will be described in more detail with reference to examples, however, these are for illustrative purposes only.

### <Preparation Example>

### [Preparation Example 1] Preparation of Compound 1-1

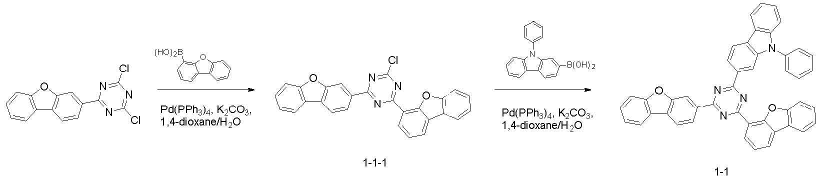

### 1) Preparation of Compound 1-1-1

In a one-neck round bottom flask, a mixture of 2,4-dichloro-6-(dibenzo[b,d]furan-3-yl)-1,3,5-triazine (10 g, 31.6 mmol), dibenzo[b,d]furan-4-ylboronic acid (6.7 g, 31.6 mmol), Pd(PPh₃)₄ (tetrakis(triphenylphosphine)palladium(0)) (1.8 g, 1.58 mmol), K₂CO₃ (potassium carbonate) (8.7 g, 63.2 mmol) and 1,4-dioxane/distilled water (100 mL/20 mL) was refluxed for 4 hours at 120°C. The result was cooled to room temperature and filtered, and then washed with 1,4-dioxane, distilled water and methanol. The result was column purified to obtain Compound 1-1-1 (7.5 g, 53%).

### 2) Preparation of Compound 1-1

In a one-neck round bottom flask, a mixture of Compound 1-1-1 (7.5 g, 16.7 mmol), (9-phenyl-9H-carbazol-2-yl)boronic acid (5.75 g, 20.04 mmol), Pd(PPh₃)₄ (0.96 g, 0.835 mmol), K₂CO₃ (4.6 g, 33.4 mmol) and 1,4-dioxane/distilled water (75 mL/15 mL) was refluxed for 4 hours at 120°C. The result was cooled to room temperature and filtered, and then washed with 1,4-dioxane, distilled water and methanol to obtain Compound 1-1 (8.4 g, 77%).

Target Compound D of the following Table 1 was synthesized in the same manner as in Preparation Example 1 except that Intermediates A, B and C of the following Table 1 were used instead of 2,4-dichloro-6-(dibenzo[b,d]furan-3-yl)-1,3,5-triazine, dibenzo[b,d]furan-4-ylboronic acid and (9-phenyl-9H-carbazol-2-yl)boronic acid in Preparation Example 1.

**[Table 1]**

| Compound | Intermediate A | Intermediate B | Intermediate C | Target Compound D | Overall Yield |
|---|---|---|---|---|---|
| 1-5 | 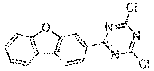 | 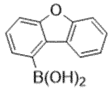 | 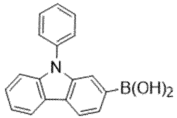 | 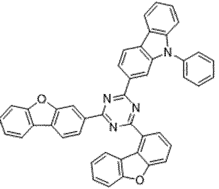 | 41% |
| 1-18 | 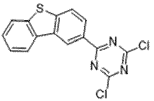 | 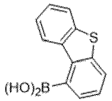 | 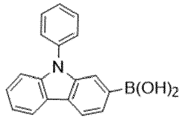 | 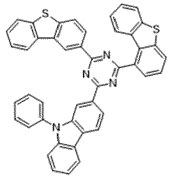 | 43% |
| 1-37 | 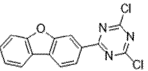 | 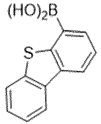 | 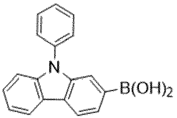 | 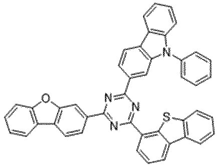 | 48% |
| 1-43 | 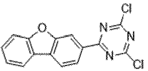 | 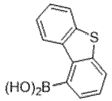 | 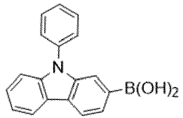 | 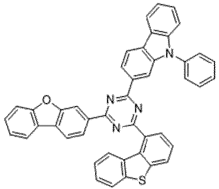 | 44% |
| 1-53 | 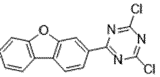 | 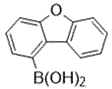 | 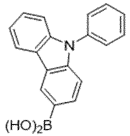 | 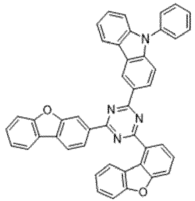 | 48% |
| 1-82 | 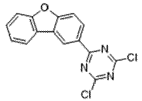 | 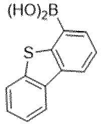 | 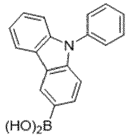 | 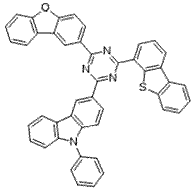 | 46% |
| 1-97 | 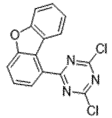 | 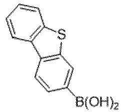 | 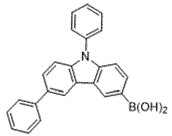 | 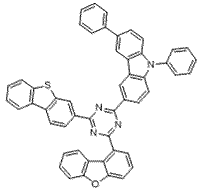 | 45% |
| 1-104 | 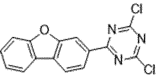 | 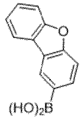 | 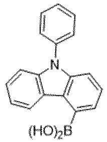 | 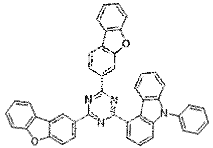 | 41% |
| 1-141 | 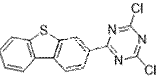 | 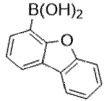 | 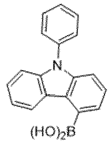 | 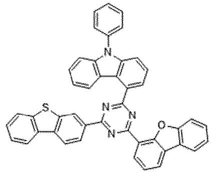 | 38% |
| 1-172 | 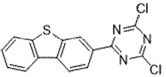 | 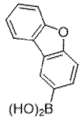 | 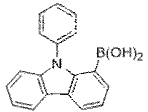 | 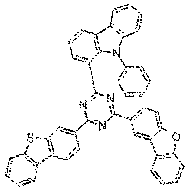 | 24% |

### [Preparation Example 2] Preparation of Compound 1-30

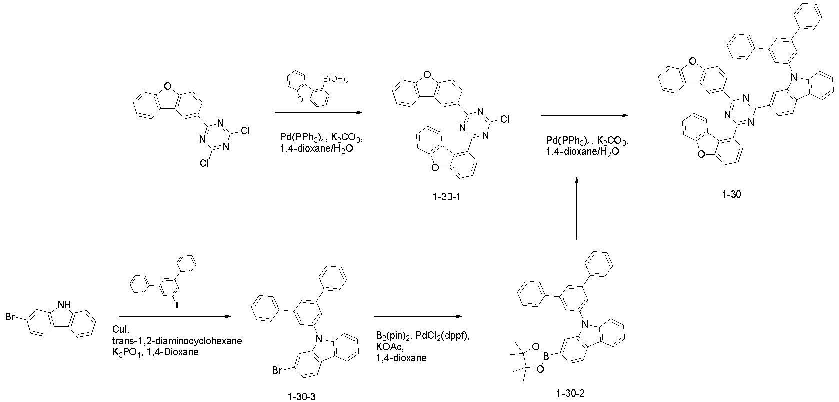

### 1) Preparation of Compound 1-30-3

To a one-neck round bottom flask, 2-bromo-9H-carbazole (15 g, 60.95 mmol), 5'-iodo-1,1':3',1"-terphenyl (23.9 g, 67.04 mmol), CuI (copper(I) iodide) (11.6 g, 60.95 mmol), trans-1,2-diaminocyclohexane (7.0 g, 60.95 mmol), K₃PO₄ (tripotassium phosphate) (25.9 g, 121.9 mmol) and 1,4-dioxane (150 mL) were introduced, and stirred for 8 hours at 120°C. The result was cooled to room temperature, and the organic layer was extracted using ethyl acetate. The organic layer was vacuum concentrated, and then separated by column chromatography to obtain Compound 1-30-3 (22.6 g, 78%).

### 2) Preparation of Compound 1-30-2

To a one-neck round bottom flask, Compound 1-30-3, B₂(pin) ₂ (bis (pinacolato) diboron) (24.1 g, 95.0 mmol), PdCl₂(dppf) (dichloro[1,1'-bis(diphenylphosphino)ferrocene]palladium(II)) (3.5 g, 4.75 mmol), potassium acetate (9.3 g, 95.0 mmol) and 1,4-dioxane (230 mL) were introduced, and stirred for 8 hours at 120°C. The result was extracted with dichloromethane (DCM) and concentrated, and then treated with dichloromethane/methanol to obtain Compound 1-30-2 (16.1 g, 65%).

### 3) Preparation of Compound 1-30-1

Compound 1-30-1 (8.5 g, 60%) was obtained in the same manner as in Preparation of Compound 1-1-1 of Preparation Example 1 except that 2-4-dichloro-6-(dibenzo[b,d]furan-2-yl)-1,3,5-triazine was used instead of 2-4-dichloro-6-(dibenzo[b,d]furan-3-yl)-1,3,5-triazine, and dibenzo[b,d]furan-1-ylboronic acid (10 g, 31.63 mmol) was used instead of dibenzo[b,d]furan-4-ylboronic acid.

### 4) Preparation of Compound 1-30

Compound 1-30 (11.5 g, 75%) was obtained in the same manner as in Preparation of Compound 1-1 of Preparation Example 1 using Compounds 1-30-2 and 1-30-1 synthesized above.

Target Compound D of the following Table 2 was synthesized in the same manner as in Preparation Examples 1 and 2 except that Intermediates A and B of the following Table 2 were used instead of 2,4-dichloro-6-(dibenzo[b,d]furan-3-yl)-1,3,5-triazine and dibenzo[b,d]furan-4-ylboronic acid in Preparation Example 1, and Intermediates C-(1) and C-(2) of the following Table 2 were used instead of 2-bromo-9H-carbazole and 5'-iodo-1,1':3',1"-terphenyl in Preparation Example 2.

**[Table 2]**

| Compound | Intermediate A | Intermediate B | Intermediate C-(1) | Intermediate C-(2) | Target Compound D | Overall Yield |
|---|---|---|---|---|---|---|
| 1-55 | 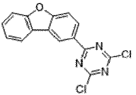 | 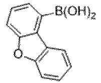 | 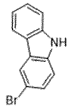 | 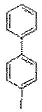 | 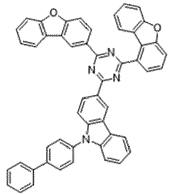 | 55% |
| 1-80 | 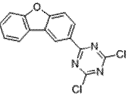 | 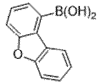 | 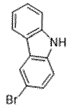 | 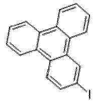 | 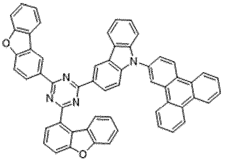 | 47% |

### [Preparation Example 3] Preparation of Compound 2-1

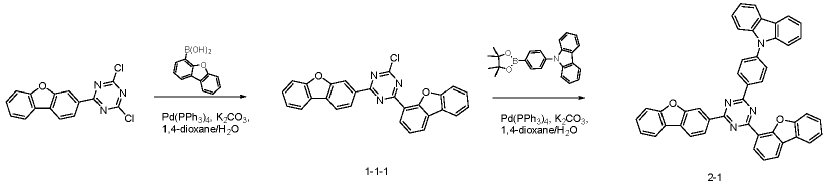

Target Compound 2-1 (11.5 g, 55%) was obtained in the same manner as in Preparation Example 1 except that 9-(4-(4,4,5,5-tetramethyl-1,3,2-dioxaborolan-2-yl)phenyl)-9H-carbazole was used instead of (9-phenyl-9H-carbazol-2-yl)boronic acid.

Target Compound D of the following Table 3 was synthesized in the same manner as in Preparation Example 1 except that Intermediates A, B and C of the following Table 3 were used instead of 2,4-dichloro-6-(dibenzo[b,d]furan-3-yl)-1,3,5-triazine, dibenzo[b,d]furan-4-ylboronic acid and (9-phenyl-9H-carbazol-2-yl)boronic acid in Preparation Example 1.

**[Table 3]**

| Compound | Intermediate A | Intermediate B | Intermediate C | Target Compound D | Overall Yield |
|---|---|---|---|---|---|
| 2-5 | 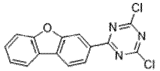 | 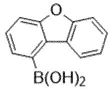 | 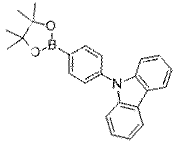 | 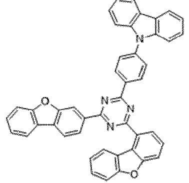 | 50% |
| 2-9 | 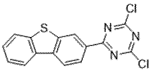 | 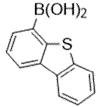 | 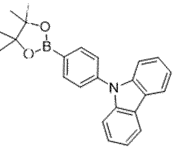 | 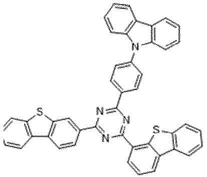 | 58% |
| 2-19 | 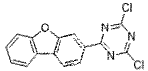 | 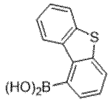 | 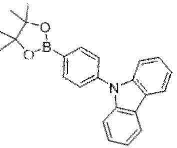 | 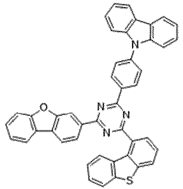 | 59% |
| 2-28 | 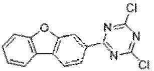 | 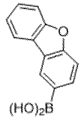 | 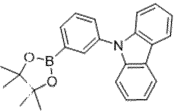 | 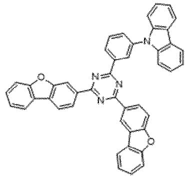 | 53% |
| 2-37 | 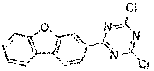 | 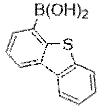 | 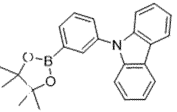 | 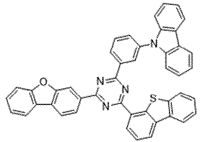 | 52% |

### [Preparation Example 4] Preparation of Compound 2-49

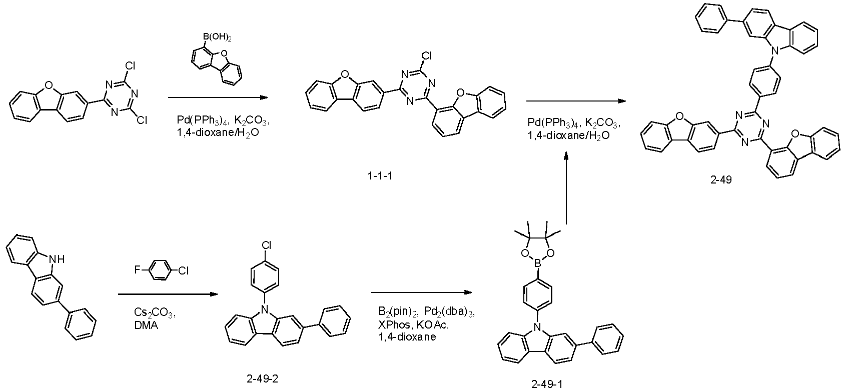

### 1) Preparation of Compound 2-49-2

In a one-neck round bottom flask, a mixture of 2-phenyl-9H-carbazole (15 g, 61.65 mmol), 1-chloro-4-fluorobenzene (9.7 g, 73.98 mmol), Cs₂CO₃ (cesium carbonate) (40.2 g, 123.3 mmol) and DMA (dimethylacetamide) (150 mL) was stirred for 8 hours at 120°C. The result was cooled and then filtered, and, after removing the solvent of the filtrate, purified by column chromatography to obtain Compound 2-49-2 (19.6 g, 90%).

### 2) Preparation of Compound 2-49-1

In a one-neck round bottom flask, a mixture of Compound 2-49-2 (19.6 g, 55.48 mmol), B₂(pin)₂ (28.2 g, 110.97 mmol), Pd₂(dba)₃ (2.54 g, 2.774 mmol), Xphos (2-dicyclohexylphosphino-2',4',6'-triisopropylbiphenyl) (2.64 g, 5.55 mmol), KOAc (potassium acetate) (10.9 g, 110.97 mmol) and 1,4-dioxane (200 mL) was refluxed for 4 hours at 140°C.

The result was extracted with dichloromethane and concentrated, and then treated with dichloromethane/methanol to obtain Compound 2-49-1 (17.8 g, 72%).

### 3) Preparation of Compound 2-49

Target Compound 2-49 (9.8 g, 80%) was obtained in the same manner as in Preparation Example 1 except that Compound 2-49-1 was used instead of (9-phenyl-9H-carbazol-2-yl)boronic acid.

Target Compound D of the following Table 4 was synthesized in the same manner as in Preparation Examples 1 and 4 except that Intermediates A and B of the following Table 4 were used instead of 2,4-dichloro-6-(dibenzo[b,d]furan-3-yl)-1,3,5-triazine and dibenzo[b,d]furan-4-ylboronic acid in Preparation Example 1, and Intermediates C-(1) and C-(2) of the following Table 4 were used instead of 2-phenyl-9H-carbazole and 1-chloro-4-fluorobenzene in Preparation Example 4.

**[Table 4]**

| Compound | Intermediate A | Intermediate B | Intermediate C- (1) | Intermediate C-(2) | Target Compound D | Overall Yield |
|---|---|---|---|---|---|---|
| 2-49 | 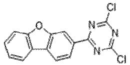 | 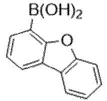 | 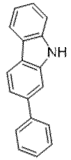 | 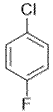 | 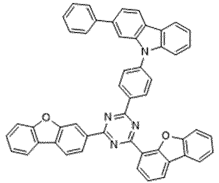 | 51% |
| 2-67 | 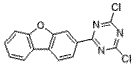 | 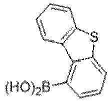 | 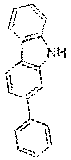 | 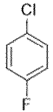 | 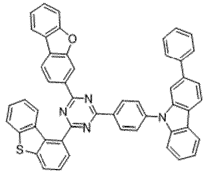 | 47% |
| 2-97 | 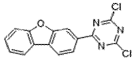 | 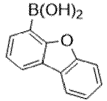 | 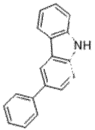 | 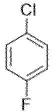 | 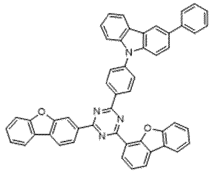 | 43% |
| 2-109 | 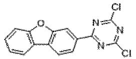 | 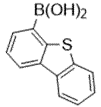 | 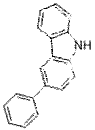 | 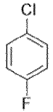 | 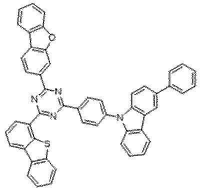 | 44% |
| 2-124 | 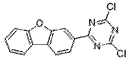 | 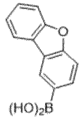 | 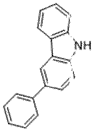 | 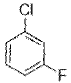 | 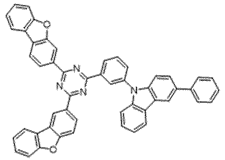 | 49% |

### [Preparation Example 5] Preparation of Compound 3-3

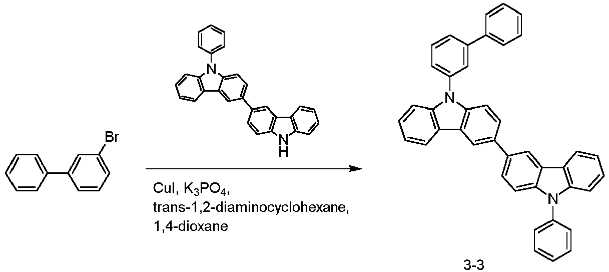

3-Bromo-1,1'-biphenyl (3.7 g, 15.8 mmol), 9-phenyl-9H,9'H-3,3'-bicarbazole (6.5 g, 15.8 mmol), CuI (3.0 g, 15.8 mmol), trans-1,2-diaminocyclohexane (1.9 mL, 15.8 mmol) and K₃PO₄ (3.3 g, 31.6 mmol) were dissolved in 1,4-dioxane (100 mL), and refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 3-3 (7.5 g, 85%).

Target Compound G of the following Table 5 was synthesized in the same manner as in Preparation Example 5 except that Intermediates E and F of the following Table 5 were used instead of 3-bromo-1,1'-biphenyl and 9-phenyl-9H,9'H-3,3'-bicarbazole in Preparation Example 5.

**[Table 5]**

| Compound | Intermediate E | Intermediate F | Target Compound G | Overall Yield |
|---|---|---|---|---|
| 3-4 | 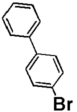 | 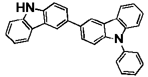 | 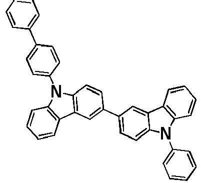 | 83% |
| 3-7 | 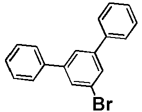 | 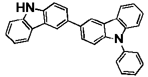 | 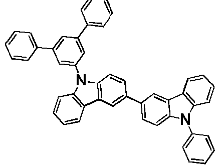 | 84% |
| 3-31 | 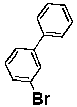 | 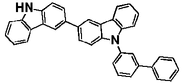 | 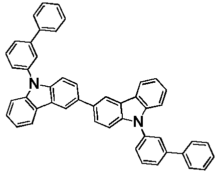 | 81% |
| 3-32 | 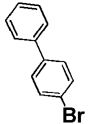 | 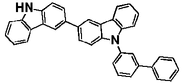 | 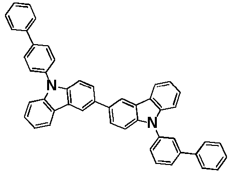 | 80% |
| 3-42 | 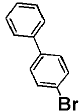 | 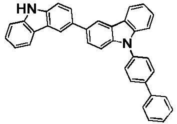 | 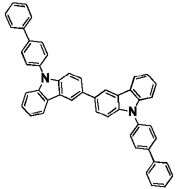 | 82% |

### [Preparation Example 6] Preparation of Compound 4-2

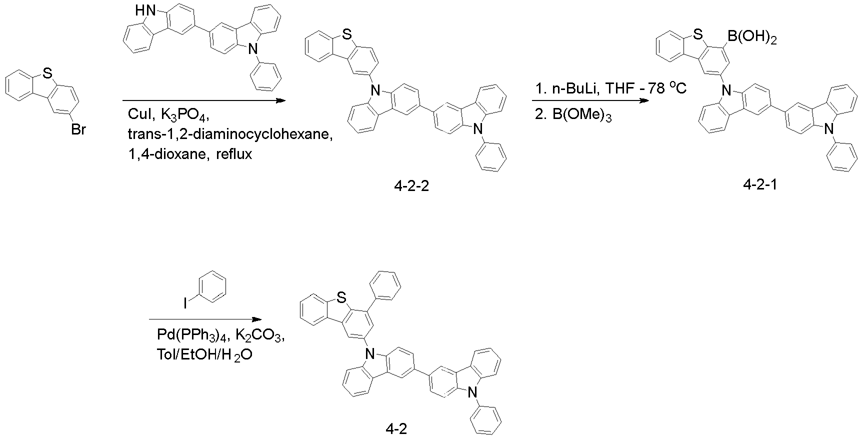

### 1) Preparation of Compound 4-2-2

2-Bromodibenzo [b,d] thiophene (4.2 g, 15.8 mmol), 9-phenyl-9H,9'H-3,3'-bicarbazole (6.5 g, 15.8 mmol), CuI (copper iodide) (3.0 g, 15.8 mmol), trans-1,2-diaminocyclohexane (1.9 mL, 15.8 mmol) and K₃PO₄ (tripotassium phosphate) (3.3 g, 31.6 mmol) were dissolved in 1,4-dioxane (100 mL), and refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain Compound 4-2-2 (7.9 g, 85%).

### 2) Preparation of Compound 4-2-1

To a mixture solution of Compound 4-2-1 (8.4 g, 14.3 mmol) and THF (100 mL), 2.5 M n-BuLi (7.4 mL, 18.6 mmol) was added dropwise at -78°C, and the mixture was stirred for 1 hour at room temperature. Trimethyl borate (4.8 mL, 42.9 mmol) was added dropwise to the reaction mixture, and the result was stirred for 2 hours at room temperature. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:MeOH=100:3), and recrystallized with DCM to obtain Compound 4-2-1 (3.9 g, 70%).

### 3) Preparation of Compound 4-2

Compound 4-2-1 (6.7 g, 10.5 mmol), iodobenzene (2.1 g, 10.5 mmol), Pd(PPh₃)₄ (606 mg, 0.52 mmol) and K₂CO₃ (2.9 g, 21.0 mmol) were dissolved in toluene/EtOH/H₂O (100 mL/20 mL/20 mL), and refluxed for 12 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:Hex=1:3), and recrystallized with methanol to obtain target Compound 4-2 (4.9 g, 70%).

### [Preparation Example 7] Preparation of Compound 4-3

Target Compound 4-3 (83%) was obtained in the same manner as in Preparation of Compound 4-2 except that 4-iodo-1,1'-biphenyl was used instead of iodobenzene in Preparation Example 6.

Compounds other than the compounds described in Preparation Example 1 to Preparation Example 7 and Tables 1 to 5 were also prepared in the same manner as in the preparation examples described above.

Synthesis identification data for the compounds prepared above are as described in the following Table 6 and Table 7. The following Table 6 shows measurement values of FD-mass spectrometry (FD-Mass: field desorption mass spectrometry), and the following Table 7 shows measurement values of ¹H NMR (CDCl₃, 200 Mz).

**[Table 6]**

| Compo und | FD-Mass | Compo und | FD-MASS |
|---|---|---|---|
| 1-1 | m/z=654.21(C45H26N4O2=654.73) | 1-2 | m/z=654.21(C45H26N4O2=654.73) |
| 1-3 | m/z=654.21(C45H26N4O2=654.73) | 1-4 | m/z=654.21(C45H26N4O2=654.73) |
| 1-5 | m/z=654.21(C45H26N4O2=654.73) | 1-6 | m/z=654.21(C45H26N4O2=654.73) |
| 1-7 | m/z=730.24(C51H30N4O2=730.83) | 1-8 | m/z=730.24(C51H30N4O2=730.83) |
| 1-9 | m/z=730.24(C51H30N4O2=730.83) | 1-10 | m/z=730.24(C51H30N4O2=730.83) |
| 1-11 | m/z=730.24(C51H30N4O2=730.83) | 1-12 | m/z=730.24(C51H30N4O2=730.83) |
| 1-13 | m/z=686.16(C45H26N4S2=686.85) | 1-14 | m/z=686.16(C45H26N4S2=686.85) |
| 1-15 | m/z=686.16(C45H26N4S2=686.85) | 1-16 | m/z=686.16(C45H26N4S2=686.85) |
| 1-17 | m/z=686.16(C45H26N4S2=686.85) | 1-18 | m/z=686.16(C45H26N4S2=686.85) |
| 1-19 | m/z=762.19(C51H30N4S2=762.95) | 1-20 | m/z=736.18(C49H28N4S2=736.91) |
| 1-21 | m/z=762.19(C51H30N4S2=762.95) | 1-22 | m/z=736.18(C49H28N4S2=736.91) |
| 1-23 | m/z=838.22(C57H34N4S2=839.05) | 1-24 | m/z=691.19(C45H21D5N4S2=691.88) |
| 1-25 | m/z=659.24(C45H21D5N4O2=659.76) | 1-26 | m/z=704.22(C49H28N4O2=704.79) |
| 1-27 | m/z=704.22(C49H28N4O2=704.79) | 1-28 | m/z=754.24(C53H30N4O2=754.85) |
| 1-29 | m/z=754.24(C53H30N4O2=754.85) | 1-30 | m/z=806.27(C57H34N4O2=806.93) |
| 1-31 | m/z=778.24(C55H30N4O2=778.87) | 1-32 | m/z=806.27(C57H34N4O2=806.93) |
| 1-33 | m/z=806.27(C57H34N4O2=806.93) | 1-34 | m/z=806.27(C57H34N4O2=806.93) |
| 1-35 | m/z=730.24(C51H30N4O2=730.83) | 1-36 | m/z=754.24(C53H30N4O2=754.85) |
| 1-37 | m/z=670.18(C45H26N4OS=670.79) | 1-38 | m/z=670.18(C45H26N4OS=670.79) |
| 1-39 | m/z=670.18(C45H26N4OS=670.79) | 1-40 | m/z=670.18(C45H26N4OS=670.79) |
| 1-41 | m/z=670.18(C45H26N4OS=670.79) | 1-42 | m/z=670.18(C45H26N4OS=670.79) |
| 1-43 | m/z=670.18(C45H26N4OS=670.79) | 1-44 | m/z=670.18(C45H26N4OS=670.79) |
| 1-45 | m/z=670.18(C45H26N4OS=670.79) | 1-46 | m/z=670.18(C45H26N4OS=670.79) |
| 1-47 | m/z=670.18(C45H26N4OS=670.79) | 1-48 | m/z=670.18(C45H26N4OS=670.79) |
| 1-49 | m/z=654.21(C45H26N4O2=654.73) | 1-50 | m/z=654.21(C45H26N4O2=654.73) |
| 1-51 | m/z=654.21(C45H26N4O2=654.73) | 1-52 | m/z=654.21(C45H26N4O2=654.73) |
| 1-53 | m/z=654.21(C45H26N4O2=654.73) | 1-54 | m/z=654.21(C45H26N4O2=654.73) |
| 1-55 | m/z=730.24(C51H30N4O2=730.83) | 1-56 | m/z=730.24(C51H30N4O2=730.83) |
| 1-57 | m/z=730.24(C51H30N4O2=730.83) | 1-58 | m/z=730.24(C51H30N4O2=730.83) |
| 1-59 | m/z=730.24(C51H30N4O2=730.83) | 1-60 | m/z=730.24(C51H30N4O2=730.83) |
| 1-61 | m/z=686.16(C45H26N4S2=686.85) | 1-62 | m/z=686.16(C45H26N4S2=686.85) |
| 1-63 | m/z=686.16(C45H26N4S2=686.85) | 1-64 | m/z=686.16(C45H26N4S2=686.85) |
| 1-65 | m/z=686.16(C45H26N4S2=686.85) | 1-66 | m/z=686.16(C45H26N4S2=686.85) |
| 1-67 | m/z=762.19(C51H30N4S2=762.95) | 1-68 | m/z=838.22(C57H34N4S2=839.05) |
| 1-69 | m/z=762.19(C51H30N4S2=762.95) | 1-70 | m/z=736.18(C49H28N4S2=736.91) |
| 1-71 | m/z=838.22(C57H34N4S2=839.05) | 1-72 | m/z=691.19(C45H21D5N4S2=691.88) |
| 1-73 | m/z=659.24(C45H21D5N4O2=659.76) | 1-74 | m/z=806.27(C57H34N4O2=806.93) |
| 1-75 | m/z=778.24(C55H30N4O2=778.87) | 1-76 | m/z=806.27(C57H34N4O2=806.93) |
| 1-77 | m/z=754.24(C53H30N4O2=754.85) | 1-78 | m/z=806.27(C57H34N4O2=806.93) |
| 1-79 | m/z=730.24(C51H30N4O2=730.83) | 1-80 | m/z=804.25(C57H32N4O2=804.91) |
| 1-81 | m/z=670.18(C45H26N4OS=670.79) | 1-82 | m/z=670.18(C45H26N4OS=670.79) |
| 1-83 | m/z=670.18(C45H26N4OS=670.79) | 1-84 | m/z=670.18(C45H26N4OS=670.79) |
| 1-85 | m/z=670.18(C45H26N4OS=670.79) | 1-86 | m/z=670.18(C45H26N4OS=670.79) |
| 1-87 | m/z=670.18(C45H26N4OS=670.79) | 1-88 | m/z=670.18(C45H26N4OS=670.79) |
| 1-89 | m/z=670.18(C45H26N4OS=670.79) | 1-90 | m/z=670.18(C45H26N4OS=670.79) |
| 1-91 | m/z=670.18(C45H26N4OS=670.79) | 1-92 | m/z=670.18(C45H26N4OS=670.79) |
| 1-93 | m/z=746.21(C51H30N4OS=746.89) | 1-94 | m/z=746.21(C51H30N4OS=746.89) |
| 1-95 | m/z=726.25(C49H34N4OS=726.90) | 1-96 | m/z=675.21(C45H21D5N4OS=675.82) |
| 1-97 | m/z=746.21(C51H30N4OS=746.89) | 1-98 | m/z=751.25(C51H25D5N4OS=751.92) |
| 1-99 | m/z=822.25(C57H34N4OS=822.99) | 1-100 | m/z=669.19(C46H27N3OS=659.80) |
| 1-101 | m/z=654.21(C45H26N4O2=654.73) | 1-102 | m/z=654.21(C45H26N4O2=654.73) |
| 1-103 | m/z=654.21(C45H26N4O2=654.73) | 1-104 | m/z=654.21(C45H26N4O2=654.73) |
| 1-105 | m/z=654.21(C45H26N4O2=654.73) | 1-106 | m/z=654.21(C45H26N4O2=654.73) |
| 1-107 | m/z=730.24(C51H30N4O2=730.83) | 1-108 | m/z=730.24(C51H30N4O2=730.83) |
| 1-109 | m/z=730.24(C51H30N4O2=730.83) | 1-110 | m/z=730.24(C51H30N4O2=730.83) |
| 1-111 | m/z=730.24(C51H30N4O2=730.83) | 1-112 | m/z=730.24(C51H30N4O2=730.83) |
| 1-113 | m/z=686.16(C45H26N4S2=686.85) | 1-114 | m/z=686.16(C45H26N4S2=686.85) |
| 1-115 | m/z=686.16(C45H26N4S2=686.85) | 1-116 | m/z=686.16(C45H26N4S2=686.85) |
| 1-117 | m/z=686.16(C45H26N4S2=686.85) | 1-118 | m/z=686.16(C45H26N4S2=686.85) |
| 1-119 | m/z=762.19(C51H30N4S2=762.95) | 1-120 | m/z=736.18(C49H28N4S2=736.91) |
| 1-121 | m/z=762.19(C51H30N4S2=762.95) | 1-122 | m/z=736.18(C49H28N4S2=736.91) |
| 1-123 | m/z=838.22(C57H34N4S2=839.05) | 1-124 | m/z=691.19(C45H21D5N4S2=691.88) |
| 1-125 | m/z=659.24(C45H21D5N4O2=659.76) | 1-126 | m/z=704.22(C49H28N4O2=704.79) |
| 1-127 | m/z=806.27(C57H34N4O2=806.93) | 1-128 | m/z=804.25(C57H32N4O2=804.91) |
| 1-129 | m/z=754.24(C53H30N4O2=754.85) | 1-130 | m/z=806.27(C57H34N4O2=806.93) |
| 1-131 | m/z=778.24(C55H30N4O2=778.87) | 1-132 | m/z=806.27(C57H34N4O2=806.93) |
| 1-133 | m/z=670.18(C45H26N4OS=670.79) | 1-134 | m/z=670.18(C45H26N4OS=670.79) |
| 1-135 | m/z=670.18(C45H26N4OS=670.79) | 1-136 | m/z=670.18(C45H26N4OS=670.79) |
| 1-137 | m/z=670.18(C45H26N4OS=670.79) | 1-138 | m/z=670.18(C45H26N4OS=670.79) |
| 1-139 | m/z=670.18(C45H26N4OS=670.79) | 1-140 | m/z=670.18(C45H26N4OS=670.79) |
| 1-141 | m/z=670.18(C45H26N4OS=670.79) | 1-142 | m/z=670.18(C45H26N4OS=670.79) |
| 1-143 | m/z=670.18(C45H26N4OS=670.79) | 1-144 | m/z=670.18(C45H26N4OS=670.79) |
| 1-145 | m/z=654.21(C45H26N4O2=654.73) | 1-146 | m/z=654.21(C45H26N4O2=654.73) |
| 1-147 | m/z=654.21(C45H26N4O2=654.73) | 1-148 | m/z=654.21(C45H26N4O2=654.73) |
| 1-149 | m/z=654.21(C45H26N4O2=654.73) | 1-150 | m/z=654.21(C45H26N4O2=654.73) |
| 1-151 | m/z=704.22(C49H28N4O2=704.79) | 1-152 | m/z=730.24(C51H30N4O2=730.83) |
| 1-153 | m/z=686.16(C45H26N4S2=686.85) | 1-154 | m/z=686.16(C45H26N4S2=686.85) |
| 1-155 | m/z=686.16(C45H26N4S2=686.85) | 1-156 | m/z=686.16(C45H26N4S2=686.85) |
| 1-157 | m/z=686.16(C45H26N4S2=686.85) | 1-158 | m/z=686.16(C45H26N4S2=686.85) |
| 1-159 | m/z=736.18(C49H28N4S2=736.91) | 1-160 | m/z=659.24(C45H21D5N4O2=659.76) |
| 1-161 | m/z=670.18(C45H26N4OS=670.79) | 1-162 | m/z=670.18(C45H26N4OS=670.79) |
| 1-163 | m/z=670.18(C45H26N4OS=670.79) | 1-164 | m/z=670.18(C45H26N4OS=670.79) |
| 1-165 | m/z=670.18(C45H26N4OS=670.79) | 1-166 | m/z=670.18(C45H26N4OS=670.79) |
| 1-167 | m/z=670.18(C45H26N4OS=670.79) | 1-168 | m/z=670.18(C45H26N4OS=670.79) |
| 1-169 | m/z=670.18(C45H26N4OS=670.79) | 1-170 | m/z=670.18(C45H26N4OS=670.79) |
| 1-171 | m/z=670.18(C45H26N4OS=670.79) | 1-172 | m/z=670.18(C45H26N4OS=670.79) |
| 1-173 | m/z=746.21(C51H30N4OS=746.89) | 1-174 | m/z=675.21(C45H21D5N4OS=675.82) |
| 1-175 | m/z=726.25(C49H34N4OS=726.90) | 1-176 | m/z=687.22(C46H25D3N4OS=687.84) |
| 2-1 | m/z=654.21(C45H26N4O2=654.73) | 2-2 | m/z=654.21(C45H26N4O2=654.73) |
| 2-3 | m/z=654.21(C45H26N4O2=654.73) | 2-4 | m/z=654.21(C45H26N4O2=654.73) |
| 2-5 | m/z=654.21(C45H26N4O2=654.73) | 2-6 | m/z=654.21(C45H26N4O2=654.73) |
| 2-7 | m/z=686.16(C45H26N4S2=686.85) | 2-8 | m/z=686.16(C45H26N4S2=686.85) |
| 2-9 | m/z=686.16(C45H26N4S2=686.85) | 2-10 | m/z=686.16(C45H26N4S2=686.85) |
| 2-11 | m/z=686.16(C45H26N4S2=686.85) | 2-12 | m/z=686.16(C45H26N4S2=686.85) |
| 2-13 | m/z=670.18(C45H26N4OS=670.79) | 2-14 | m/z=670.18(C45H26N4OS=670.79) |
| 2-15 | m/z=670.18(C45H26N4OS=670.79) | 2-16 | m/z=670.18(C45H26N4OS=670.79) |
| 2-17 | m/z=670.18(C45H26N4OS=670.79) | 2-18 | m/z=670.18(C45H26N4OS=670.79) |
| 2-19 | m/z=670.18(C45H26N4OS=670.79) | 2-20 | m/z=670.18(C45H26N4OS=670.79) |
| 2-21 | m/z=670.18(C45H26N4OS=670.79) | 2-22 | m/z=670.18(C45H26N4OS=670.79) |
| 2-23 | m/z=670.18(C45H26N4OS=670.79) | 2-24 | m/z=670.18(C45H26N4OS=670.79) |
| 2-25 | m/z=654.21(C45H26N4O2=654.73) | 2-26 | m/z=654.21(C45H26N4O2=654.73) |
| 2-27 | m/z=654.21(C45H26N4O2=654.73) | 2-28 | m/z=654.21(C45H26N4O2=654.73) |
| 2-29 | m/z=654.21(C45H26N4O2=654.73) | 2-30 | m/z=654.21(C45H26N4O2=654.73) |
| 2-31 | m/z=686.16(C45H26N4S2=686.85) | 2-32 | m/z=686.16(C45H26N4S2=686.85) |
| 2-33 | m/z=686.16(C45H26N4S2=686.85) | 2-34 | m/z=686.16(C45H26N4S2=686.85) |
| 2-35 | m/z=686.16(C45H26N4S2=686.85) | 2-36 | m/z=686.16(C45H26N4S2=686.85) |
| 2-37 | m/z=670.18(C45H26N4OS=670.79) | 2-38 | m/z=670.18(C45H26N4OS=670.79) |
| 2-39 | m/z=670.18(C45H26N4OS=670.79) | 2-40 | m/z=670.18(C45H26N4OS=670.79) |
| 2-41 | m/z=670.18(C45H26N4OS=670.79) | 2-42 | m/z=670.18(C45H26N4OS=670.79) |
| 2-43 | m/z=670.18(C45H26N4OS=670.79) | 2-44 | m/z=670.18(C45H26N4OS=670.79) |
| 2-45 | m/z=670.18(C45H26N4OS=670.79) | 2-46 | m/z=670.18(C45H26N4OS=670.79) |
| 2-47 | m/z=670.18(C45H26N4OS=670.79) | 2-48 | m/z=670.18(C45H26N4OS=670.79) |
| 2-49 | m/z=760.24(C51H30N4O2=730.83) | 2-50 | m/z=760.24(C51H30N4O2=730.83) |
| 2-51 | m/z=760.24(C51H30N4O2=730.83) | 2-52 | m/z=760.24(C51H30N4O2=730.83) |
| 2-53 | m/z=760.24(C51H30N4O2=730.83) | 2-54 | m/z=760.24(C51H30N4O2=730.83) |
| 2-55 | m/z=762.19(C51H30N4S2=762.95) | 2-56 | m/z=762.19(C51H30N4S2=762.95) |
| 2-57 | m/z=762.19(C51H30N4S2=762.95) | 2-58 | m/z=762.19(C51H30N4S2=762.95) |
| 2-59 | m/z=762.19(C51H30N4S2=762.95) | 2-60 | m/z=762.19(C51H30N4S2=762.95) |
| 2-61 | m/z=746.21(C51H30N4OS=746.89) | 2-62 | m/z=746.21(C51H30N4OS=746.89) |
| 2-63 | m/z=746.21(C51H30N4OS=746.89) | 2-64 | m/z=746.21(C51H30N4OS=746.89) |
| 2-65 | m/z=746.21(C51H30N4OS=746.89) | 2-66 | m/z=746.21(C51H30N4OS=746.89) |
| 2-67 | m/z=746.21(C51H30N4OS=746.89) | 2-68 | m/z=746.21(C51H30N4OS=746.89) |
| 2-69 | m/z=746.21(C51H30N4OS=746.89) | 2-70 | m/z=746.21(C51H30N4OS=746.89) |
| 2-71 | m/z=746.21(C51H30N4OS=746.89) | 2-72 | m/z=746.21(C51H30N4OS=746.89) |
| 2-73 | m/z=730.24(C51H30N4O=730.83) | 2-74 | m/z=730.24(C51H30N4O=730.83) |
| 2-75 | m/z=730.24(C51H30N4O=730.83) | 2-76 | m/z=730.24(C51H30N4O=730.83) |
| 2-77 | m/z=730.24(C51H30N4O=730.83) | 2-78 | m/z=730.24(C51H30N4O=730.83) |
| 2-79 | m/z=762.19(C51H30N4S2=762.95) | 2-80 | m/z=762.19(C51H30N4S2=762.95) |
| 2-81 | m/z=762.19(C51H30N4S2=762.95) | 2-82 | m/z=762.19(C51H30N4S2=762.95) |
| 2-83 | m/z=762.19(C51H30N4S2=762.95) | 2-84 | m/z=762.19(C51H30N4S2=762.95) |
| 2-85 | m/z=746.21(C51H30N4OS=746.89) | 2-86 | m/z=746.21(C51H30N4OS=746.89) |
| 2-87 | m/z=746.21(C51H30N4OS=746.89) | 2-88 | m/z=746.21(C51H30N4OS=746.89) |
| 2-89 | m/z=746.21(C51H30N4OS=746.89) | 2-90 | m/z=746.21(C51H30N4OS=746.89) |
| 2-91 | m/z=746.21(C51H30N4OS=746.89) | 2-92 | m/z=746.21(C51H30N4OS=746.89) |
| 2-93 | m/z=746.21(C51H30N4OS=746.89) | 2-94 | m/z=746.21(C51H30N4OS=746.89) |
| 2-95 | m/z=746.21(C51H30N4OS=746.89) | 2-96 | m/z=746.21(C51H30N4OS=746.89) |
| 2-97 | m/z=760.24(C51H30N4O2=730.83) | 2-98 | m/z=760.24(C51H30N4O2=730.83) |
| 2-99 | m/z=760.24(C51H30N4O2=730.83) | 2-100 | m/z=760.24(C51H30N4O2=730.83) |
| 2-101 | m/z=760.24(C51H30N4O2=730.83) | 2-102 | m/z=760.24(C51H30N4O2=730.83) |
| 2-103 | m/z=762.19(C51H30N4S2=762.95) | 2-104 | m/z=762.19(C51H30N4S2=762.95) |
| 2-105 | m/z=762.19(C51H30N4S2=762.95) | 2-106 | m/z=762.19(C51H30N4S2=762.95) |
| 2-107 | m/z=762.19(C51H30N4S2=762.95) | 2-108 | m/z=762.19(C51H30N4S2=762.95) |
| 2-109 | m/z=746.21(C51H30N4OS=746.89) | 2-110 | m/z=746.21(C51H30N4OS=746.89) |
| 2-111 | m/z=746.21(C51H30N4OS=746.89) | 2-112 | m/z=746.21(C51H30N4OS=746.89) |
| 2-113 | m/z=746.21(C51H30N4OS=746.89) | 2-114 | m/z=746.21(C51H30N4OS=746.89) |
| 2-115 | m/z=746.21(C51H30N4OS=746.89) | 2-116 | m/z=746.21(C51H30N4OS=746.89) |
| 2-117 | m/z=746.21(C51H30N4OS=746.89) | 2-118 | m/z=746.21(C51H30N4OS=746.89) |
| 2-119 | m/z=746.21(C51H30N4OS=746.89) | 2-120 | m/z=746.21(C51H30N4OS=746.89) |
| 2-121 | m/z=730.24(C51H30N4O=730.83) | 2-122 | m/z=730.24(C51H30N4O=730.83) |
| 2-123 | m/z=730.24(C51H30N4O=730.83) | 2-124 | m/z=730.24(C51H30N4O=730.83) |
| 2-125 | m/z=730.24(C51H30N4O=730.83) | 2-126 | m/z=730.24(C51H30N4O=730.83) |
| 2-127 | m/z=762.19(C51H30N4S2=762.95) | 2-128 | m/z=762.19(C51H30N4S2=762.95) |
| 2-129 | m/z=762.19(C51H30N4S2=762.95) | 2-130 | m/z=762.19(C51H30N4S2=762.95) |
| 2-131 | m/z=762.19(C51H30N4S2=762.95) | 2-132 | m/z=762.19(C51H30N4S2=762.95) |
| 2-133 | m/z=746.21(C51H30N4OS=746.89) | 2-134 | m/z=746.21(C51H30N4OS=746.89) |
| 2-135 | m/z=746.21(C51H30N4OS=746.89) | 2-136 | m/z=746.21(C51H30N4OS=746.89) |
| 2-137 | m/z=746.21(C51H30N4OS=746.89) | 2-138 | m/z=746.21(C51H30N4OS=746.89) |
| 2-139 | m/z=746.21(C51H30N4OS=746.89) | 2-140 | m/z=746.21(C51H30N4OS=746.89) |
| 2-141 | m/z=746.21(C51H30N4OS=746.89) | 2-142 | m/z=746.21(C51H30N4OS=746.89) |
| 2-143 | m/z=746.21(C51H30N4OS=746.89) | 2-144 | m/z=746.21(C51H30N4OS=746.89) |
| 2-145 | m/z=654.21(C45H26N4O2=654.73) | 2-146 | m/z=654.21(C45H26N4O2=654.73) |
| 2-147 | m/z=654.21(C45H26N4O2=654.73) | 2-148 | m/z=654.21(C45H26N4O2=654.73) |
| 2-149 | m/z=654.21(C45H26N4O2=654.73) | 2-150 | m/z=654.21(C45H26N4O2=654.73) |
| 2-151 | m/z=686.16(C45H26N4S2=686.85) | 2-152 | m/z=686.16(C45H26N4S2=686.85) |
| 2-153 | m/z=686.16(C45H26N4S2=686.85) | 2-154 | m/z=686.16(C45H26N4S2=686.85) |
| 2-155 | m/z=686.16(C45H26N4S2=686.85) | 2-156 | m/z=686.16(C45H26N4S2=686.85) |
| 2-157 | m/z=670.18(C45H26N4OS=670.79) | 2-158 | m/z=670.18(C45H26N4OS=670.79) |
| 2-159 | m/z=670.18(C45H26N4OS=670.79) | 2-160 | m/z=670.18(C45H26N4OS=670.79) |
| 2-161 | m/z=670.18(C45H26N4OS=670.79) | 2-162 | m/z=670.18(C45H26N4OS=670.79) |
| 2-163 | m/z=670.18(C45H26N4OS=670.79) | 2-164 | m/z=670.18(C45H26N4OS=670.79) |
| 2-165 | m/z=670.18(C45H26N4OS=670.79) | 2-166 | m/z=670.18(C45H26N4OS=670.79) |
| 2-167 | m/z=670.18(C45H26N4OS=670.79) | 2-168 | m/z=670.18(C45H26N4OS=670.79) |
| 2-169 | m/z=730.24(C51H30N4O=730.83) | 2-170 | m/z=730.24(C51H30N4O=730.83) |
| 2-171 | m/z=730.24(C51H30N4O=730.83) | 2-172 | m/z=730.24(C51H30N4O=730.83) |
| 2-173 | m/z=730.24(C51H30N4O=730.83) | 2-174 | m/z=730.24(C51H30N4O=730.83) |
| 2-175 | m/z=762.19(C51H30N4S2=762.95) | 2-176 | m/z=762.19(C51H30N4S2=762.95) |
| 2-177 | m/z=762.19(C51H30N4S2=762.95) | 2-178 | m/z=762.19(C51H30N4S2=762.95) |
| 2-179 | m/z=762.19(C51H30N4S2=762.95) | 2-180 | m/z=762.19(C51H30N4S2=762.95) |
| 2-181 | m/z=746.21(C51H30N4OS=746.89) | 2-182 | m/z=746.21(C51H30N4OS=746.89) |
| 2-183 | m/z=746.21(C51H30N4OS=746.89) | 2-184 | m/z=746.21(C51H30N4OS=746.89) |
| 2-185 | m/z=746.21(C51H30N4OS=746.89) | 2-186 | m/z=746.21(C51H30N4OS=746.89) |
| 2-187 | m/z=762.19(C51H30N4S2=762.95) | 2-188 | m/z=796.23(C55H32N4OS=796.95) |
| 2-189 | m/z=746.21(C51H30N4OS=746.89) | 2-190 | m/z=796.23(C55H32N4OS=796.95) |
| 2-191 | m/z=751.25(C51H25D5N4OS=751.92) | 2-192 | m/z=802.28(C55H38N4OS=803.00) |
| 2-193 | m/z=780.25(C55H32N4O2=780.89) | 2-194 | m/z=806.27(C57H34N4O2=806.93) |
| 2-195 | m/z=816.33(C57H24D10N4O2=816.99) | 2-196 | m/z=918.39(C65H50N4O2=919.14) |
| 2-197 | m/z=822.25(C57H34N4OS=822.99) | 2-198 | m/z=822.25(C57H34N4OS=822.99) |
| 2-199 | m/z=896.26(C63H36N4OS=897.07) | 2-200 | m/z=898.28(C63H38N4OS=899.08) |
| 3-3 | m/z=560.23 (C42H28N2=560.70) | 3-4 | m/z=560.23 (C42H28N2=560.70) |
| 3-7 | m/z=636.26 (C48H32N2=636.80) | 3-31 | m/z=636.26 (C48H32N2=636.80) |
| 3-32 | m/z=636.26 (C48H32N2=636.80) | 4-2 | m/z=666.84 (C48H30N2=666.21) |

**[Table 7]**

| Compound | ¹H NMR (CDCl₃, 200 Mz) |
|---|---|
| 1-1 | δ=8.55(1H, d), 8.31(1H, d), 7.76~8.08(10H, m), 7.50~7.62(8H, m), 7.31~7.39(5H, m), 7.16(1H, t) |
| 1-5 | δ=8.55(1H, d), 8.31(1H, d), 7.31~8.03(23H, m), 7.16(1H, t) |
| 1-18 | δ=8.55(1H, d), 8.45(2H, d), 8.31(1H, d), 8.12(1H, s), 7.93~8.03(8H, m), 7.49~7.74(10H, m), 7.35(1H, t), 7.16(1H, t) |
| 1-30 | δ=8.55 (1H, d), 8.31 (2H, s), 7.69~7.98 (15H, m), 7.31~7.57 (14H, m), 7.16(1H, t) |
| 1-37 | δ=8.55(2H, d), 8.45(1H, d), 8.31(1H, d), 7.91~7.98(6H, m), 7.31~7.82(15H, m), 7.16(1H, t) |
| 1-43 | δ=8.55(1H, d), 8.45(1H, d), 8.31(1H, d), 7.91~8.03(7H, m), 7.31~7.82(15H, m), 7.16(1H, t) |
| 1-53 | δ=8.55(1H, d), 7.54~7.98(19H, m), 7.31~7.39(5H, m), 7.16(1H, t) |
| 1-55 | δ=8.55(1H, d), 7.69~7.98(17H, m), 7.31~7.57(11H, m), 7.16(1H, t) |
| 1-80 | δ=9.05~9.08(2H, m), 8.55(1H, d), 8.33(1H, d), 8.25(1H, d), 7.52~7.98(21H, m), 7.31~7.39(6H, m), 7.16(1H, t) |
| 1-82 | δ=8.55(2H, d), 8.45(1H, d), 7.31~7.98(22H, m), 7.16(1H, t) |
| 1-97 | δ=8.45(1H, d), 8.20~8.30(3H, m), 8.13(1H, d), 7.39~7.98(25H, m) |
| 1-104 | δ=8.55(1H, d), 7.76~7.98(10H, m), 7.31~7.62(14H, m), 7.16(1H, t) |
| 1-141 | δ=8.55(1H, d), 8.45(1H, d), 8.20~8.24(2H, m), 8.08(1H, d), 7.88(6H, m), 7.31~7.62(14H, m), 7.16(1H, t) |
| 1-172 | δ=8.55(1H, d), 8.45(1H, d), 8.20~8.29(3H, m), 7.58~7.99(8H, m), 7.31~7.62(12H, m), 7.16(1H, t) |
| 2-1 | δ=8.55(1H, d), 8.19(1H, d), 7.82~8.08(12H, m), 7.50~7.58(5H, m), 7.31~7.39(5H, m), 7.16(1H, t) |
| 2-5 | δ=8.55(1H, d), 8.19(1H, d), 7.91~7.98(8H, m), 7.76~7.82(3H, m), 7.69(1H, d), 7.50~7.57(5H, m), 7.31~7.39(5H, m), 7.16~7.20(2H, m) |
| 2-9 | δ=8.55(2H, d), 8.45(2H, d), 8.19~8.24(3H, m), 7.91~7.94(9H, m), 7.70(1H, t), 7.49~7.58(6H, m), 7.35(1H, t), 7.16~7.20(2H, m) |
| 2-19 | δ=8.55(1H, d), 8.45(1H, d), 8.19(1H, d), 7.91~7.98(10H, m), 7.76~7.82(2H, m), 7.68(1H, t), 7.49~7.58(5H, m), 7.31~7.39(3H, m), 7.16~7.20(2H, m) |
| 2-28 | δ=8.55(1H, d), 8.19~8.24(3H, m), 7.50-8.03(15H, m), 7.31~7.39(5H, m), 7.16~7.20(2H, m) |
| 2-37 | δ=8.55(1H, d), 8.56(1H, d), 8.19~8.24(3H, m), 7.92~8.03(5H, m), 7.31~7.70(13H, m), 7.16~7.20(2H, m) |
| 2-49 | δ=8.55(1H, d), 8.31(1H, d), 7.75~8.08(14H, m), 7.31~7.54(11H, m), 7.16(1H, t) |
| 2-67 | δ=8.55(1H, d), 8.45(1H, d), 8.31(1H, d), 7.91~8.03(7H, m), 7.75~7.82(6H, m), 7.31~7.56(9H, m), 7.16(1H, t) |
| 2-97 | δ=8.55(1H, d), 7.75(14H, m), 7.31~7.54(11H, m), 7.16(1H, m) |
| 2-109 | δ=8.55(1H, d), 8.45(1H, d), 7.89~8.03(11H, m), 7.70~7.82(6H, m), 7.31~7.56(9H, m), 7.16(1H, t) |
| 2-124 | δ=8.55(1H, d), 8.21~8.24(2H, m), 7.39~8.03(26H, m), 7.16(1H, t) |
| 3-3 | δ=8.55 (1H, d), 8.30 (1H, d), 8.21-8.13 (3H, m), 7.99-7.89 (4H, m), 7.77-7.35 (17H, m), 7.20-7.16 (2H, m) |
| 3-4 | δ=8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 3-7 | δ=8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 3-31 | δ=8.55 (1H, d), 8.30 (1H, d), 8.21-8.13 (4H, m), 7.99-7.89 (4H, m), 7.77-7.35 (20H, m), 7.20-7.16 (2H, m) |
| 3-32 | δ=8.55 (1H, d), 8.30 (1H, d), 8.21-8.13 (3H, m), 7.99-7.89 (8H, m), 7.77-7.35 (17H, m), 7.20-7.16 (2H, m) |
| 4-2 | δ=8.55(1H, d), 8.45(1H, d), 8.30(1H, d), 8.19(1H, d), 8.13(1H, d), 8.00~7.89(6H, m), 7.77(2H, m), 7.62~7.35(15H, m), 7.20~7.16(2H, m) |

### [Experimental Example 1]

### 1) Manufacture of Organic Light Emitting Device

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, a compound of the following Table 8 was deposited to 400 Å as a host, and Ir(ppy)₃, a green phosphorescent dopant, was doped and deposited by 7% of the deposited thickness of the light emitting layer. After that, BCP was deposited to 60 Å as a hole blocking layer, and Alq₃ was deposited to 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 133.3*10⁻⁸ Pa (10⁻⁸ torr) to 133.3*10⁻⁶ Pa (10⁻⁶ torr) for each material to be used in the OLED manufacture.

### 2) Evaluation on Organic Light Emitting Device

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime of the organic light emitting devices manufactured according to the present disclosure are as shown in the following Table 8.

### [Comparative Example 1]

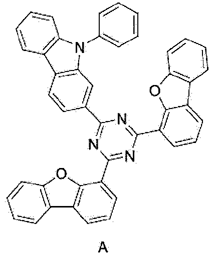

### [Comparative Example 2]

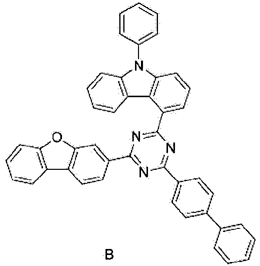

### [Comparative Example 3]

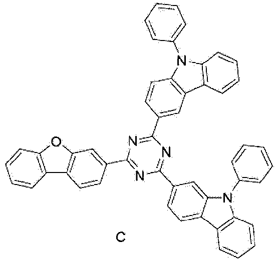

### [Comparative Example 4]

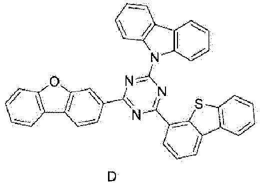

### [Comparative Example 5]

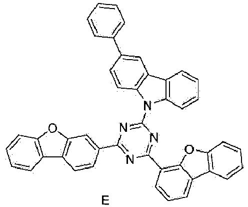

### [Comparative Example 6]

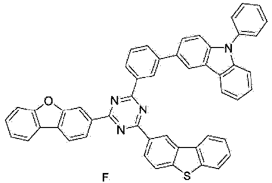

### [Comparative Example 7]

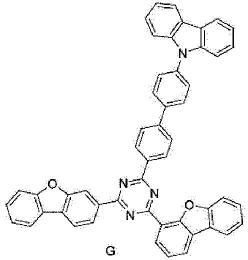

### [Comparative Example 8]

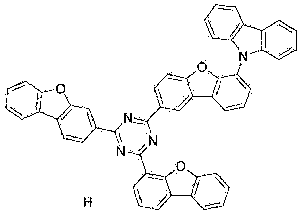

### [Comparative Example 9]

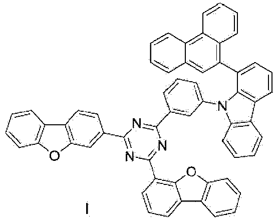

**[Table 8]**

| | Light Emitting Layer Compound | Driving Voltage (V) | Efficie ncy (cd/A) | Color Coordinate (x, y) | Lifet ime (T₉₀) |
|---|---|---|---|---|---|
| Comparative Example 1 | A | 5.24 | 48.9 | (0.256, 0.717) | 78 |
| Comparative Example 2 | B | 5.74 | 45.9 | (0.266, 0.686) | 55 |
| Comparative Example 3 | C | 5.33 | 49.2 | (0.267, 0.727) | 35 |
| Comparative Example 4 | D | 5.31 | 45.7 | (0.263, 0.693) | 28 |
| Comparative Example 5 | E | 5.72 | 46.7 | (0.273, 0.684) | 33 |
| Comparative Example 6 | F | 5.55 | 50.2 | (0.277, 0.674) | 44 |
| Comparative Example 7 | G | 5.23 | 59.2 | (0.271, 0.686) | 49 |
| Comparative Example 8 | H | 5.82 | 47.4 | (0.273, 0.682) | 82 |
| Comparative Example 9 | I | 5.99 | 48.7 | (0.278, 0.686) | 42 |
| Comparative Example 10 | 3-3 | 4.83 | 50.9 | (0.233, 0.703) | 60 |
| Comparative Example 11 | 3-4 | 4.69 | 69.2 | (0.231, 0.712) | 66 |
| Comparative Example 12 | 3-7 | 5.21 | 57 | (0.247, 0.727) | 62 |
| Comparative | 3-31 | 4.75 | 51.2 | (0.254, 0.724) | 58 |
| Example 13 | | | | | |
| Comparative Example 14 | 3-32 | 4.48 | 70.2 | (0.241, 0.714) | 59 |
| Comparative Example 15 | 4-2 | 4.83 | 61.9 | (0.233, 0.703) | 75 |
| Example 1 | 1-01 | 3.99 | 62.9 | (0.276, 0.671) | 138 |
| Example 2 | 1-05 | 4.12 | 64.2 | (0.272, 0.666) | 164 |
| Example 3 | 1-18 | 3.89 | 73.4 | (0.276, 0.670) | 182 |
| Example 4 | 1-30 | 3.6 | 72.9 | (0.280, 0.673) | 173 |
| Example 5 | 1-37 | 3.63 | 69.3 | (0.281, 0.678) | 169 |
| Example 6 | 1-43 | 3.92 | 69.7 | (0.281, 0.672) | 178 |
| Example 7 | 1-53 | 4.38 | 59 | (0.240, 0.712) | 171 |
| Example 8 | 1-55 | 4.33 | 69.1 | (0.282, 0.679) | 142 |
| Example 9 | 1-80 | 4.42 | 67.5 | (0.284, 0.680) | 149 |
| Example 10 | 1-82 | 4.24 | 73.3 | (0.276, 0.678) | 165 |
| Example 11 | 1-97 | 4.11 | 79.3 | (0.282, 0.676) | 152 |
| Example 12 | 1-104 | 4.32 | 65.2 | (0.276, 0.677) | 129 |
| Example 13 | 1-141 | 3.87 | 71.8 | (0.278, 0.670) | 138 |
| Example 14 | 1-172 | 4.23 | 69.3 | (0.279, 0.670) | 128 |
| Example 15 | 2-1 | 4.12 | 70.2 | (0.279, 0.674) | 123 |
| Example 16 | 2-5 | 4.39 | 67.3 | (0.231, 0.714) | 198 |
| Example 17 | 2-9 | 4.42 | 72.2 | (0.231, 0.711) | 111 |
| Example 18 | 2-19 | 4.55 | 62.4 | (0.246, 0.717) | 196 |
| Example 19 | 2-28 | 4.53 | 67.6 | (0.275, 0.673) | 126 |
| Example 20 | 2-37 | 4.73 | 80.4 | (0.279, 0.674) | 136 |
| Example 21 | 2-49 | 4.45 | 55.8 | (0.251, 0.713) | 176 |
| Example 22 | 2-67 | 3.8 | 67.1 | (0.283, 0.680) | 141 |
| Example 23 | 2-97 | 4.76 | 74.2 | (0.279, 0.688) | 131 |
| Example 24 | 2-109 | 4.85 | 66.8 | (0.285, 0.669) | 139 |
| Example 25 | 2-124 | 4.87 | 64.8 | (0.284, 0.677) | 159 |

As seen from the results of Table 8, it was identified that the organic electroluminescent device using the compound of Chemical Formula 1 of the present disclosure as the light emitting layer material had significantly enhanced driving voltage, light emission efficiency and lifetime compared to Comparative Examples 1 to 15.

Particularly, when heteroaryl bonds to Chemical Formula 1 at the same position as in Comparative Example 1, the LUMO orbital is widened. In addition, when amine of N-heteroaryl directly bonds as in Comparative Examples 4 and 5, the HOMO/LUMO orbitals overlap. It is identified that this is disadvantageous compared to the light emitting layer material of the present disclosure stable in terms of charge balance by bonding at different positions.

### [Experimental Example 2]

### 1) Manufacture of Organic Light Emitting Device

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl (phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, two types of compounds described in the following Table 9 were pre-mixed and then deposited to 400 Å in one source of supply as a host, and Ir(ppy)₃, a green phosphorescent dopant, was doped and deposited by 7% of the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 133.3*10⁻⁸ Pa (10⁻⁸ torr) to 133.3*10⁻⁶ Pa (10⁻⁶ torr) for each material to be used in the OLED manufacture.

### 2) Evaluation on Organic Light Emitting Device

For each of the organic electroluminescent devices manufactured as above, electroluminescent (EL) properties were measured using M7000 manufactured by McScience Inc., and with the measurement results, T₉₀ was measured when standard luminance was 6,000 cd/m² through a lifetime measurement system (M6000) manufactured by McScience Inc..

Results of measuring driving voltage, light emission efficiency, color coordinate (CIE) and lifetime (T₉₀) of the organic light emitting devices manufactured according to the present disclosure are as shown in the following Table 9.

**[Table 9]**

| | Light Emitting Layer Compound | Ratio (Weight Ratio) | Driving Voltage (V) | Effici ency (cd/A) | Color Coordinate (x, y) | Life time (T₉₀) |
|---|---|---|---|---|---|---|
| Example 26 | | 1:4 | 4.44 | 78.5 | (0.272, 0.666) | 374 |
| Example 27 | | 1:3 | 5.06 | 66.5 | (0.273, 0.665) | 395 |
| Example 28 | | 1:2 | 4.69 | 79.2 | (0.270, 0.068) | 442 |
| Example 29 | 1-1:3-3 | 1:1 | 4.63 | 74.5 | (0.271, 0.663) | 438 |
| Example 30 | | 2:1 | 4.86 | 75.6 | (0.274, 0.681) | 419 |
| Example 31 | | 3:1 | 4.9 | 74.9 | (0.278, 0.677) | 371 |
| Example 32 | | 4:1 | 5.17 | 79.9 | (0.280, 0.675) | 362 |
| Example 33 | | 1:2 | 4.15 | 80.4 | (0.276, 0.0674) | 450 |
| Example 34 | 1-37:3-4 | 1:1 | 4.73 | 72.5 | (0.279, 0.0674) | 441 |
| Example 35 | | 2:1 | 4.76 | 74.2 | (0.279, 0.688) | 428 |
| Example 36 | | 1:2 | 4.18 | 67.9 | (0.280, 0.670) | 501 |
| Example 37 | 1-97:3-7 | 1:1 | 4.87 | 64.8 | (0.284, 0.677) | 469 |
| Example 38 | | 2:1 | 4.94 | 64 | (0.277, 0.680) | 458 |
| Example 39 | | 1:2 | 4.31 | 68.7 | (0.273, 0.677) | 477 |
| Example 40 | 2-5:3-31 | 1:1 | 4.87 | 64.8 | (0.284, 0.677) | 467 |
| Example 41 | | 2:1 | 4.94 | 64 | (0.277, 0.680) | 452 |
| Example 42 | 2-28:3-32 | 1:2 | 3.89 | 79.6 | (0.279, 0.676) | 438 |
| Example 43 | | 1:1 | 4.55 | 80.4 | (0.279, 0.683) | 426 |
| Example 44 | | 2:1 | 4.12 | 73.5 | (0.276, 0.680) | 409 |
| Example 45 | 2-124:3-33 | 1:2 | 3.84 | 78.8 | (0.274, 0.680) | 460 |
| Example 46 | | 1:1 | 4.06 | 67.6 | (0.279, 0.677) | 451 |
| Example 47 | | 2:1 | 4.09 | 58.5 | (0.280, 0.683) | 444 |
| Example 48 | | 1:2 | 4.01 | 69.5 | (0.288, 0.0674) | 459 |
| Example 49 | 2-1:4-2 | 1:1 | 4.50 | 66.6 | (0.285, 0.670) | 455 |
| Example 50 | | 2:1 | 4.65 | 64.9 | (0.291, 0.671 | 431 |
| Comparative Example 16 | | 1:2 | 4.99 | 72.9 | (0.276, 0.671) | 188 |
| Comparative Example 17 | C:3-3 | 1:1 | 5.12 | 54.2 | (0.272, 0.666) | 167 |
| Comparative Example 18 | | 2:1 | 5.33 | 52.2 | (0.274, 0.0667) | 139 |
| Comparative | D:3-4 | 1:2 | 6.33 | 59.1 | (0.282, 0.679) | 220 |
| Example 19 | | | | | | |
| Comparative Example 20 | | 1:1 | 6.42 | 57.5 | (0.284, 0.680) | 195 |
| Comparative Example 21 | | 2:1 | 6.21 | 51.9 | (0.277, 0.679) | 191 |
| Comparative Example 22 | | 1:2 | 5.01 | 60.2 | (0.275, 0.673) | 212 |
| Comparative Example 23 | G:3-31 | 1:1 | 5.99 | 59.1 | (0.277, 0.674) | 210 |
| Comparative Example 24 | | 2:1 | 5.14 | 57.4 | (0.277, 0.677) | 199 |

As seen from the results of Table 8 and Table 9, effects of more superior efficiency and lifetime are obtained when including the compound of Chemical Formula 1 and the compound of Chemical Formula 3 at the same time. Such results may lead to a forecast that an exciplex phenomenon occurs when including the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime. In the disclosure of the present application, the heterocyclic compound of Chemical Formula 1 and the compound of Chemical Formula 3 are used as the light emitting layer host, and it was identified that excellent device properties were obtained when using the compound of Chemical Formula 3 as a donor role and the heterocyclic compound of Chemical Formula 1 as an acceptor role.

## Claims

1. A heterocyclic compound of the following Chemical Formula 1: 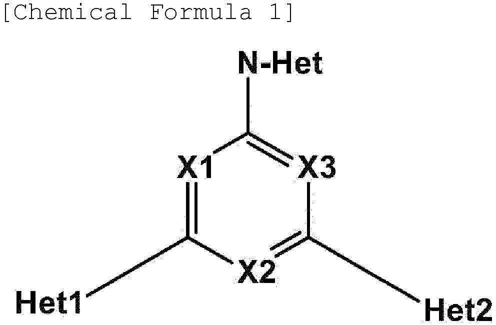 in Chemical Formula 1,
X1 to X3 are N,
N-Het is represented by the following Chemical Formula N-1 or N-2, 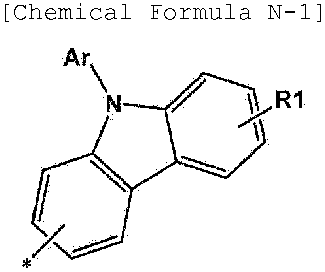 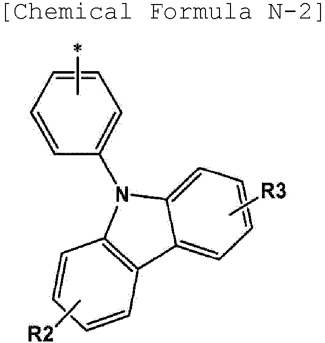
in Chemical Formulae N-1 and N-2,
R1 is hydrogen; deuterium; a halogen group; a cyano group; a substituted or unsubstituted silyl group; a substituted or unsubstituted phosphine oxide group; a substituted or unsubstituted C1 to C60 alkyl group; a substituted or unsubstituted C3 to C60 cycloalkyl group; a substituted or unsubstituted bicyclic or lower aryl group; a substituted or unsubstituted terphenyl group; a substituted or unsubstituted fluorenyl group; a substituted or unsubstituted tetracyclic or higher aryl group; or a substituted or unsubstituted C2 to C60 heteroaryl group,
R2 and R3 are each independently hydrogen; deuterium; a substituted or unsubstituted phenyl group; a substituted or unsubstituted biphenyl group; or a substituted or unsubstituted terphenyl group,
Ar is a substituted or unsubstituted C6 to C60 aryl group; or a substituted or unsubstituted C2 to C60 heteroaryl group, and
Het1 is represented by the following Chemical Formula **H-**1, and Het2 is represented by the following Chemical Formula **H-**2, 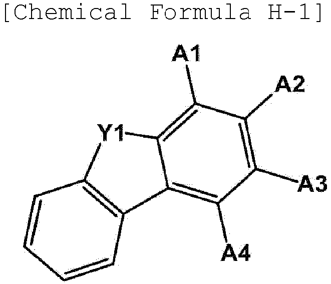 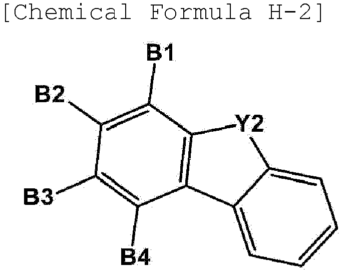
in Chemical Formulae H-1 and H-2,
Y1 and Y2 are each independently O or S,
A1 to A4 and B1 to B4 each independently bond to Chemical Formula 1, or are hydrogen; or deuterium, and
Chemical Formula 1 bonds to any one of A1 to A4 of Chemical Formula H-1 and any one of B1 to B4 of Chemical Formula H-2, which is represented by Am-Bn, m and n are each 1, 2, 3 or 4, and m and n are different.

2. The heterocyclic compound of Claim 1, wherein Ar is a substituted or unsubstituted phenyl group; a substituted or unsubstituted biphenyl group; a substituted or unsubstituted terphenyl group; a substituted or unsubstituted naphthyl group; a substituted or unsubstituted phenanthrenyl group; a substituted or unsubstituted triphenylenyl group; or a substituted or unsubstituted pyrenyl group.

3. The heterocyclic compound of Claim 1, wherein Chemical Formula 1 is represented by any one of the following compounds:

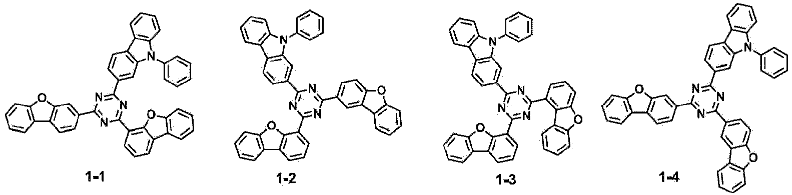

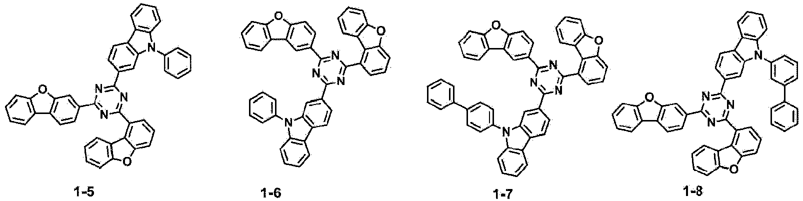

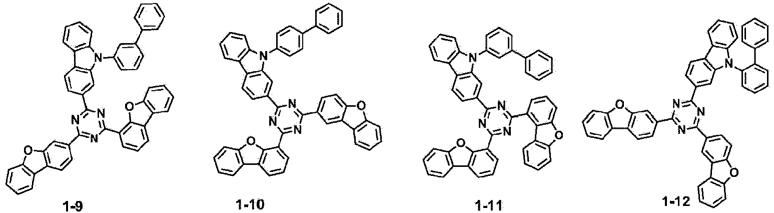

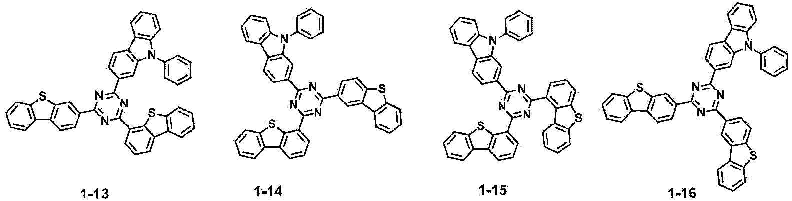

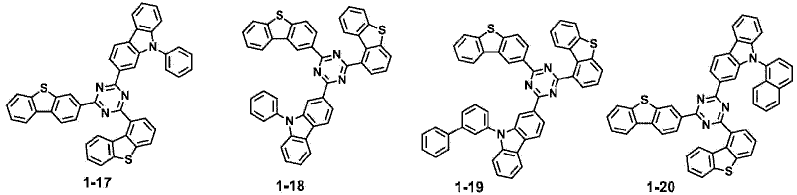

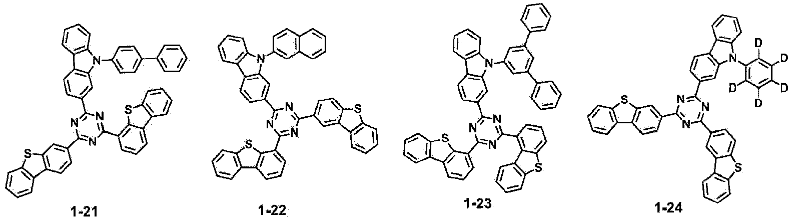

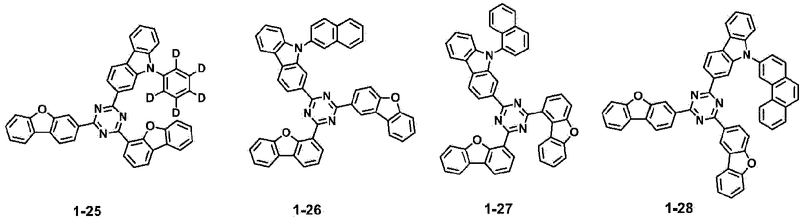

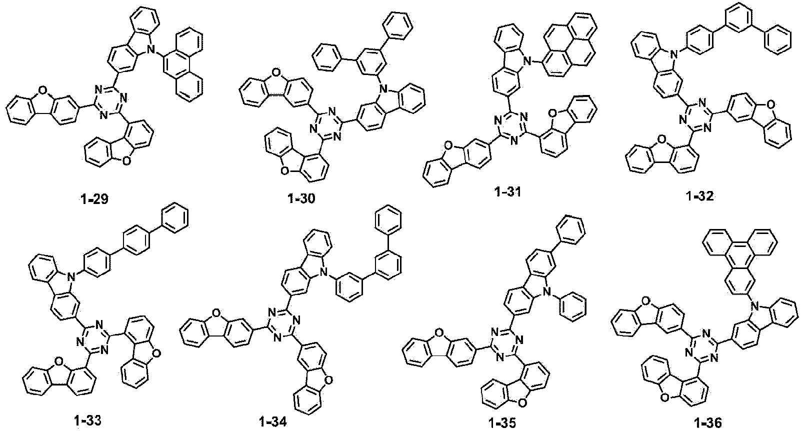

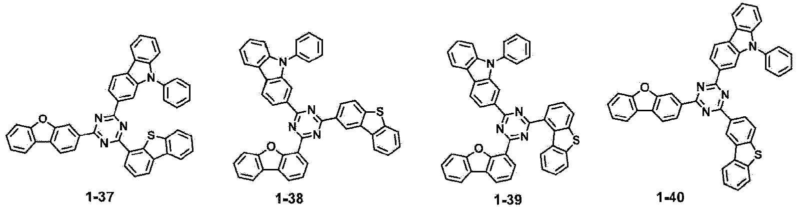

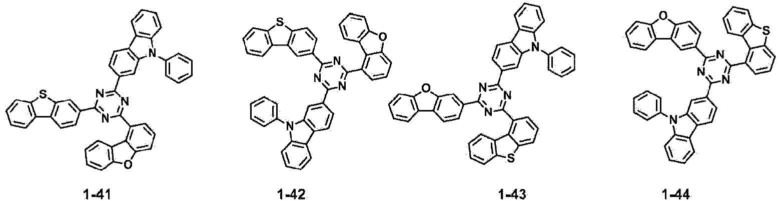

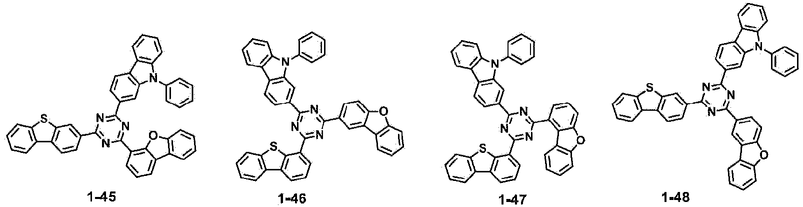

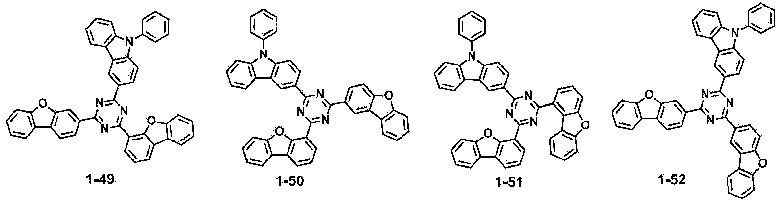

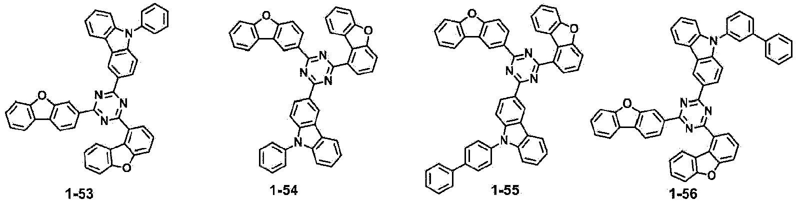

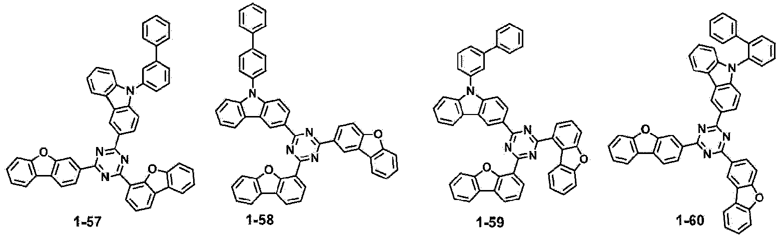

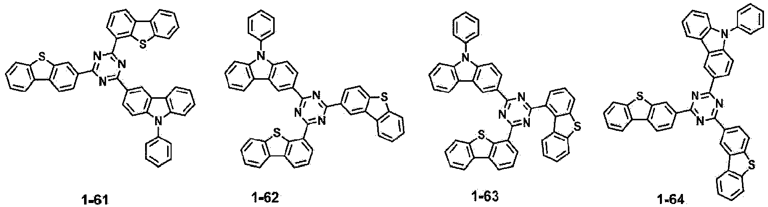

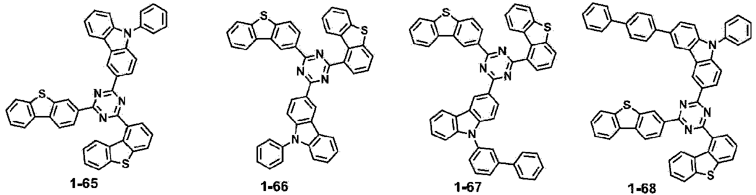

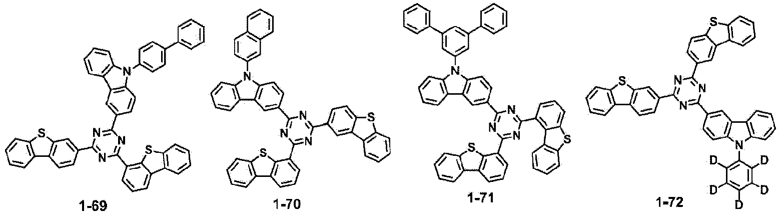

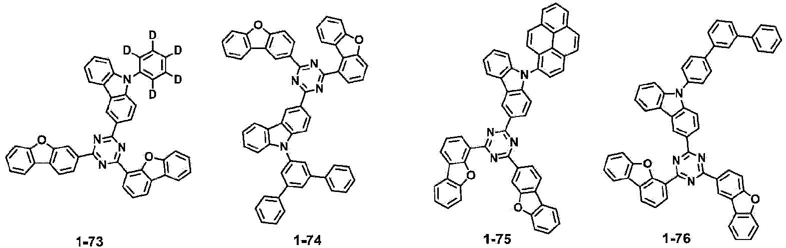

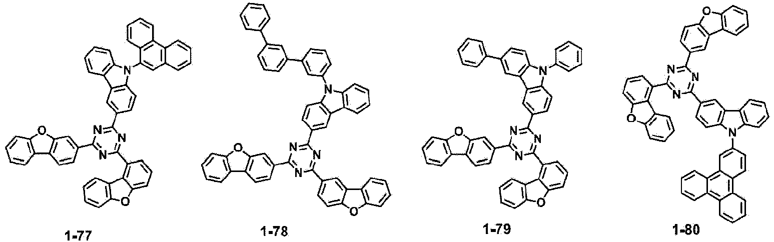

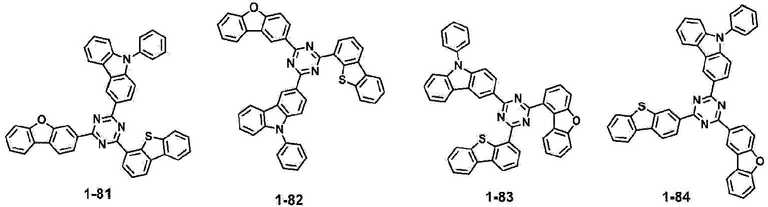

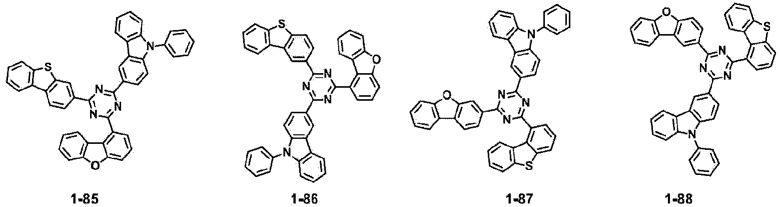

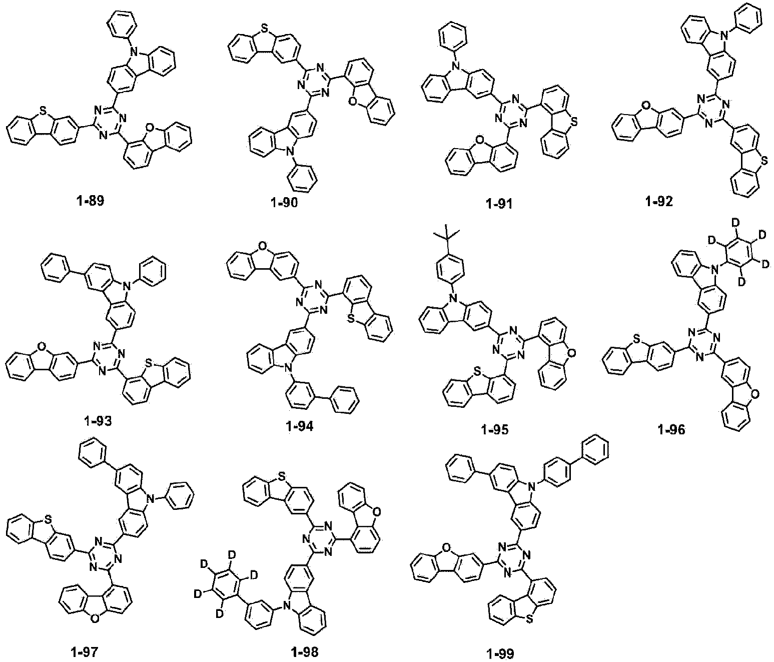

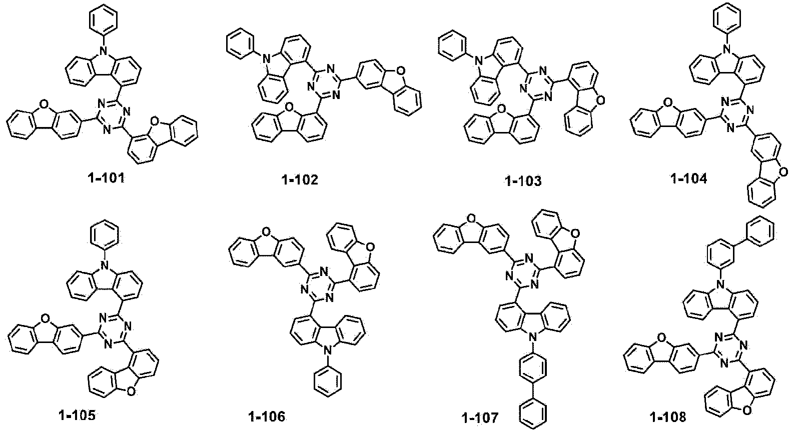

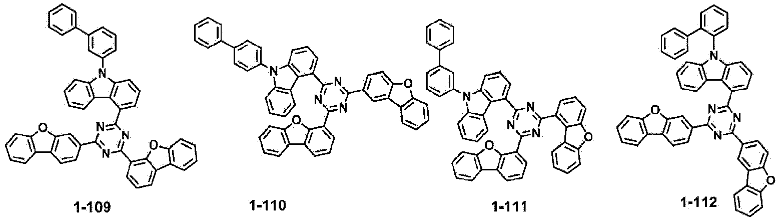

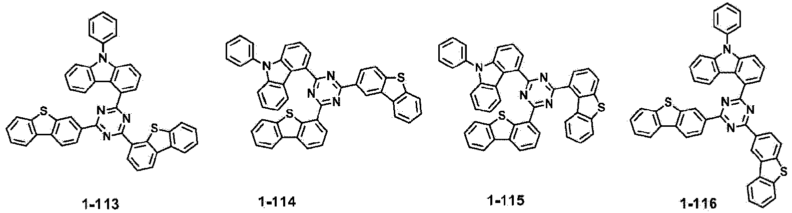

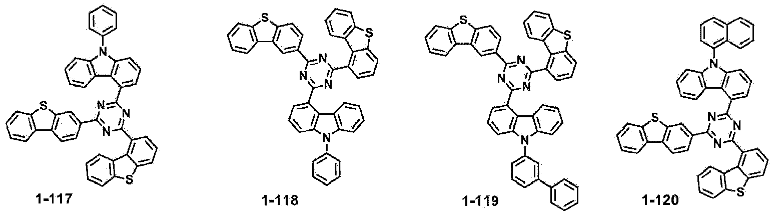

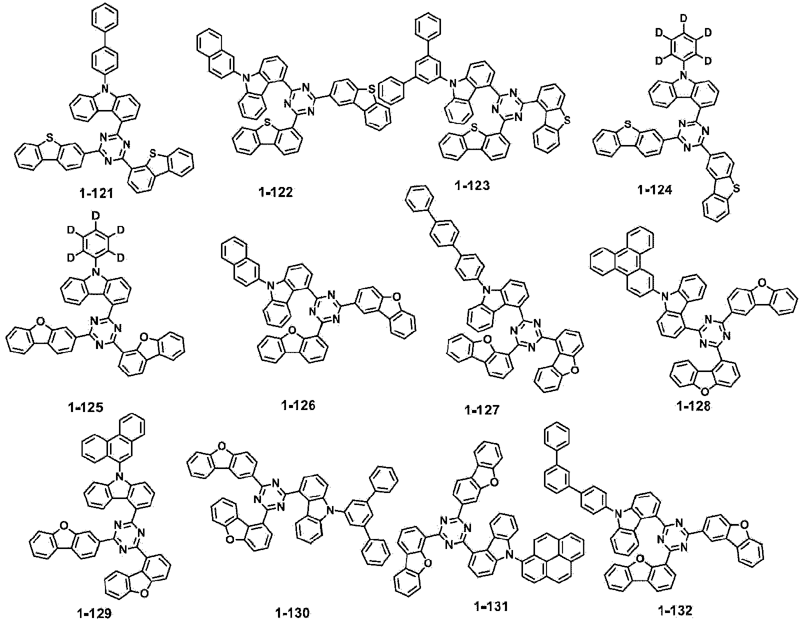

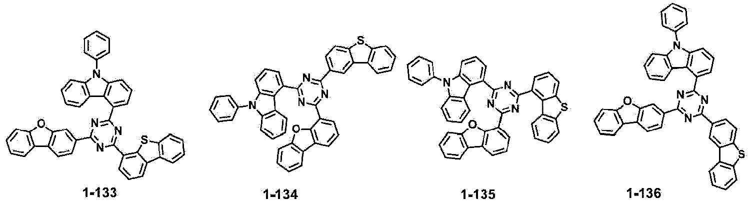

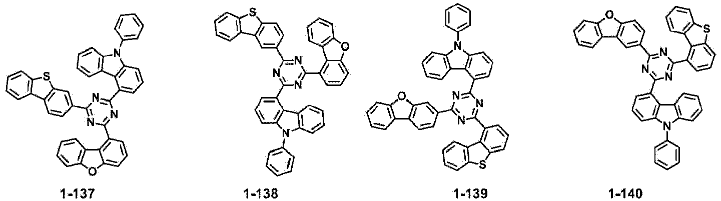

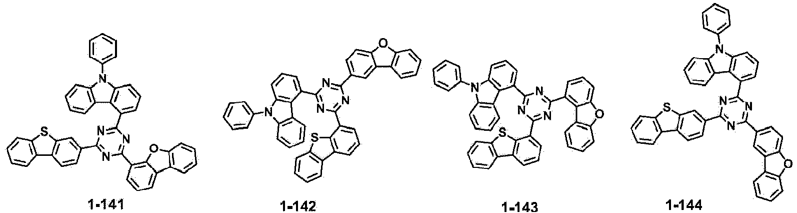

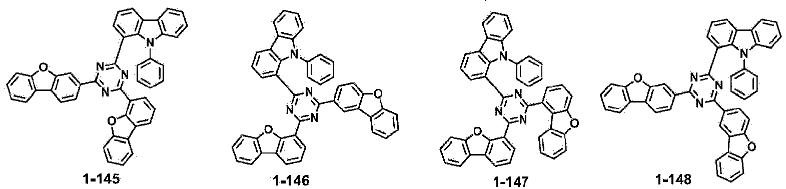

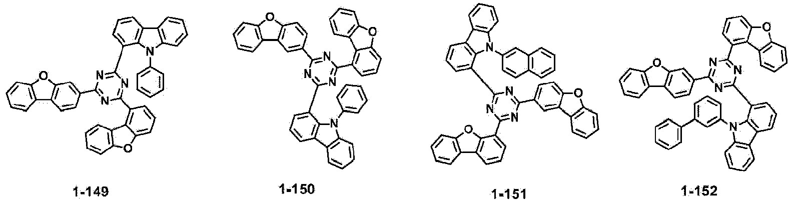

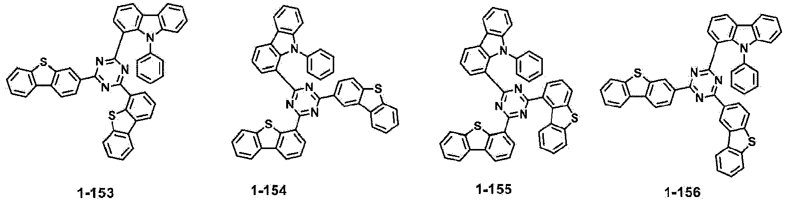

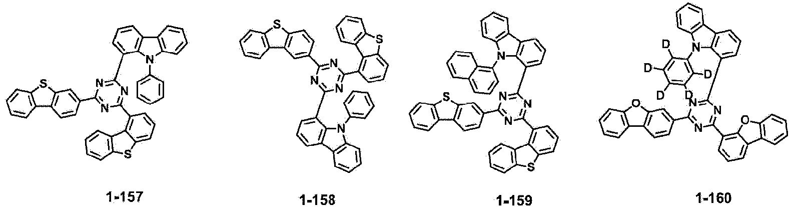

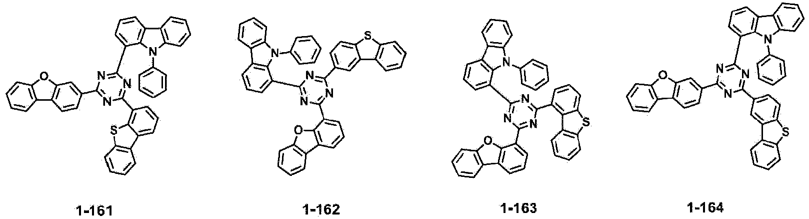

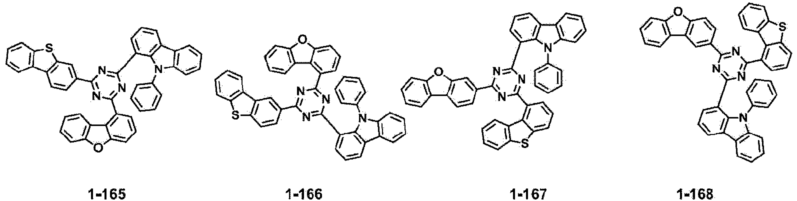

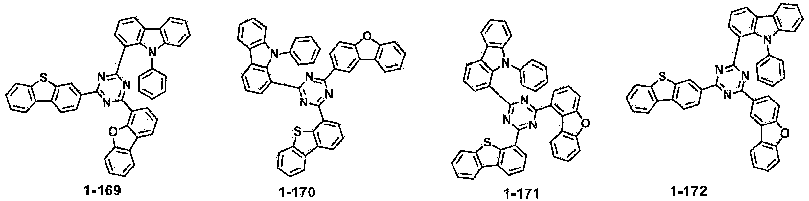

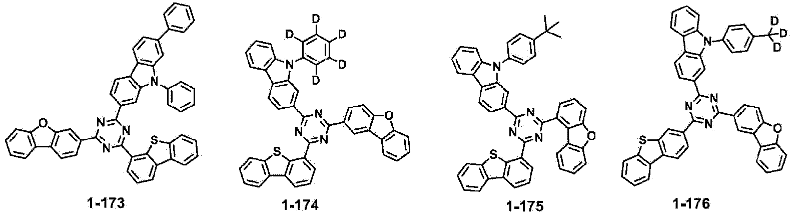

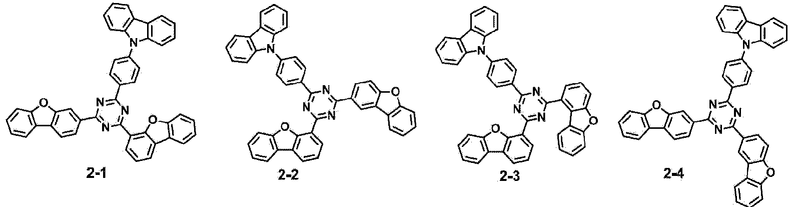

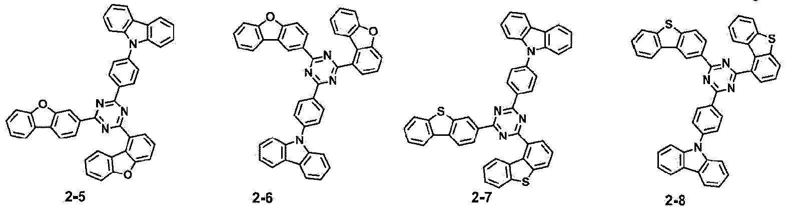

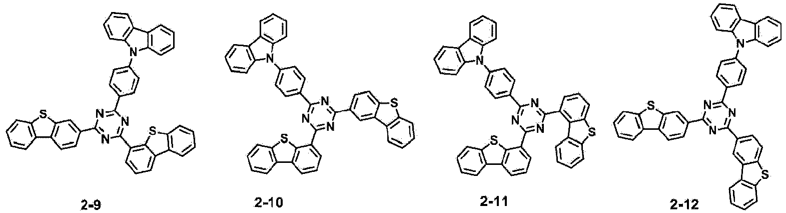

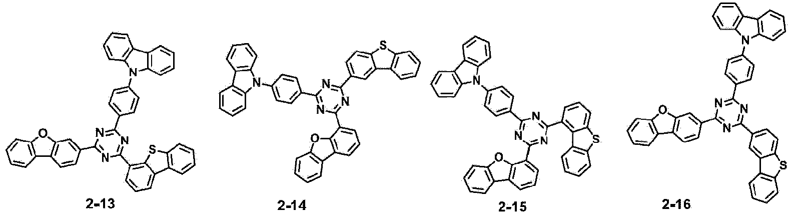

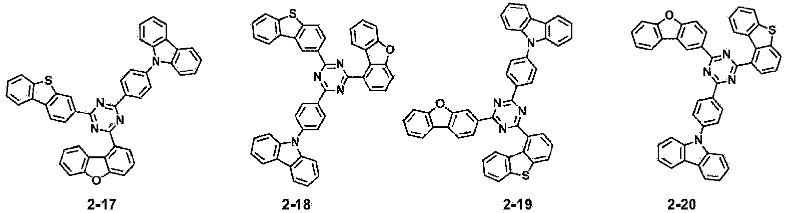

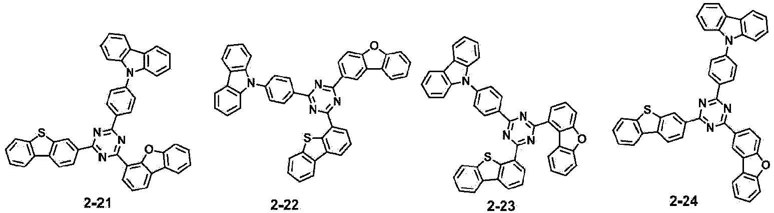

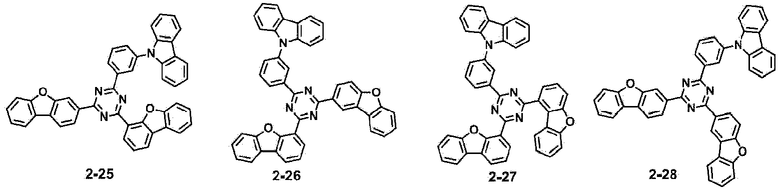

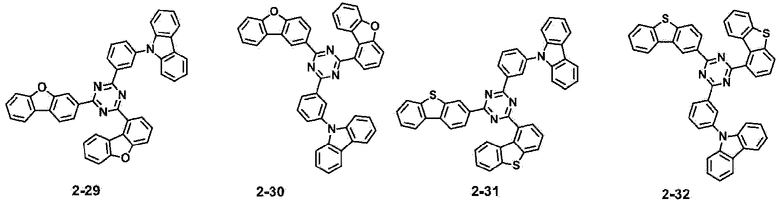

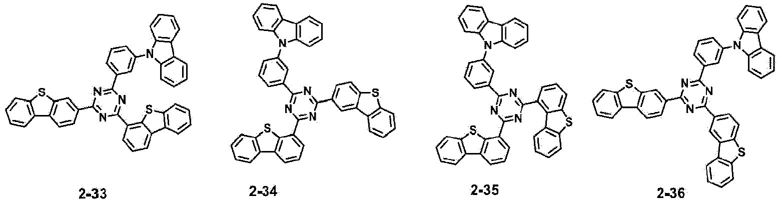

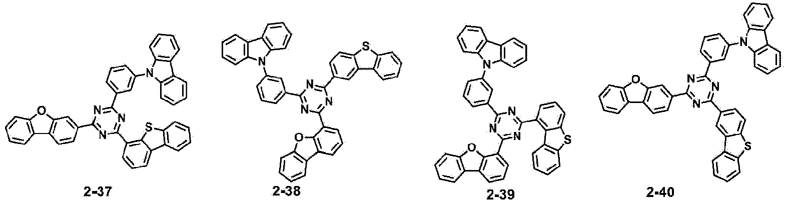

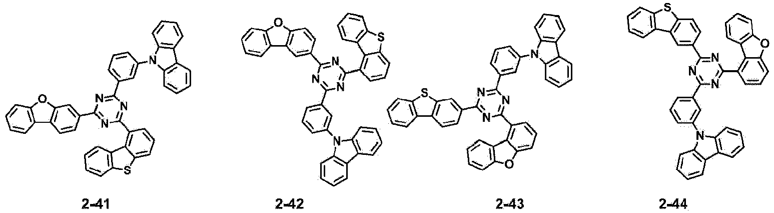

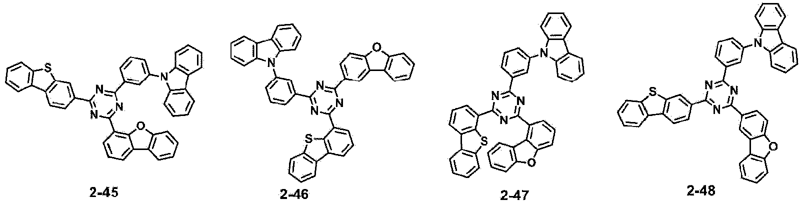

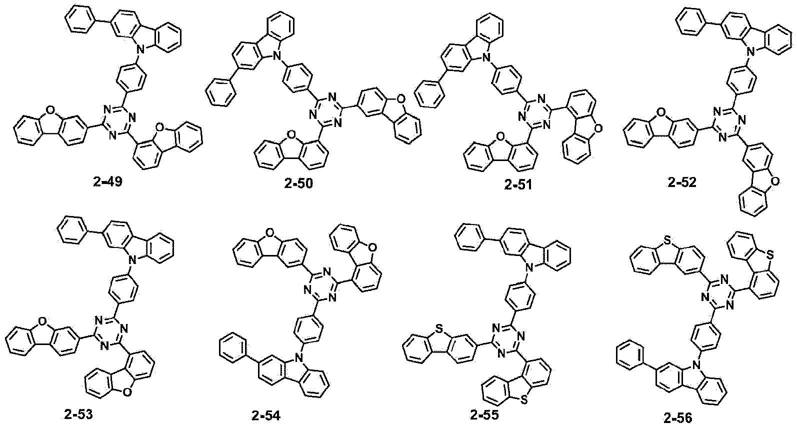

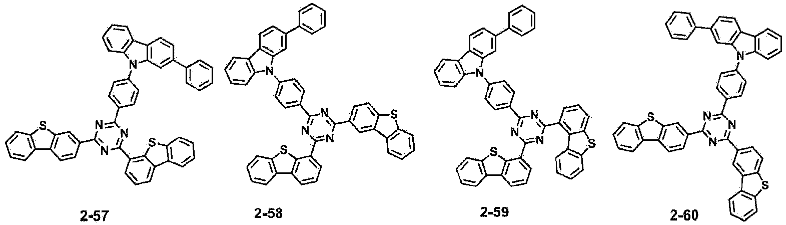

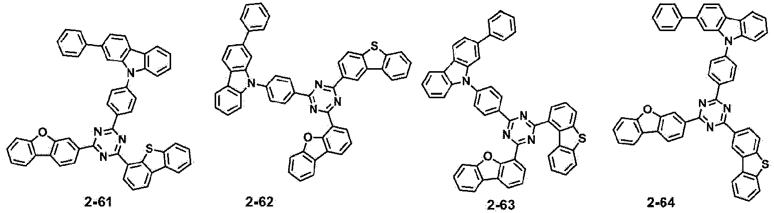

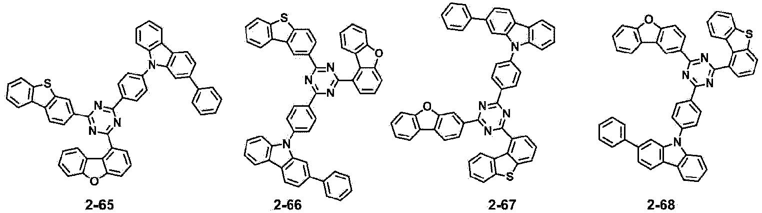

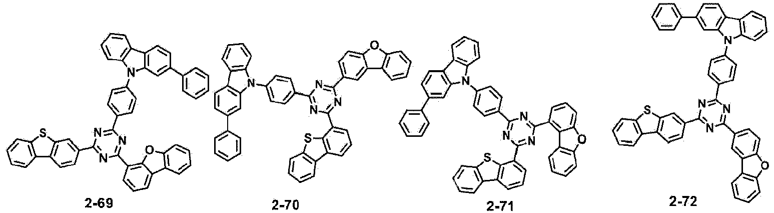

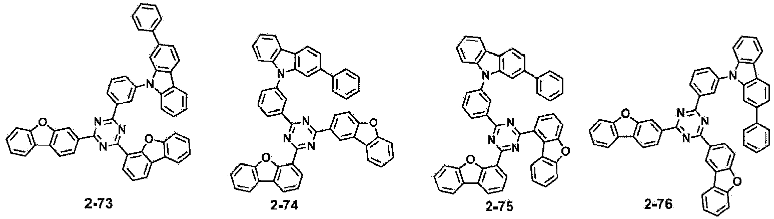

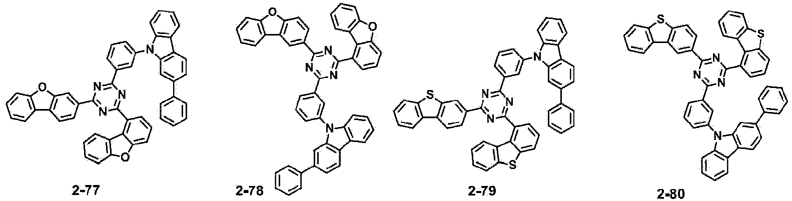

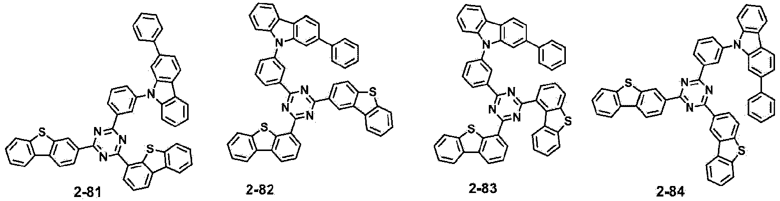

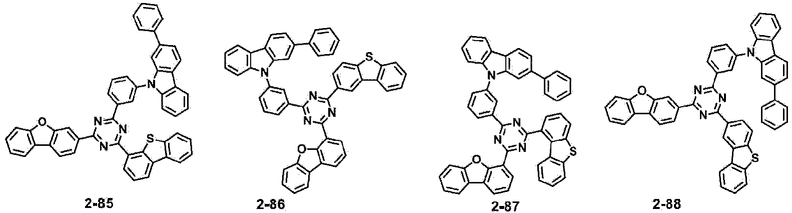

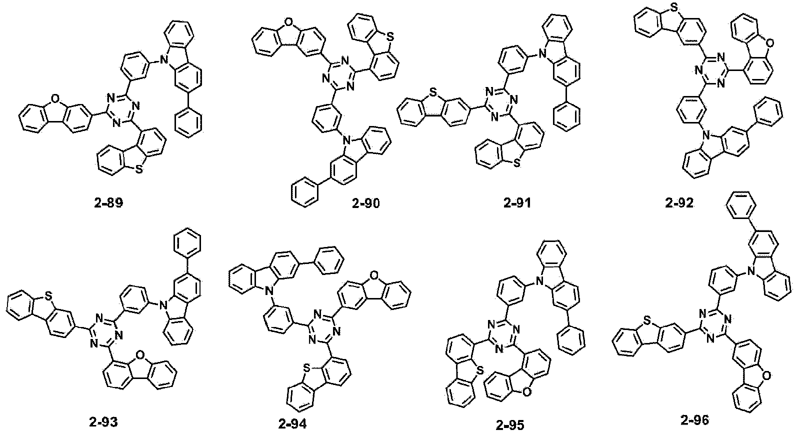

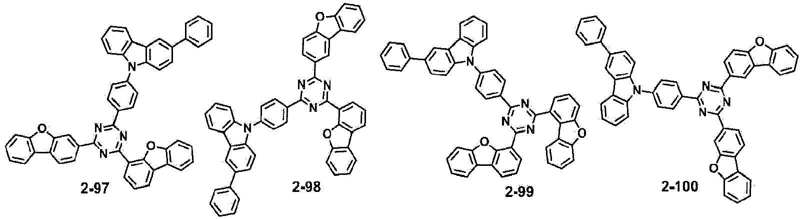

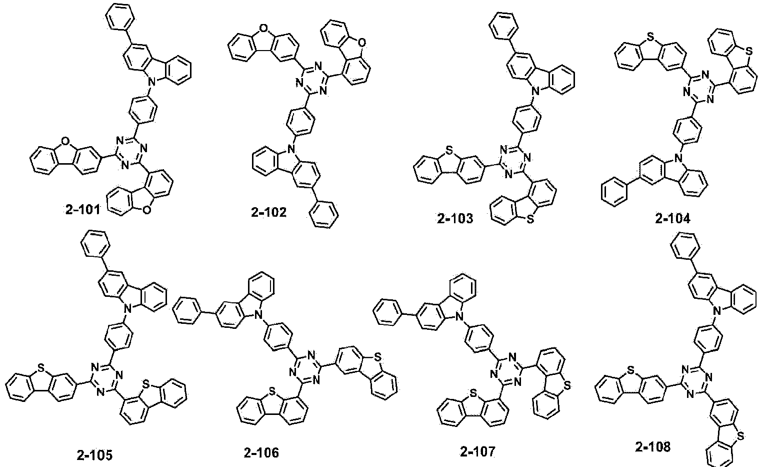

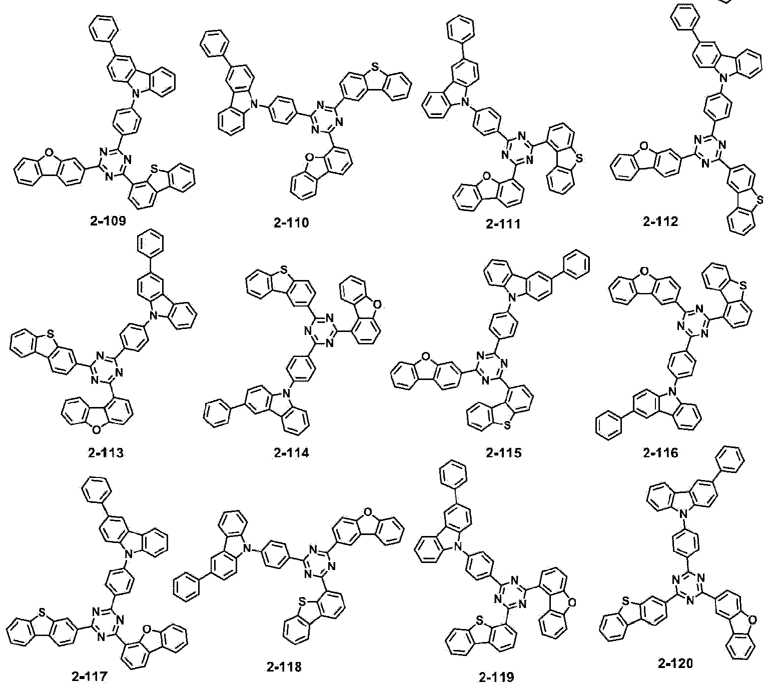

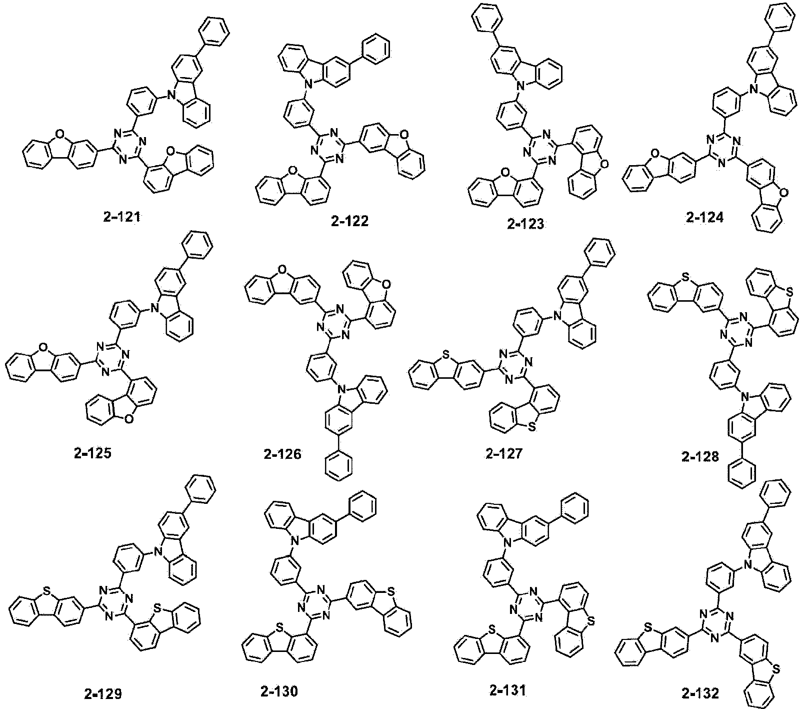

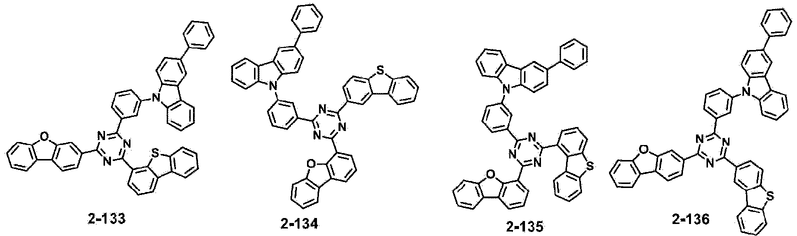

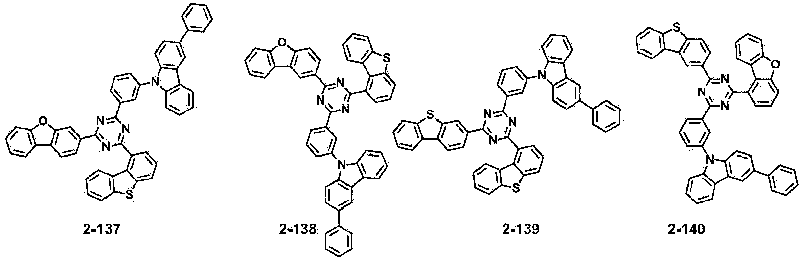

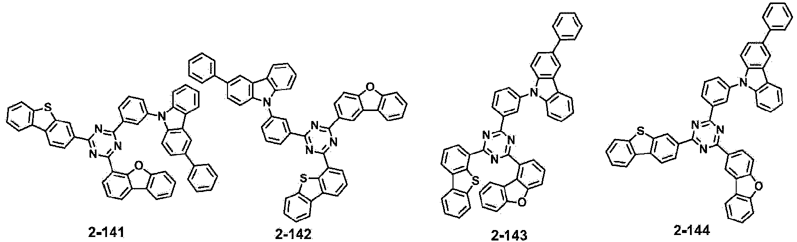

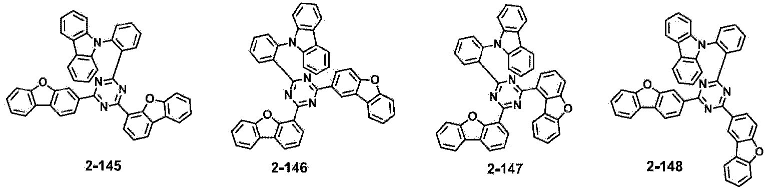

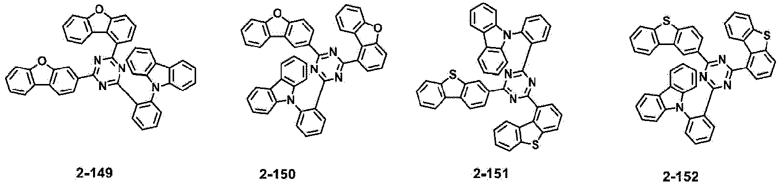

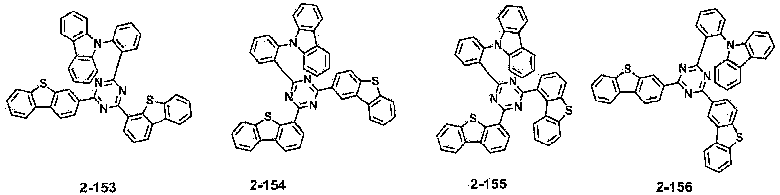

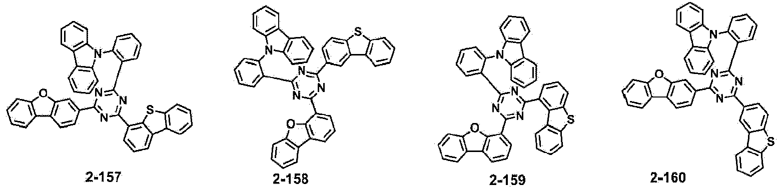

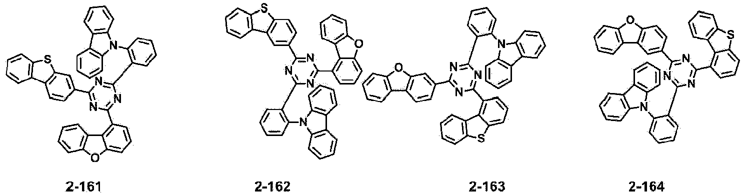

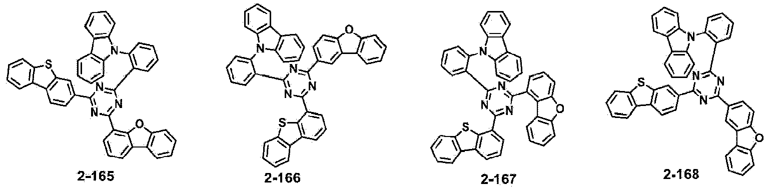

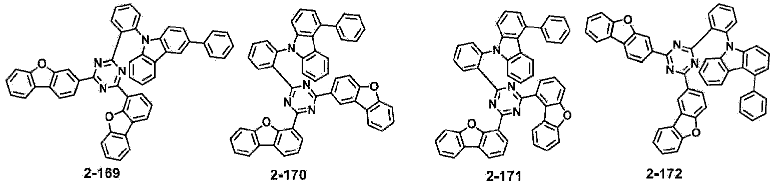

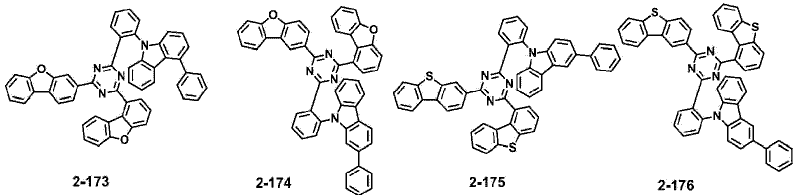

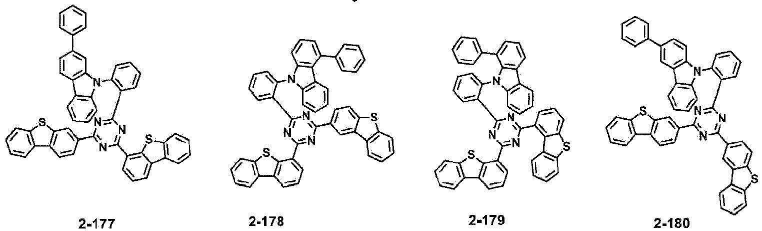

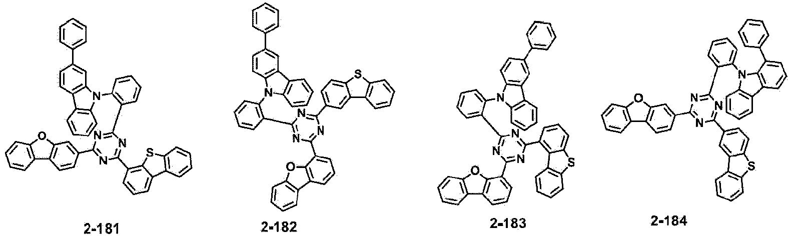

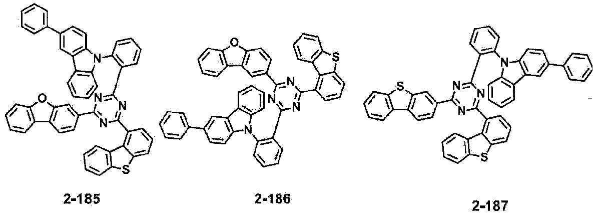

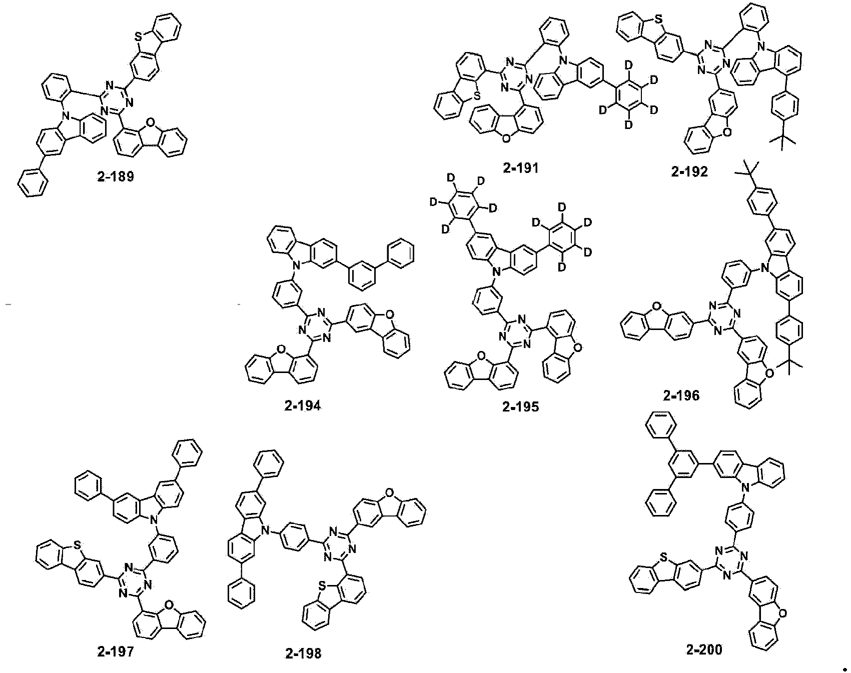

4. An organic light emitting device comprising:
a first electrode;
a second electrode; and
an organic material layer provided between the first electrode and the second electrode,
wherein the organic material layer includes the heterocyclic compound of any one of Claims 1 to 3.

5. The organic light emitting device of Claim 4, wherein the organic material layer includes a light emitting layer, and the light emitting layer includes the heterocyclic compound.

6. The organic light emitting device of Claim 4, wherein the organic material layer includes a light emitting layer, the light emitting layer includes a host, and the host includes the heterocyclic compound.

7. The organic light emitting device of Claim 5, wherein the light emitting layer further includes a compound of the following Chemical Formula 3: 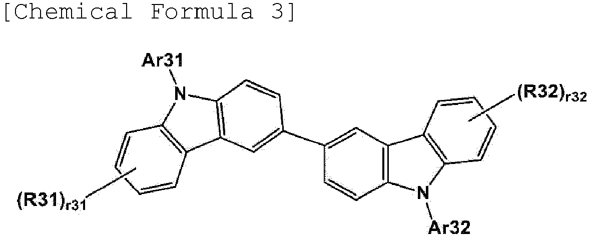 in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group,
Ar31 and Ar32 are each independently a substituted or unsubstituted C6 to C60 aryl group; or a substituted or unsubstituted C2 to C60 heteroaryl group,
r31 is an integer of 0 to 4, and when 2 or greater, R31s are the same as or different from each other, and
r32 is an integer of 0 to 4, and when 2 or greater, R32s are the same as or different from each other.

8. The organic light emitting device of Claim 9, wherein Chemical Formula 3 is represented by any one of the following compounds:

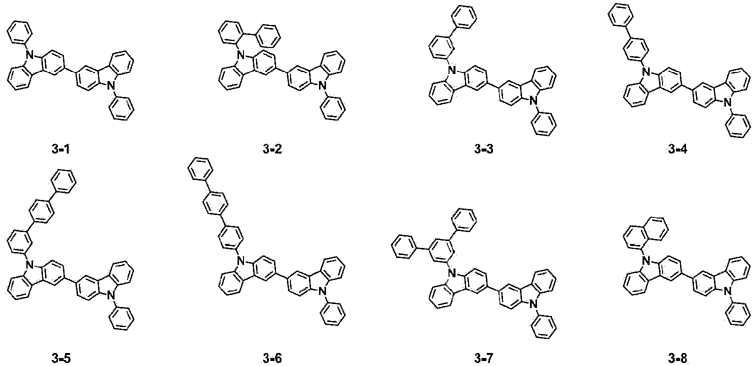

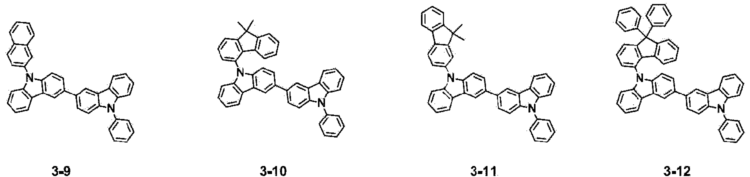

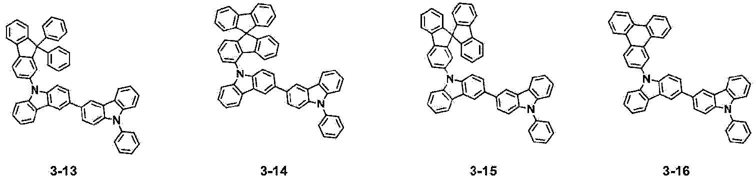

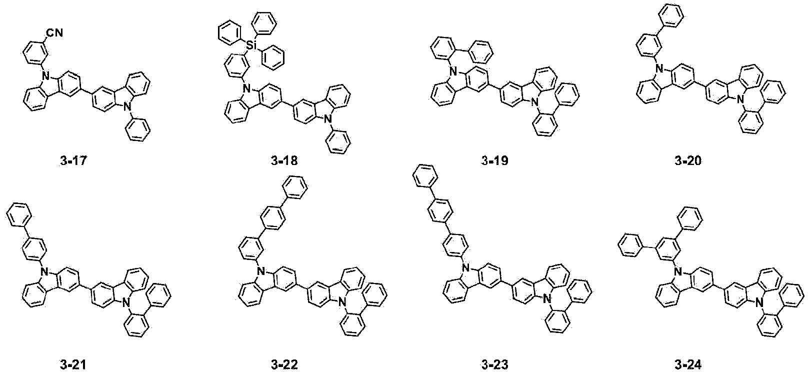

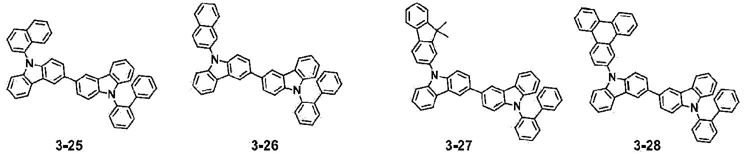

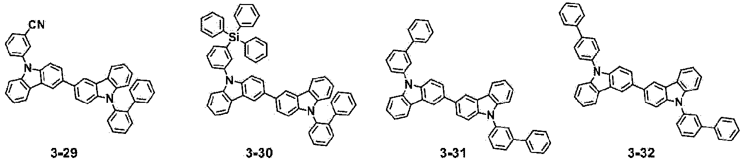

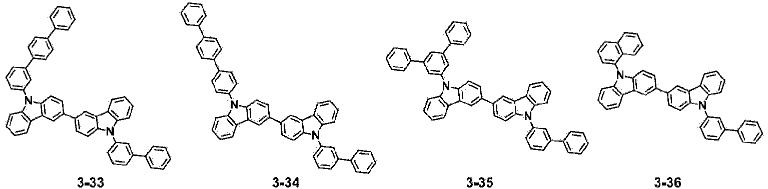

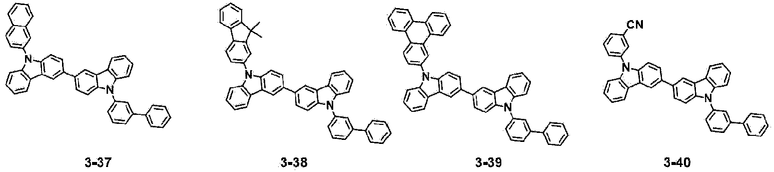

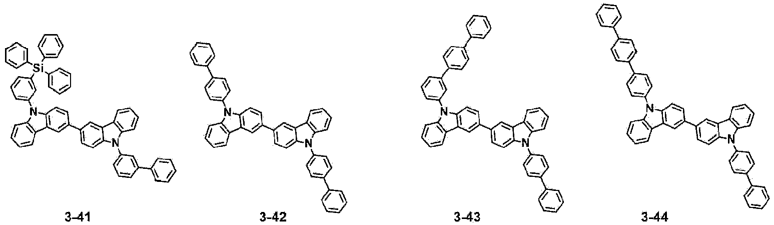

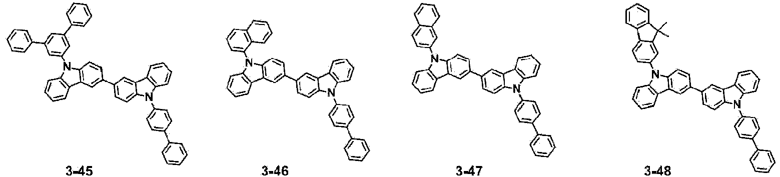

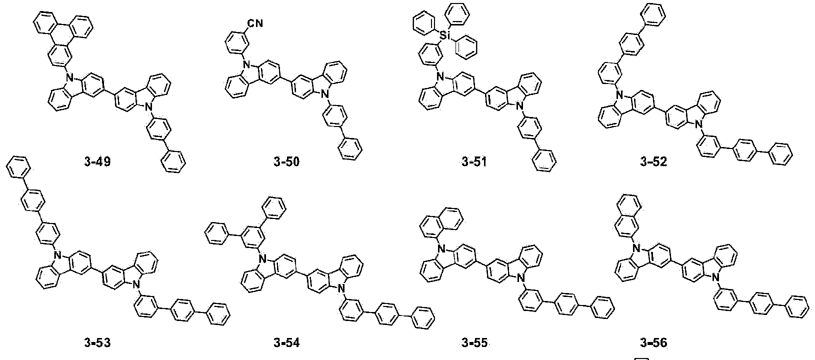

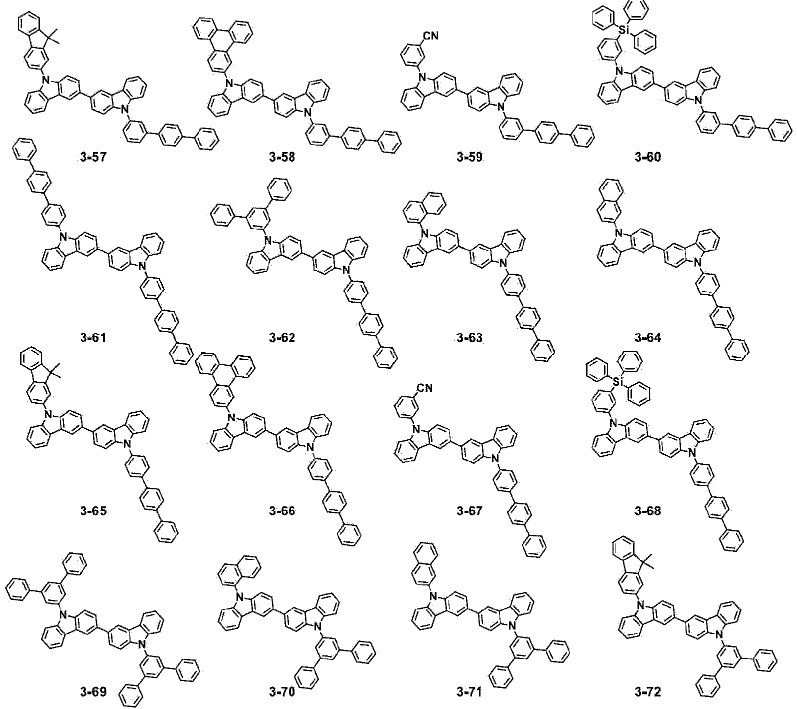

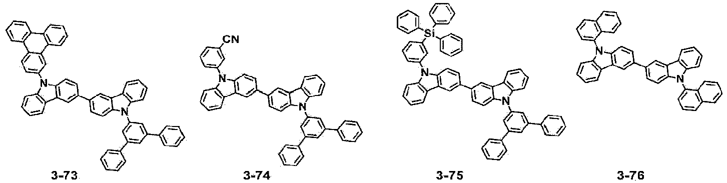

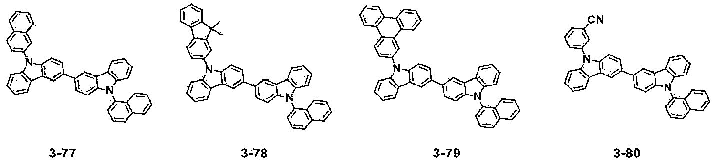

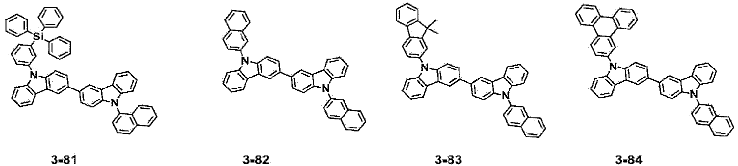

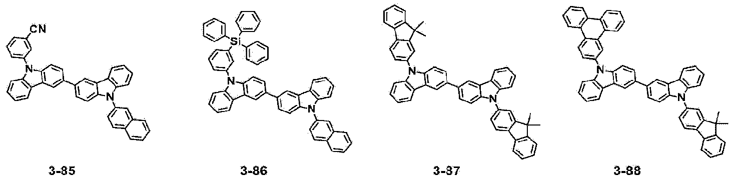

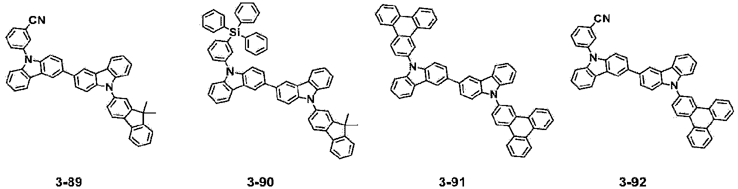

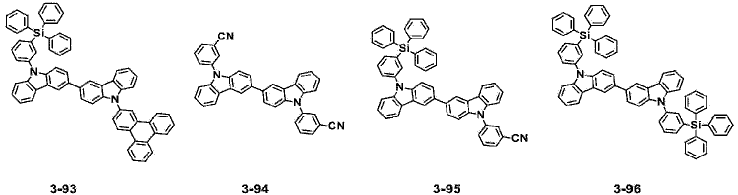

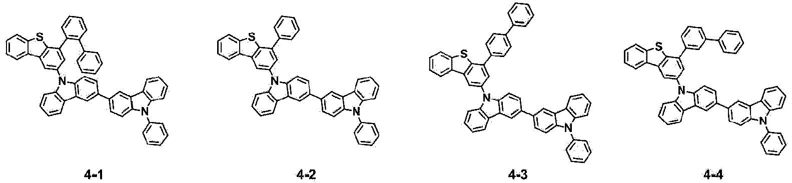

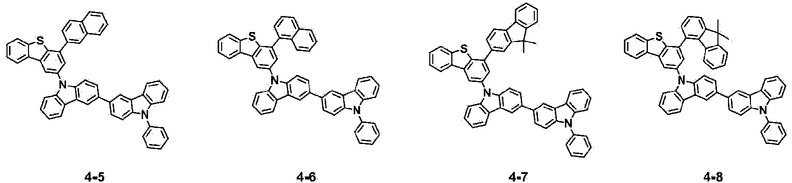

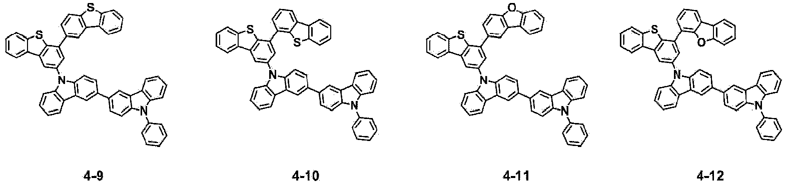

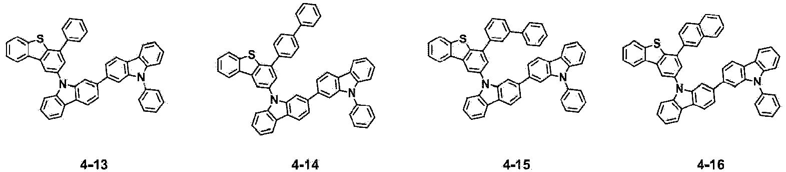

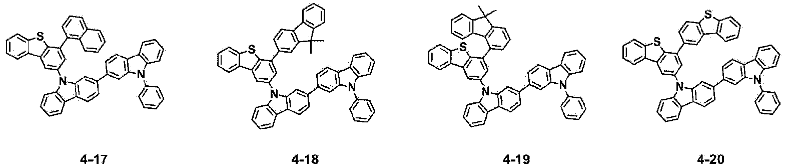

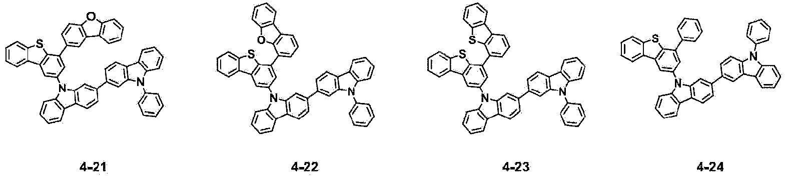

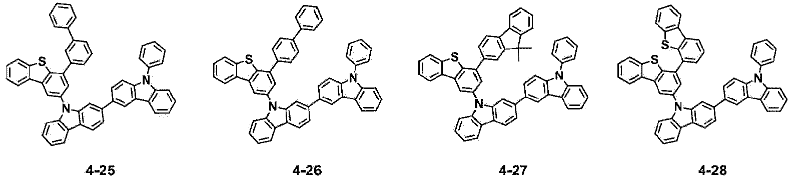

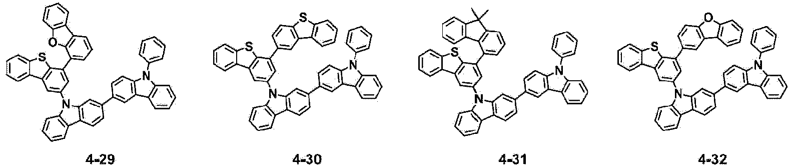

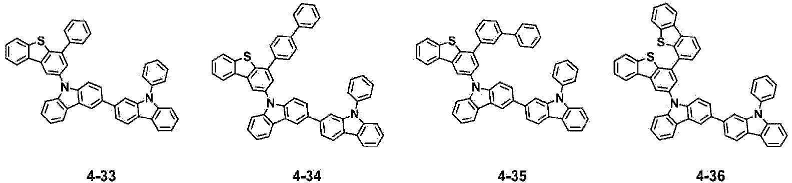

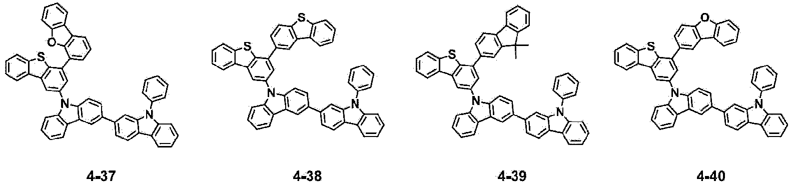

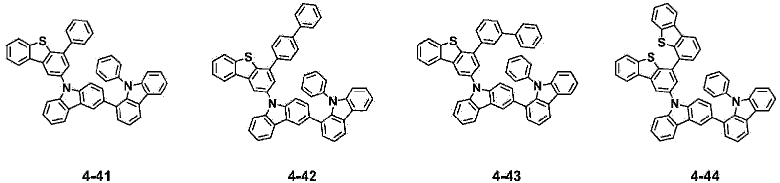

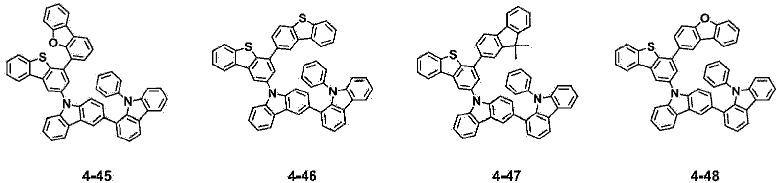

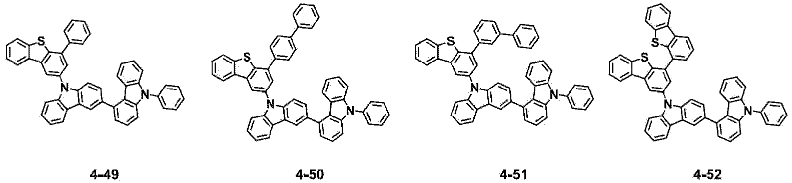

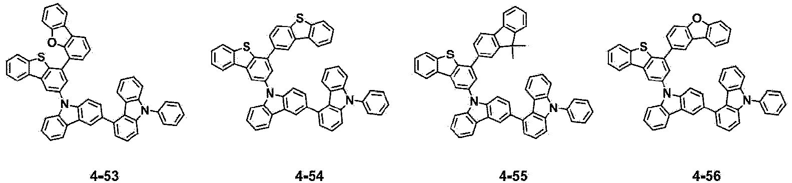

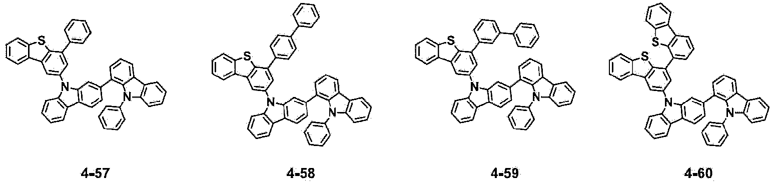

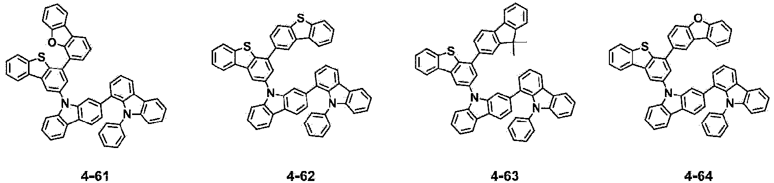

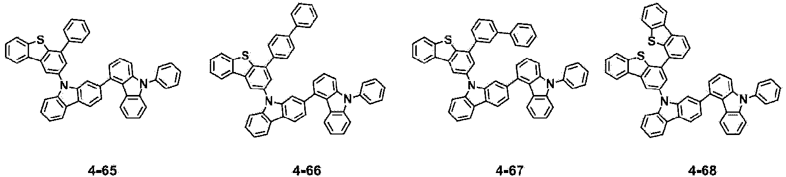

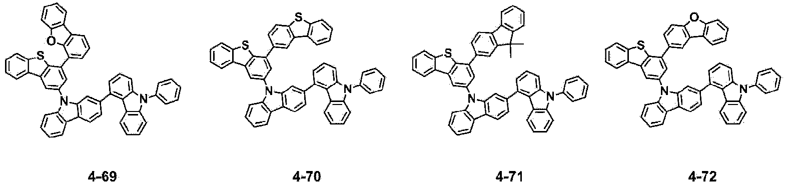

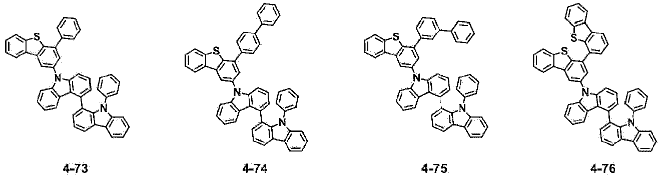

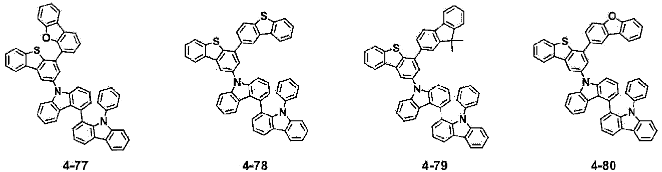

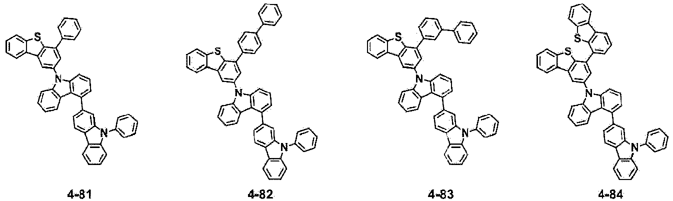

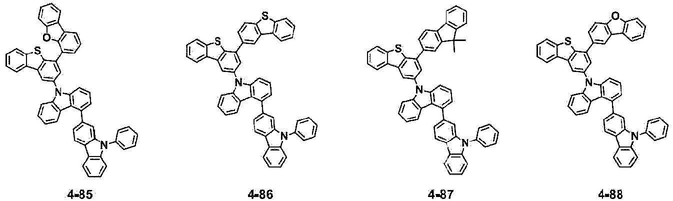

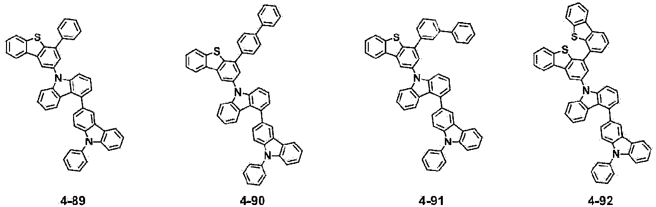

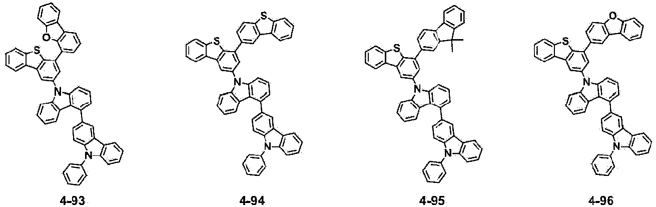

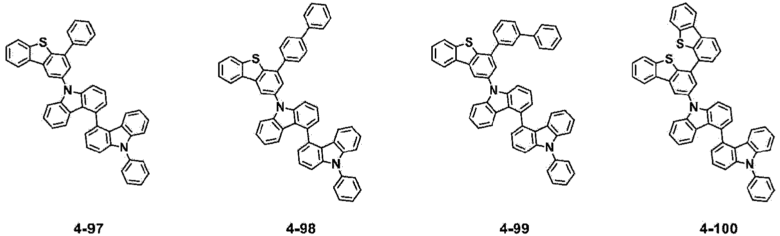

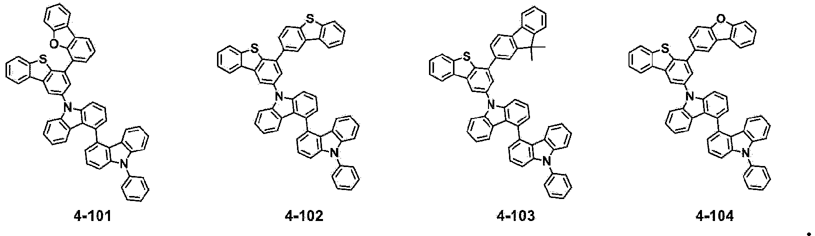

9. A composition for forming an organic material layer, the composition comprising:
the heterocyclic compound of Claim 1; and
a compound of the following Chemical Formula 3: 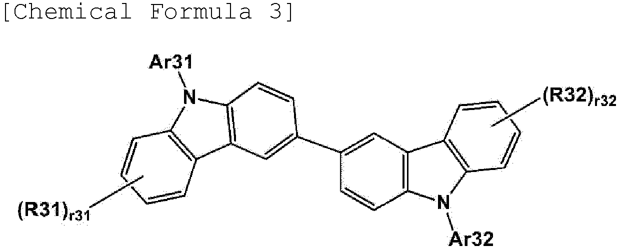
in Chemical Formula 3,
R31 and R32 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heteroaryl group;
Ar31 and Ar32 are each independently a substituted or unsubstituted C6 to C60 aryl group; or a substituted or unsubstituted C2 to C60 heteroaryl group;
r31 is an integer of 0 to 4, and when 2 or greater, R31s are the same as or different from each other; and
r32 is an integer of 0 to 4, and when 2 or greater, R32s are the same as or different from each other.

10. The composition for forming an organic material layer of Claim 9, wherein the heterocyclic compound and the compound of Chemical Formula 3 have a weight ratio of 1:10 to 10:1.

## Patentansprüche

1. Heterocyclische Verbindung der folgenden chemischen Formel 1: 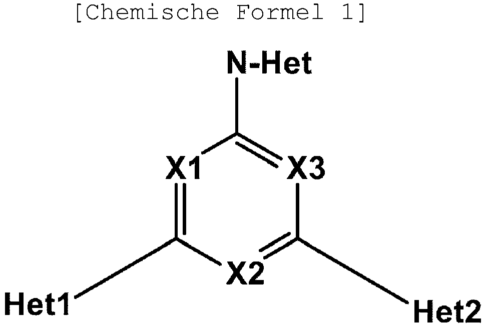 wobei in der chemischen Formel 1
X1 bis X3 N sind,
N-Het durch die folgende chemische Formel N-1 oder N-2 dargestellt ist, 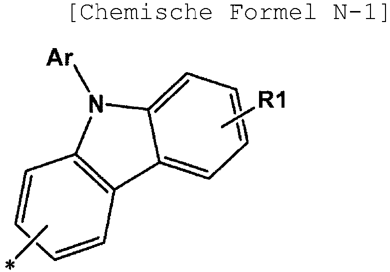 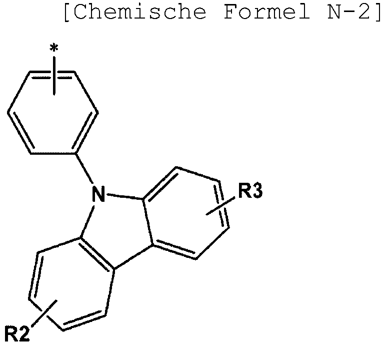
wobei in den chemischen Formeln N-1 und N-2
R1 Wasserstoff; Deuterium; eine Halogengruppe; eine Cyanogruppe; eine substituierte oder unsubstituierte Silylgruppe; eine substituierte oder unsubstituierte Phosphinoxidgruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte bicyclische oder niedere Arylgruppe; eine substituierte oder unsubstituierte Terphenylgruppe; eine substituierte oder unsubstituierte Fluorenylgruppe; eine substituierte oder unsubstituierte tetracyclische oder höhere Arylgruppe; oder eine substituierte oder unsubstituierte C2 - bis C60 -Heteroarylgruppe ist,
R2 und R3 jeweils unabhängig Wasserstoff; Deuterium; eine substituierte oder unsubstituierte Phenylgruppe; eine substituierte oder unsubstituierte Biphenylgruppe; oder eine substituierte oder unsubstituierte Terphenylgruppe sind,
Ar eine substituierte oder unsubstituierte C6 - bis C60 - Arylgruppe; oder eine substituierte oder unsubstituierte C2 - bis C60 -Heteroarylgruppe ist, und
Het1 durch die folgende chemische Formel H-1 dargestellt ist, und Het2 durch die folgende chemische Formel H-2 dargestellt ist, 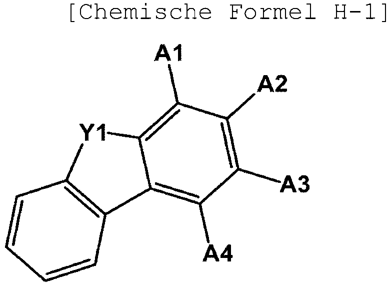 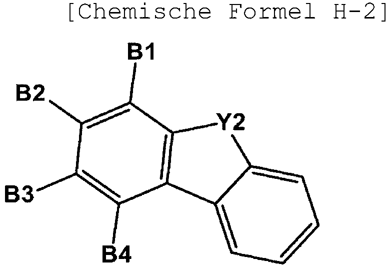
wobei in den chemischen Formeln H-1 und H-2,
Y1 und Y2 jeweils unabhängig O oder S sind,
A1 bis A4 und B1 bis B4 jeweils unabhängig an die chemische Formel 1 binden, oder Wasserstoff; oder Deuterium sind, und
die chemische Formel 1 an eines von A1 bis A4 der chemischen Formel H-1 und eines von B1 bis B4 der chemischen Formel H-2 bindet, welches durch Am-Bn dargestellt ist, m und n jeweils 1, 2, 3 oder 4 sind, und m und n verschieden sind.

2. Heterocyclische Verbindung nach Anspruch 1, wobei Ar eine substituierte oder unsubstituierte Phenylgruppe; eine substituierte oder unsubstituierte Biphenylgruppe; eine substituierte oder unsubstituierte Terphenylgruppe; eine substituierte oder unsubstituierte Naphthylgruppe; eine substituierte oder unsubstituierte Phenanthrenylgruppe; eine substituierte oder unsubstituierte Triphenylenylgruppe; oder eine substituierte oder unsubstituierte Pyrenylgruppe ist.

3. Heterocyclische Verbindung nach Anspruch 1, wobei die chemische Formel 1 durch eine der folgenden Verbindungen dargestellt ist:

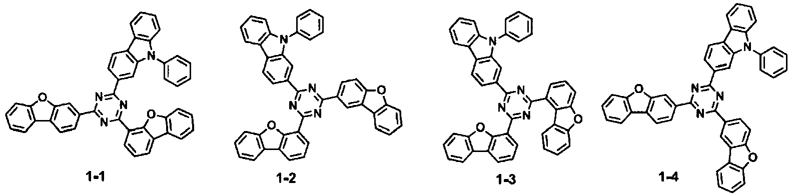

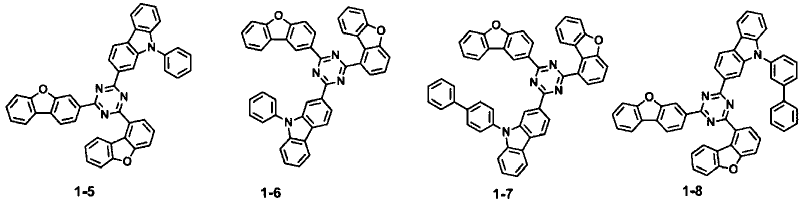

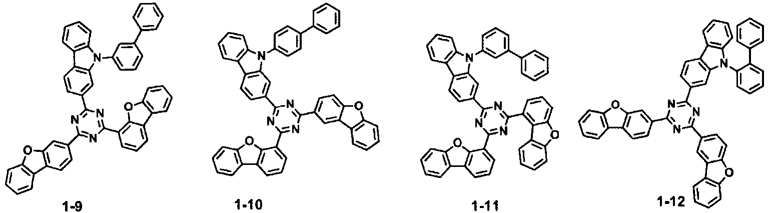

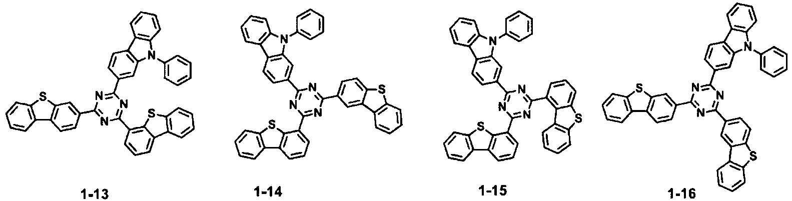

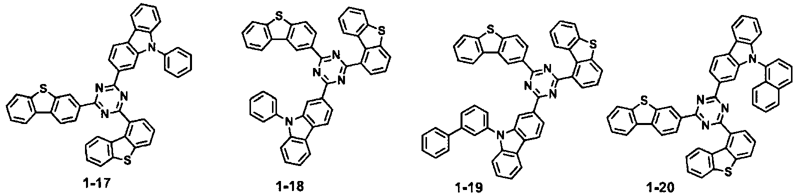

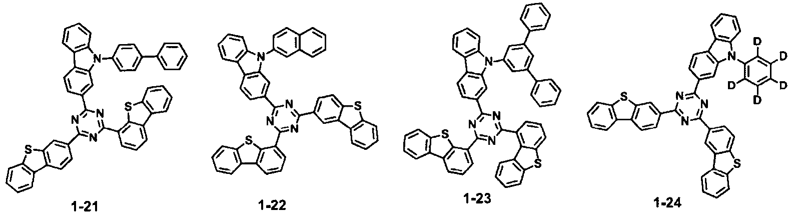

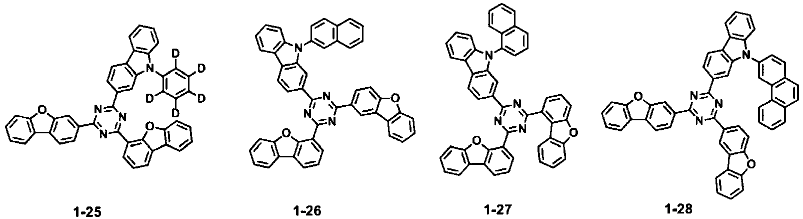

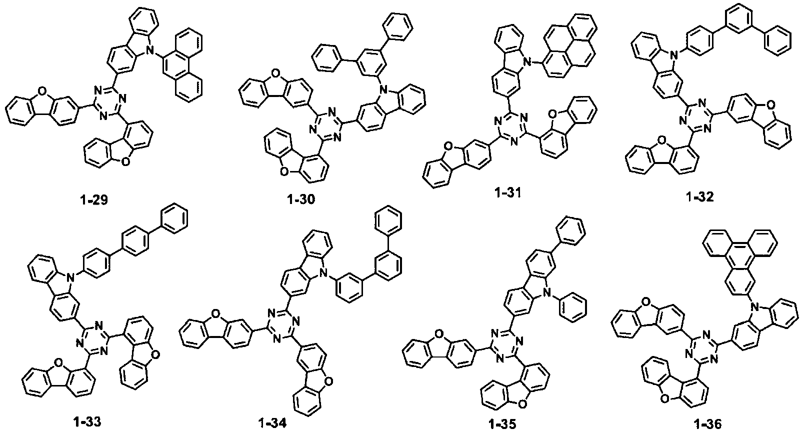

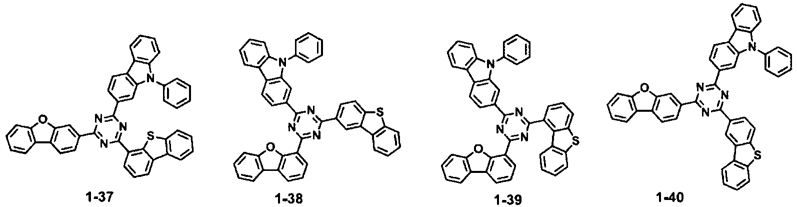

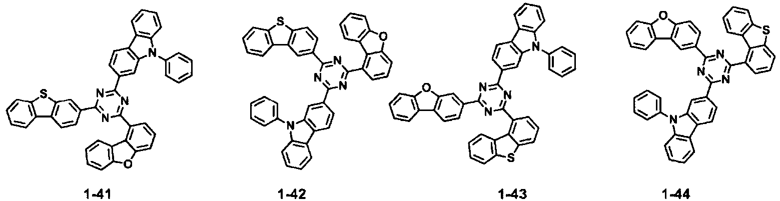

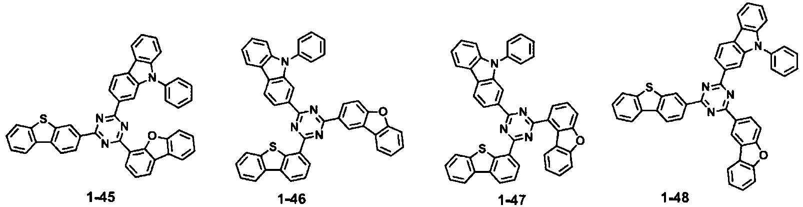

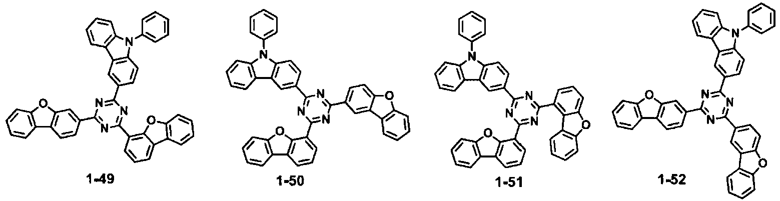

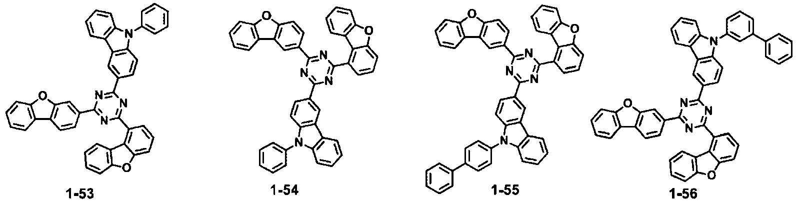

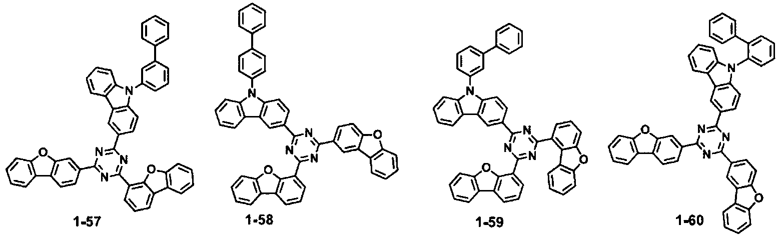

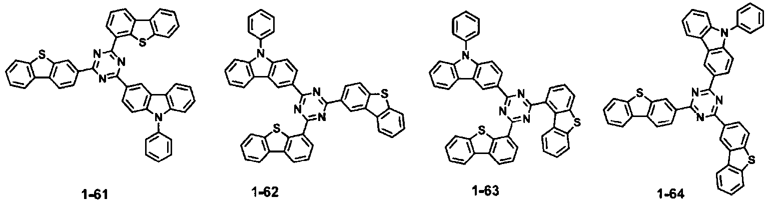

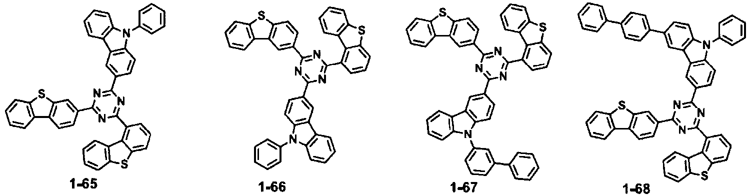

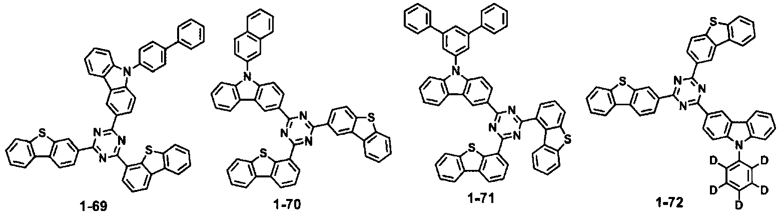

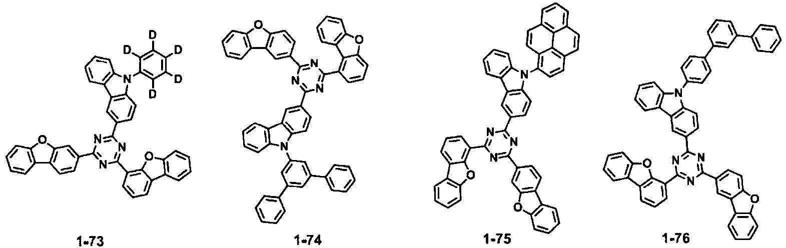

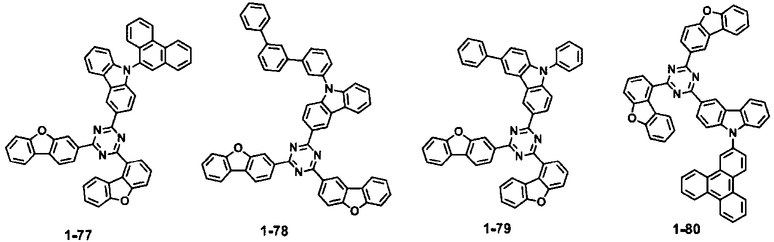

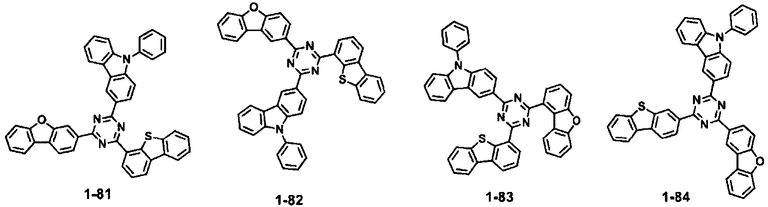

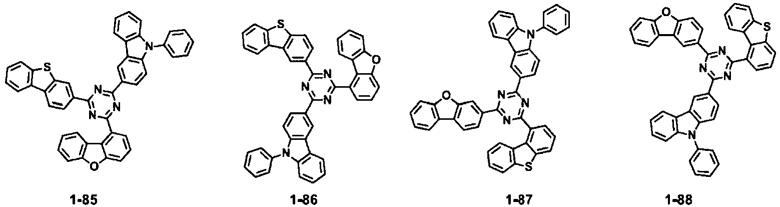

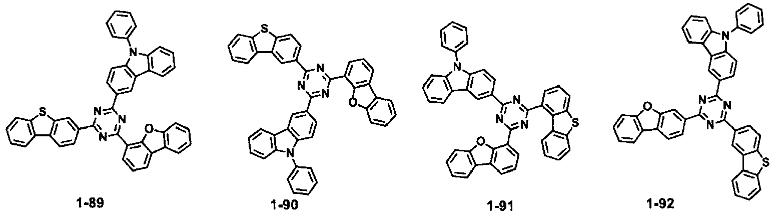

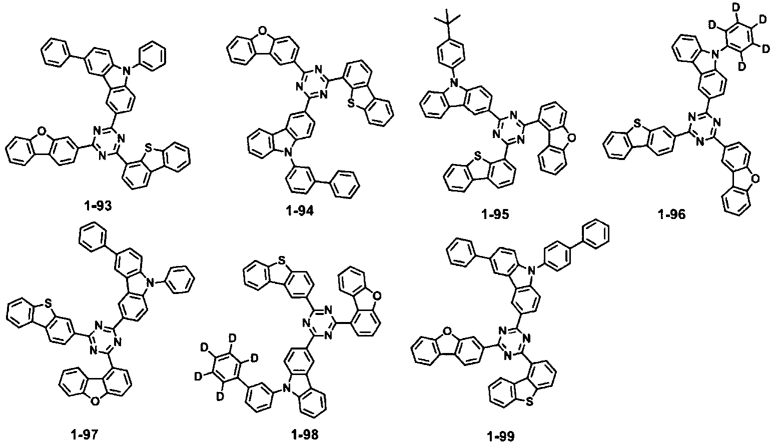

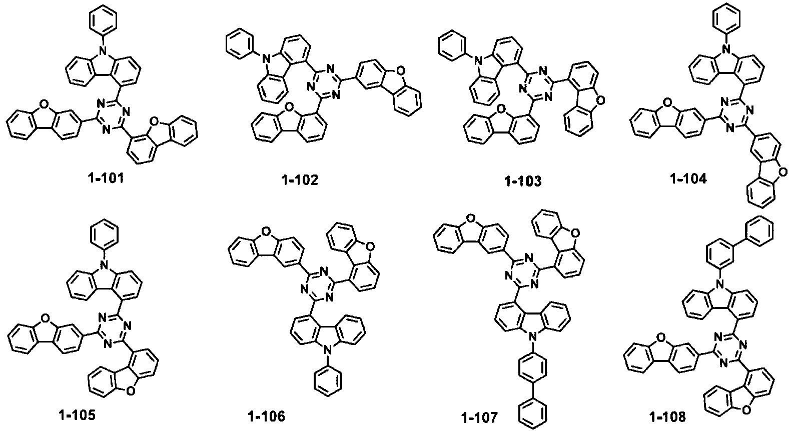

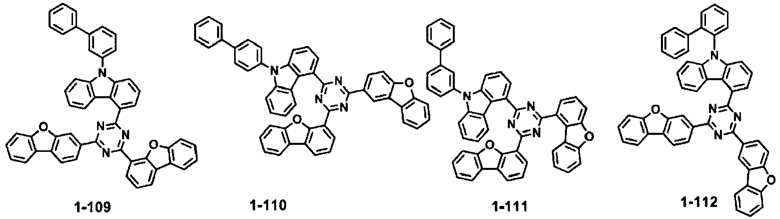

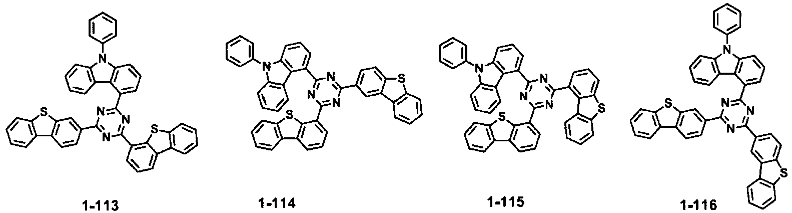

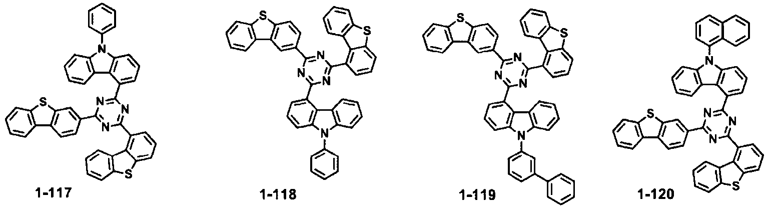

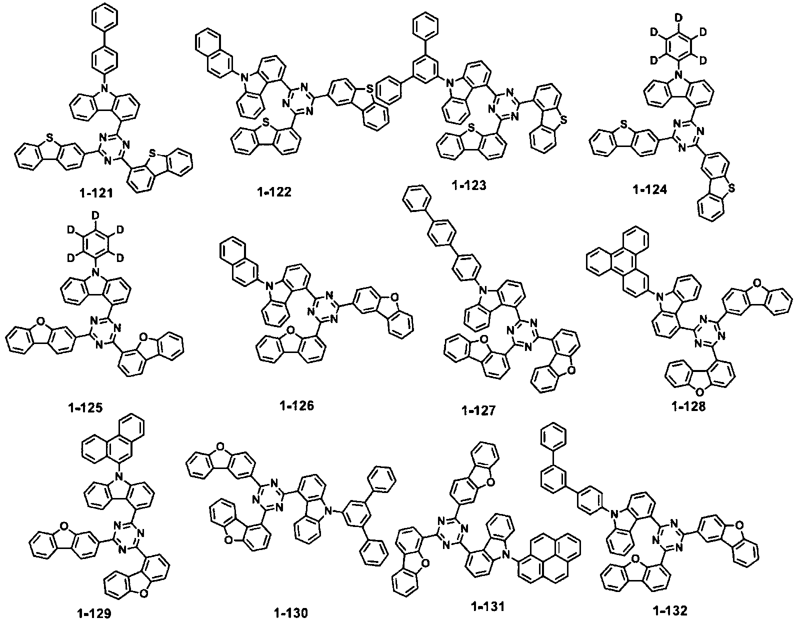

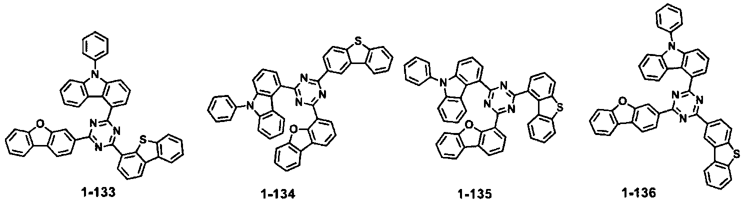

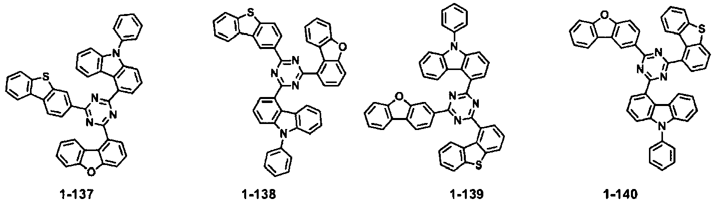

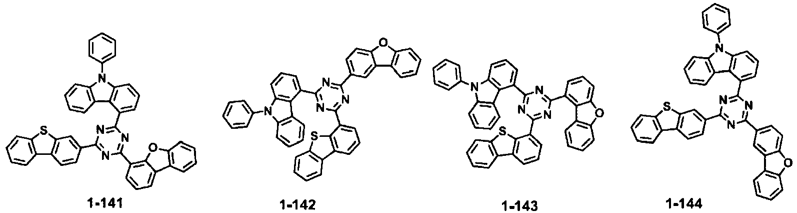

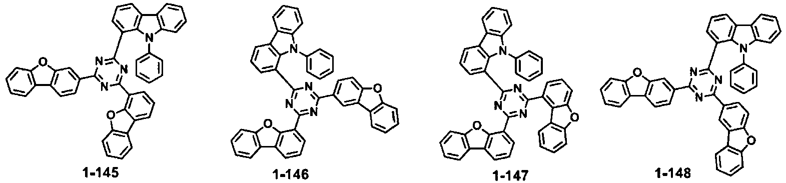

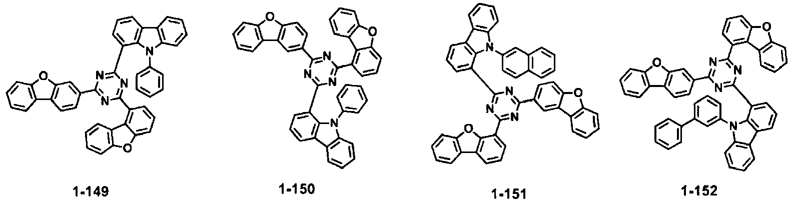

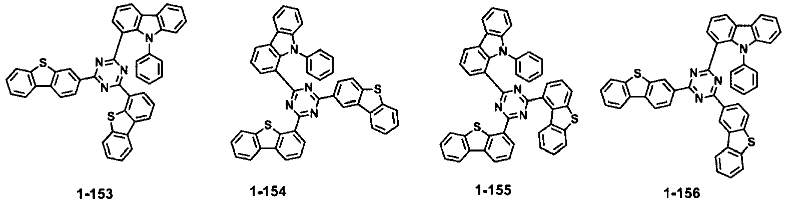

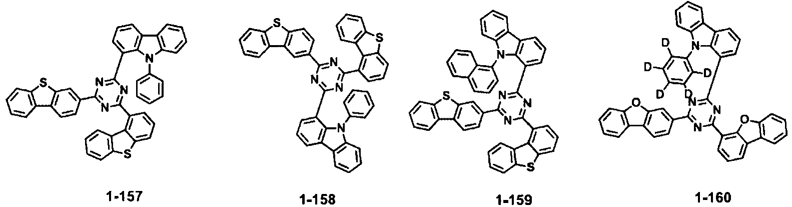

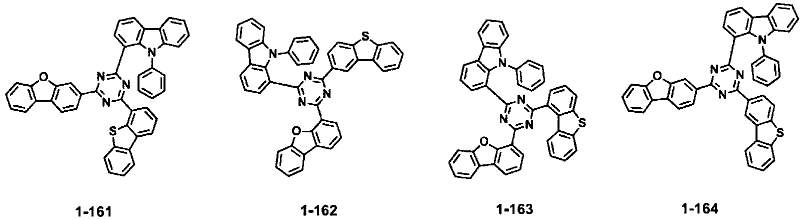

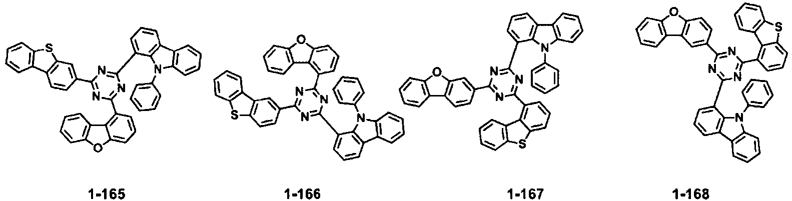

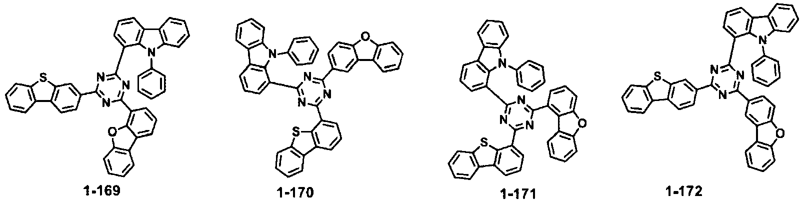

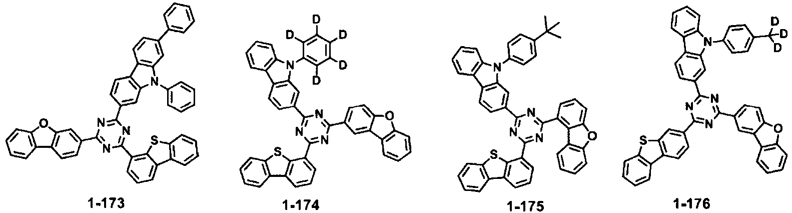

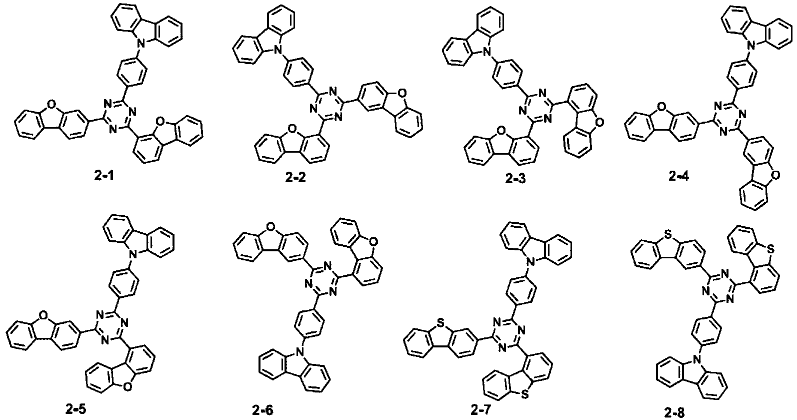

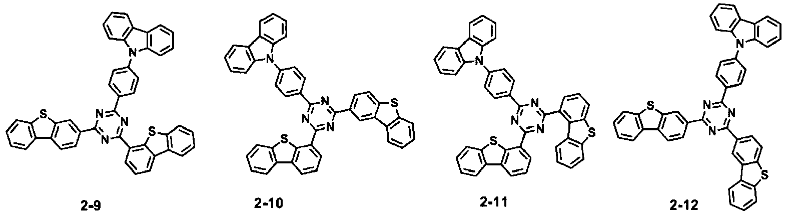

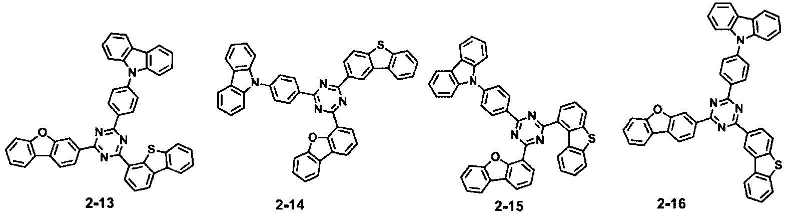

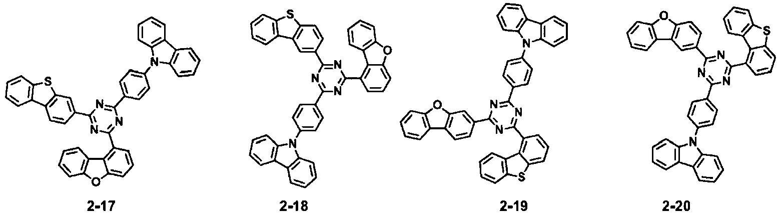

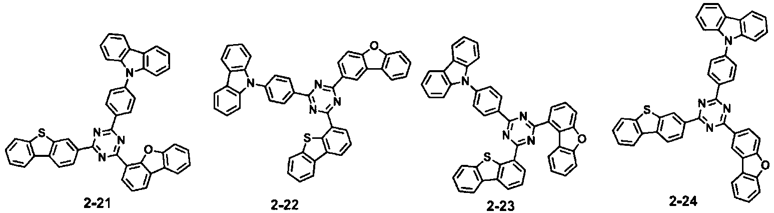

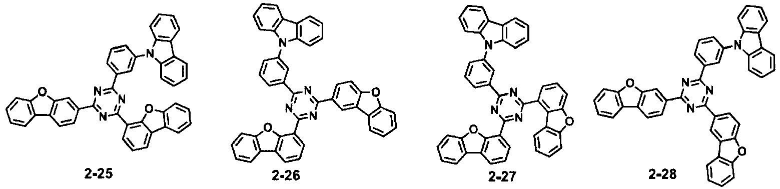

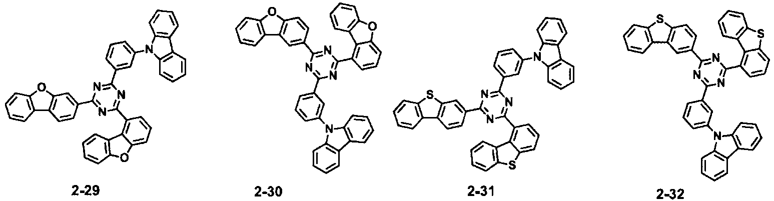

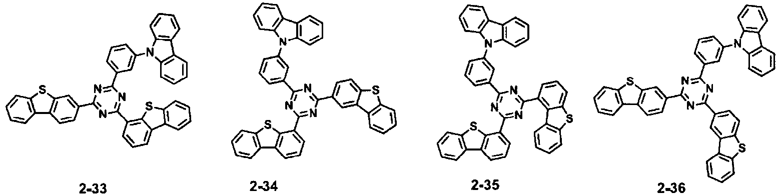

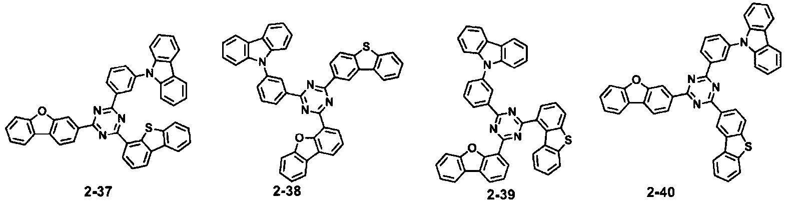

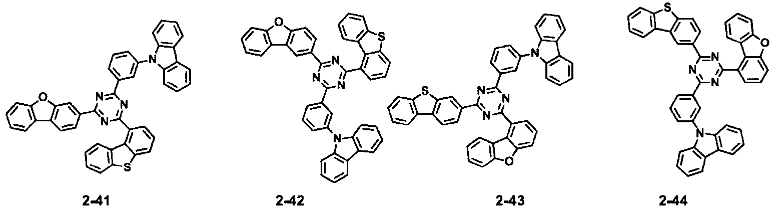

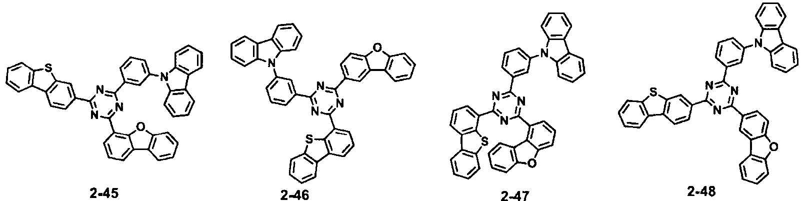

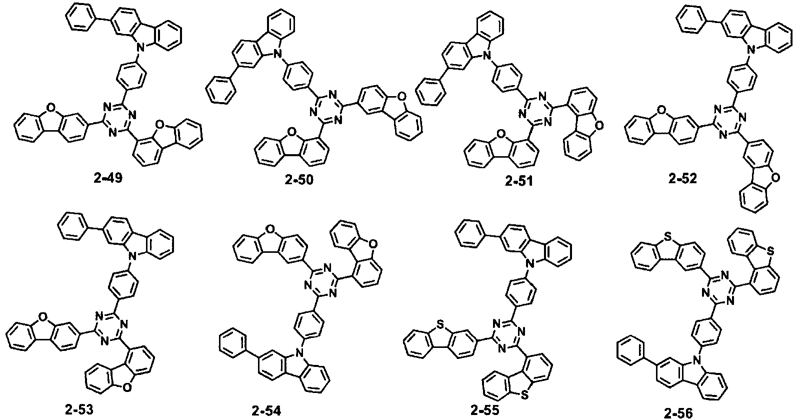

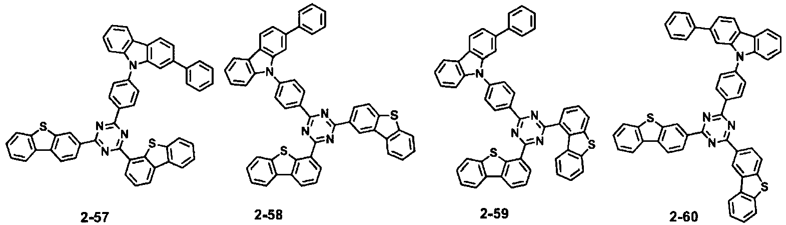

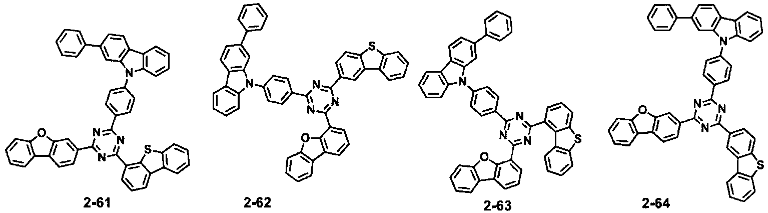

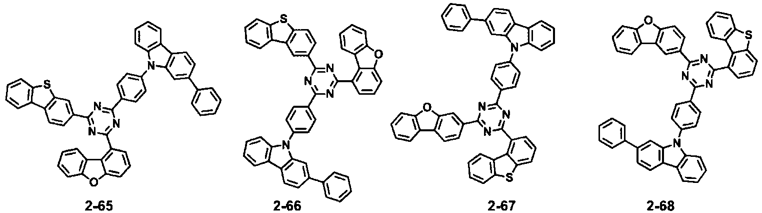

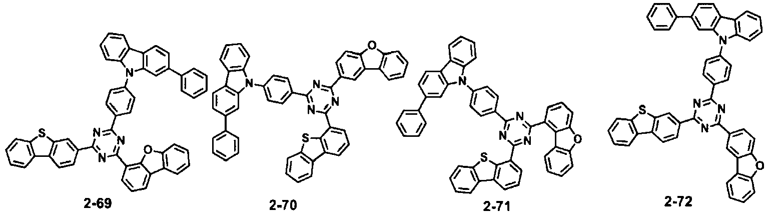

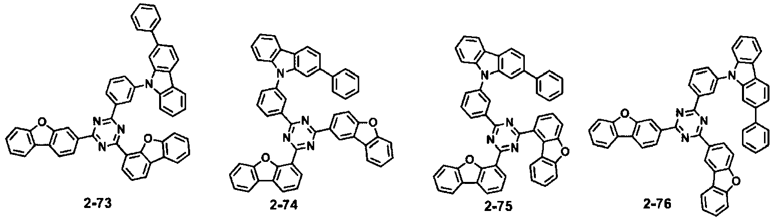

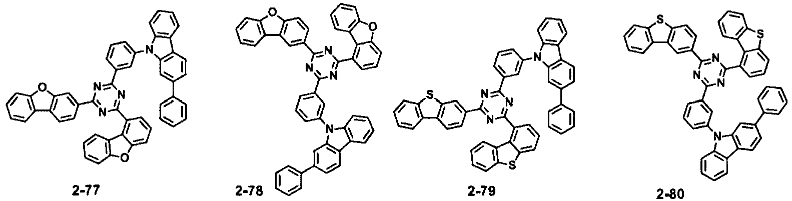

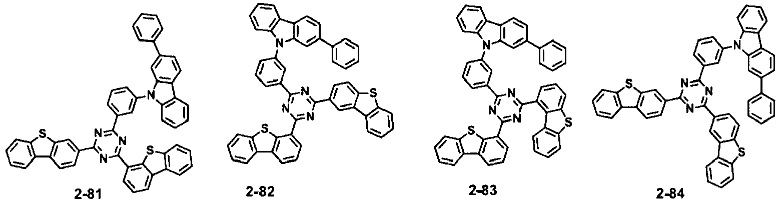

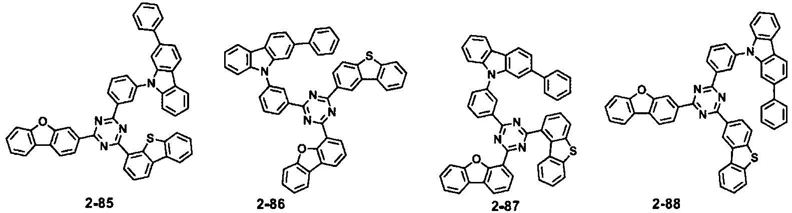

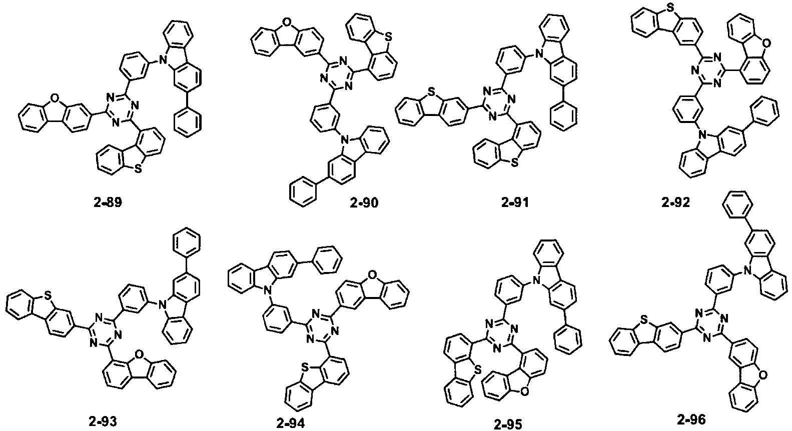

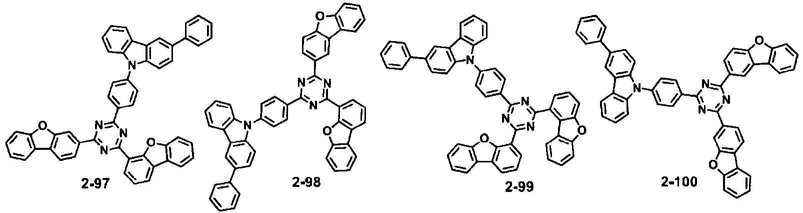

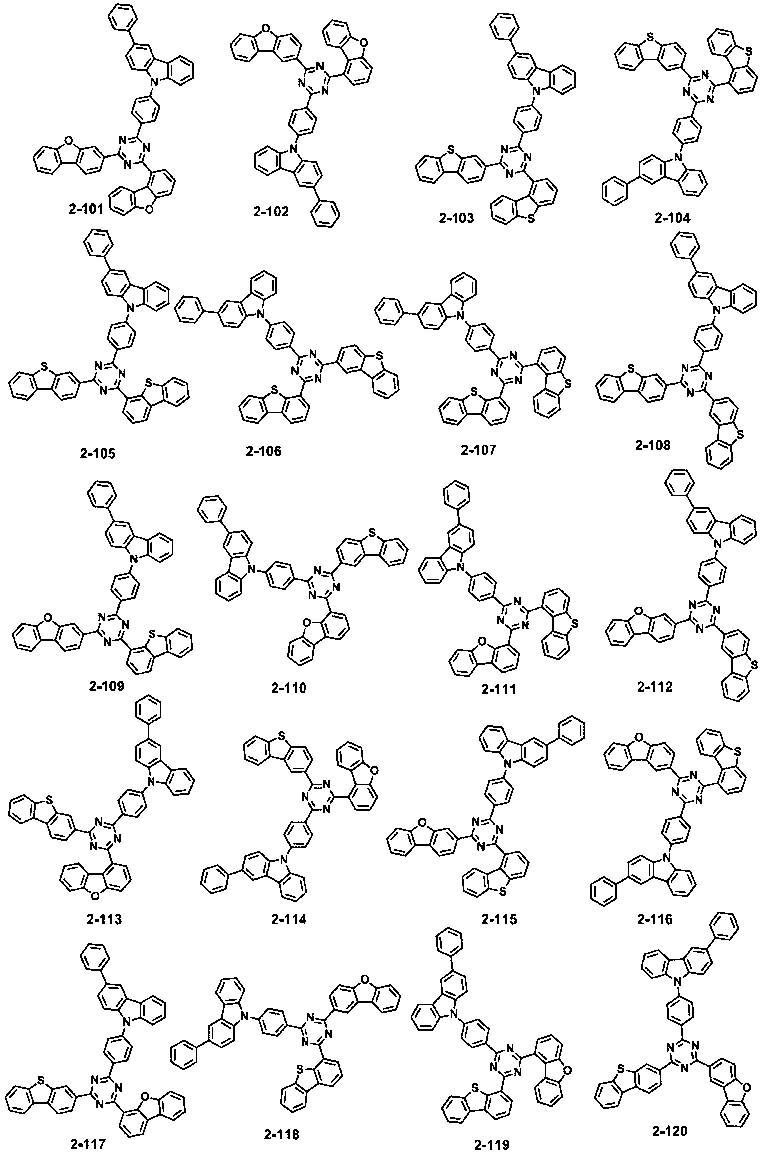

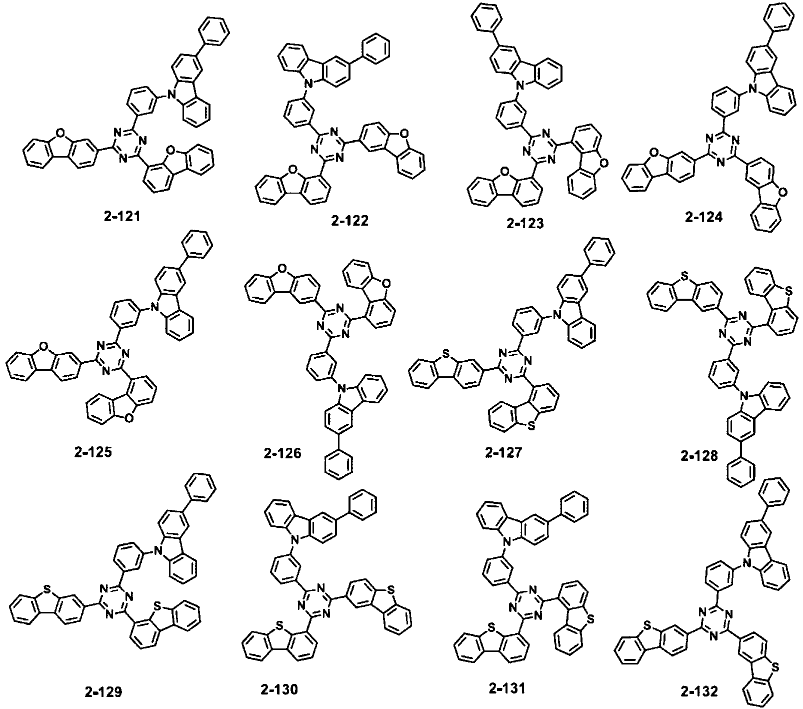

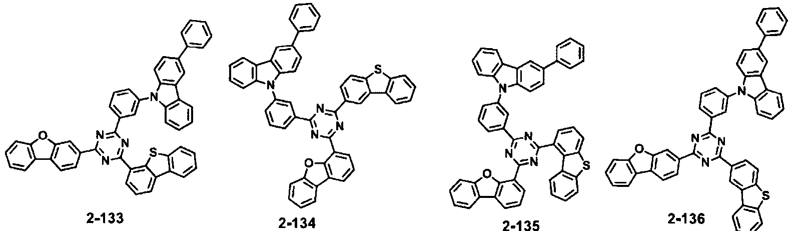

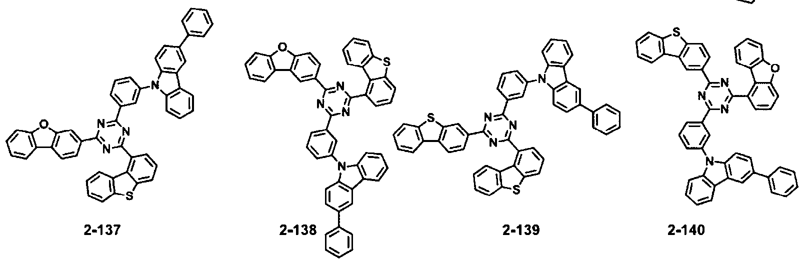

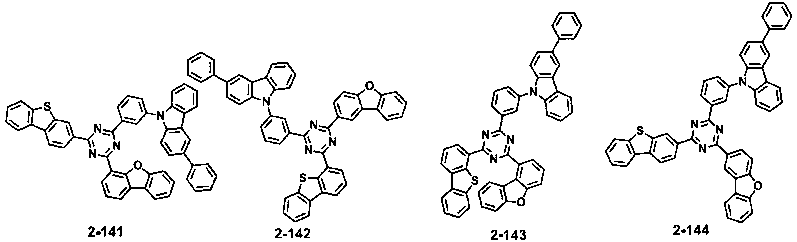

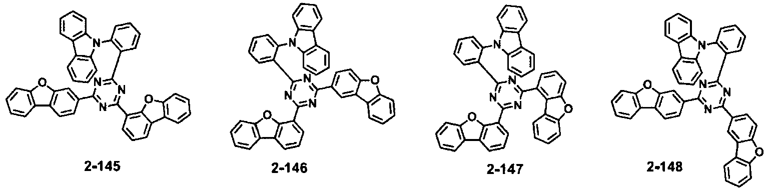

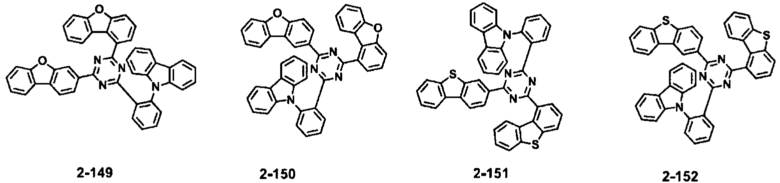

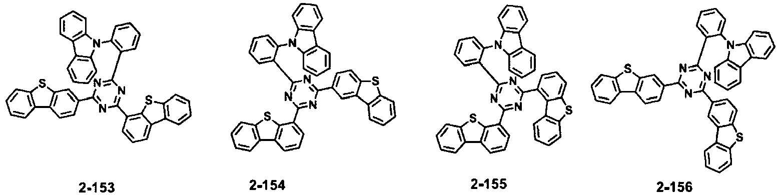

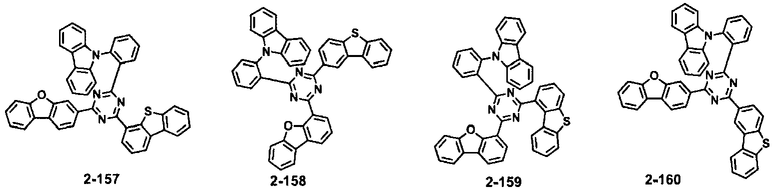

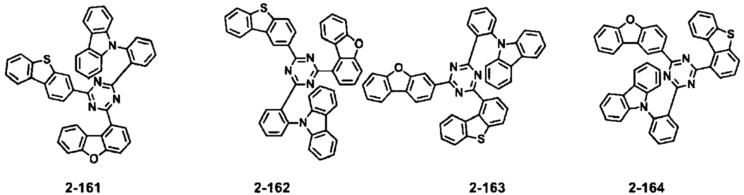

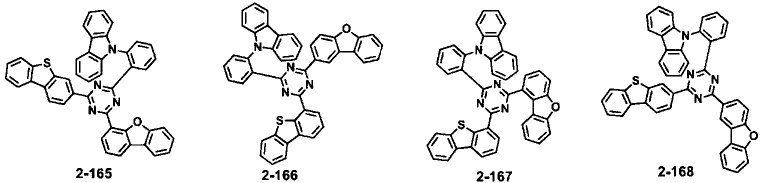

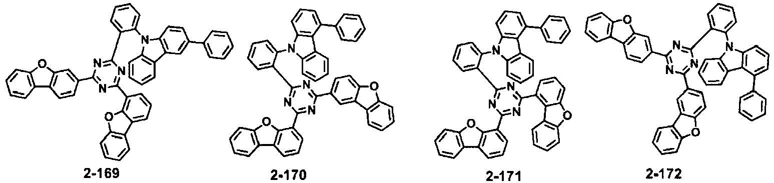

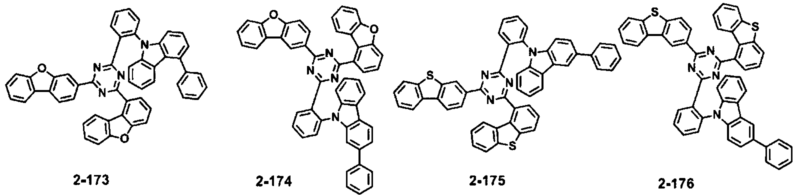

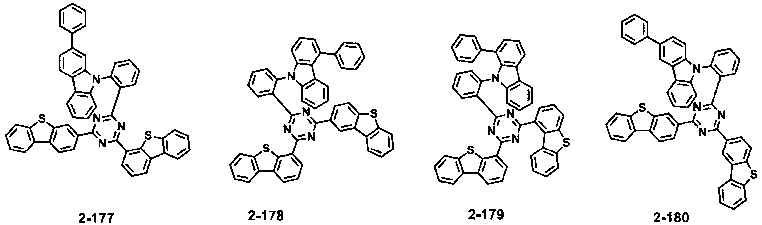

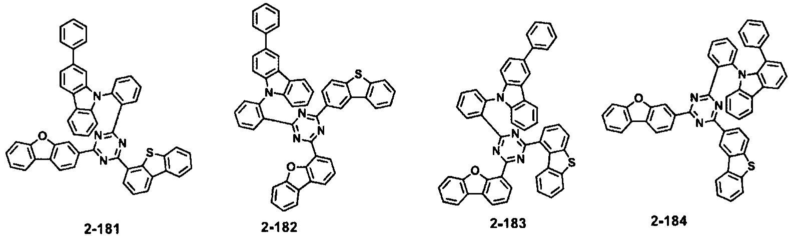

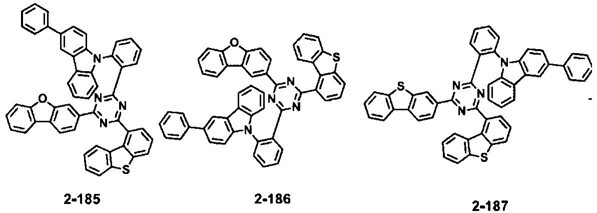

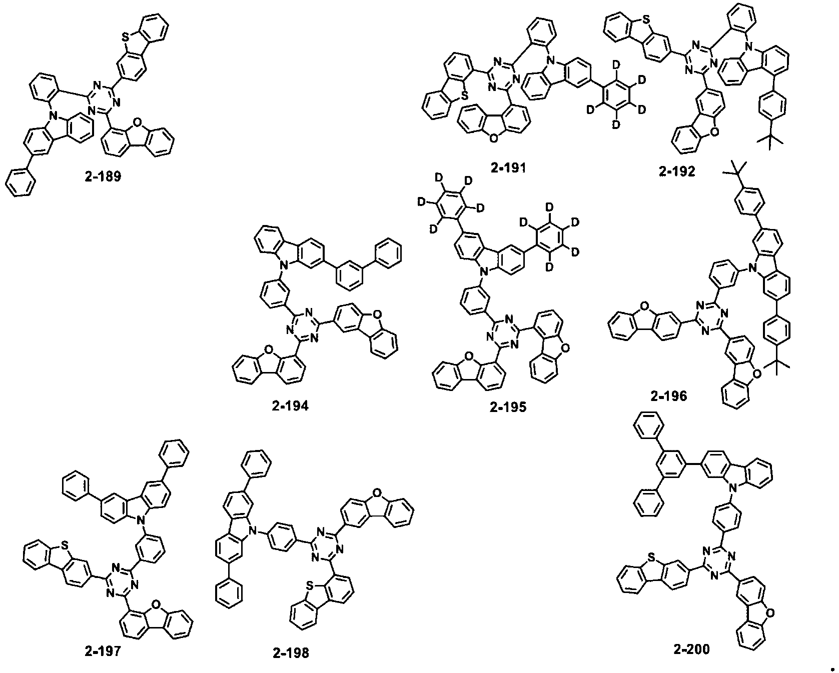

4. Organische lichtemittierende Vorrichtung, umfassend:
eine erste Elektrode;
eine zweite Elektrode; und
eine organische Materialschicht, die zwischen der ersten Elektrode und der zweiten Elektrode bereitgestellt ist,
wobei die organische Materialschicht die heterocyclische Verbindung nach einem der Ansprüche 1 bis 3 umfasst.

5. Organische lichtemittierende Vorrichtung nach Anspruch 4, wobei die organische Materialschicht eine lichtemittierende Schicht umfasst und die lichtemittierende Schicht die heterocyclische Verbindung umfasst.

6. Organische lichtemittierende Vorrichtung nach Anspruch 4, wobei die organische Materialschicht eine lichtemittierende Schicht umfasst, die lichtemittierende Schicht einen Wirt umfasst und der Wirt die heterocyclische Verbindung umfasst.

7. Organische lichtemittierende Vorrichtung nach Anspruch 5, wobei die lichtemittierende Schicht ferner eine Verbindung der folgenden chemischen Formel 3 umfasst: 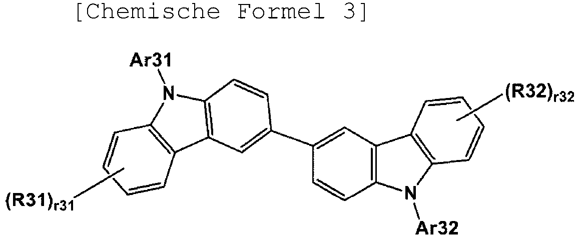 wobei in der chemischen Formel 3
R31 und R32 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe; oder eine substituierte oder unsubstituierte C2 - bis C60 - Heteroarylgruppe sind,
Ar31 und Ar32 jeweils unabhängig eine substituierte oder unsubstituierte C6 - bis C60 -Arylgruppe; oder eine substituierte oder unsubstituierte C2 - bis C60 - Heteroarylgruppe sind,
r31 eine ganze Zahl von 0 bis 4 ist, und wenn 2 oder größer, R31s gleich oder verschieden voneinander sind, und
r32 eine ganze Zahl von 0 bis 4 ist, und wenn 2 oder größer, R32s gleich oder verschieden voneinander sind.

8. Organische lichtemittierende Vorrichtung nach Anspruch 9, wobei die chemische Formel 3 durch eine der folgenden Verbindungen dargestellt ist:

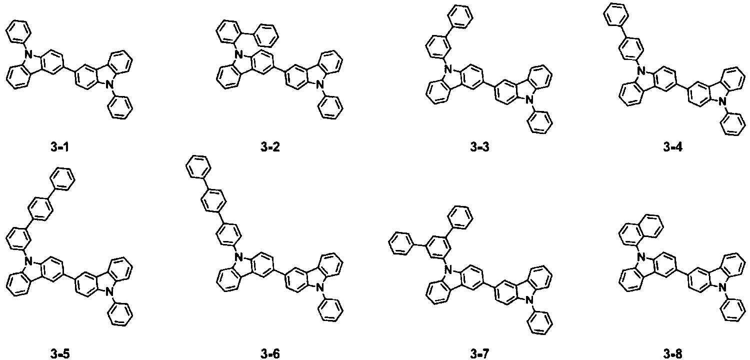

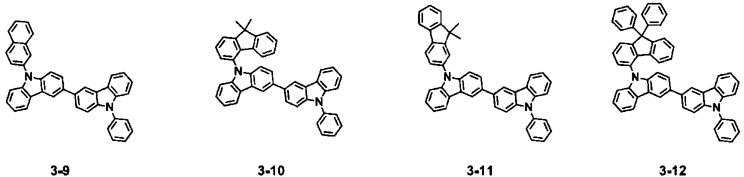

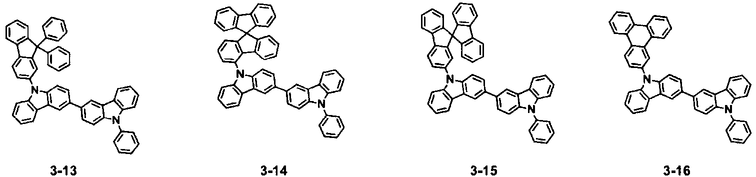

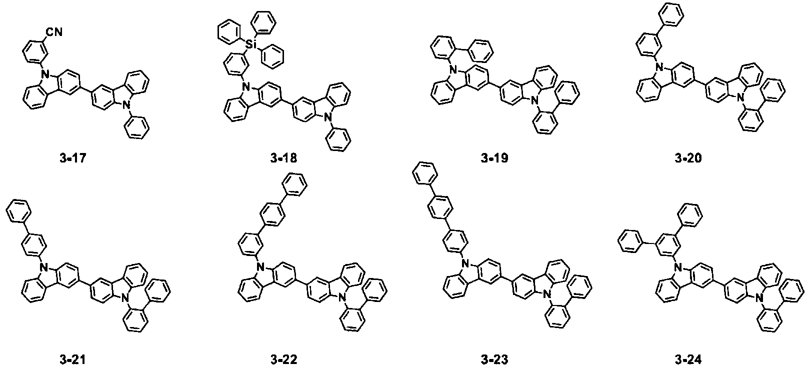

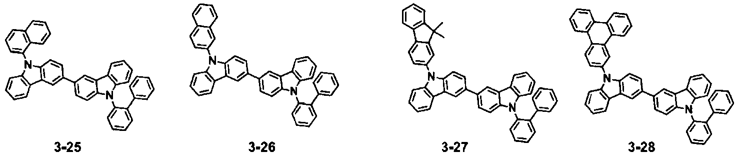

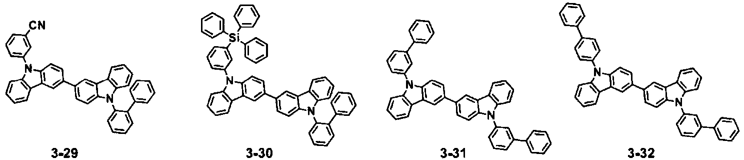

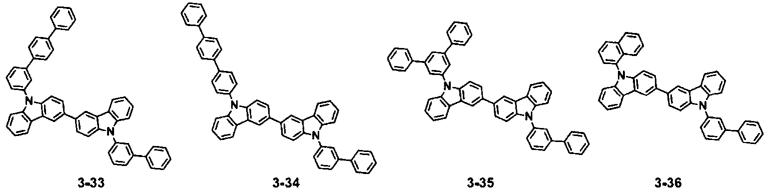

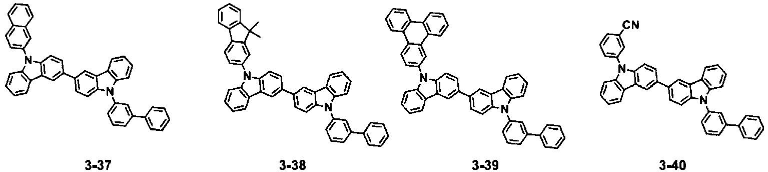

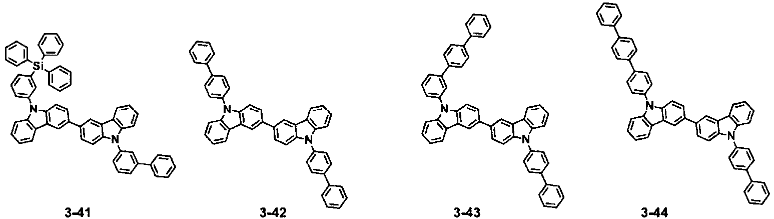

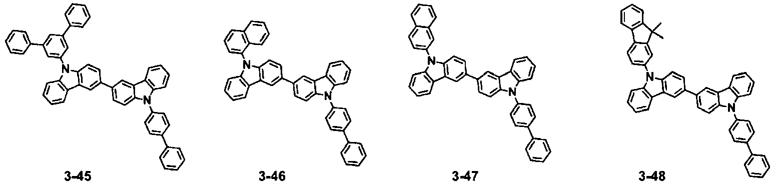

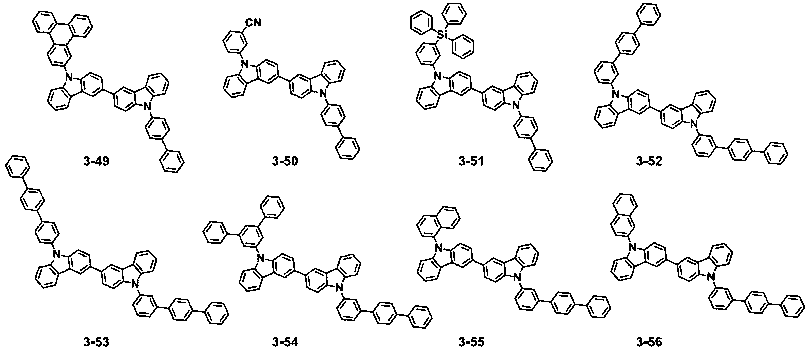

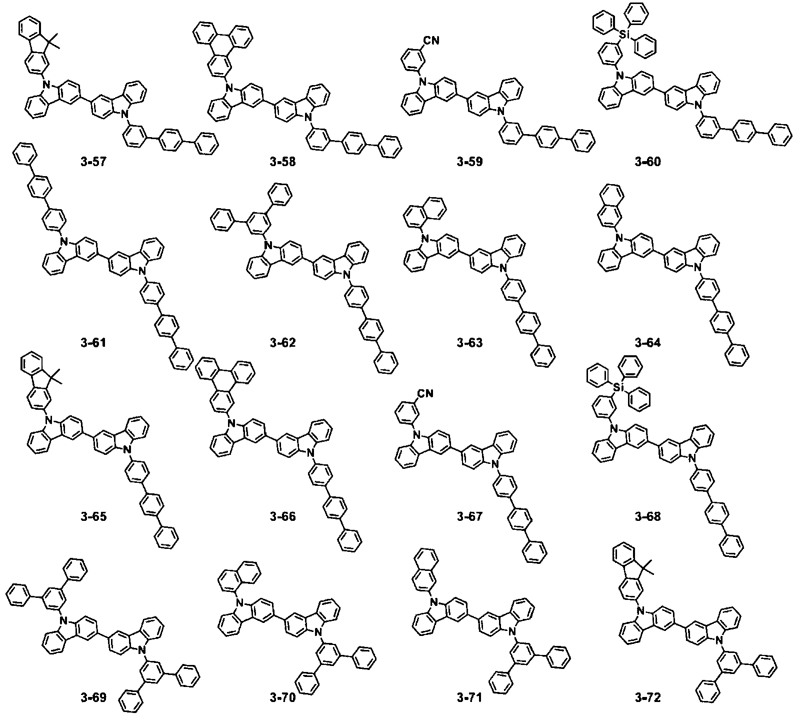

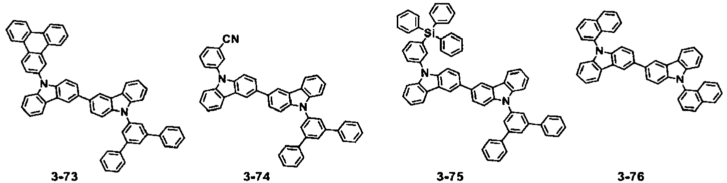

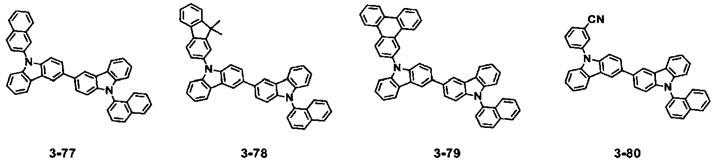

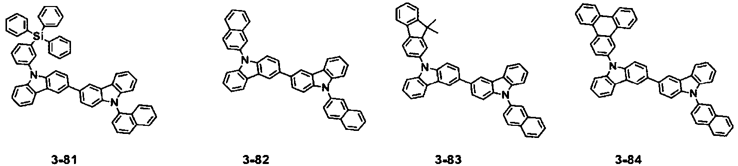

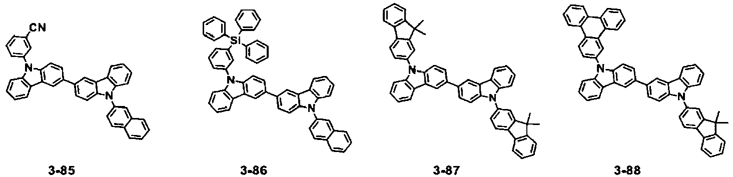

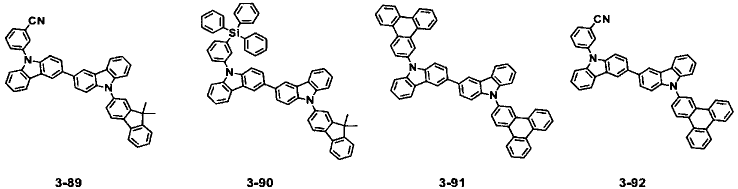

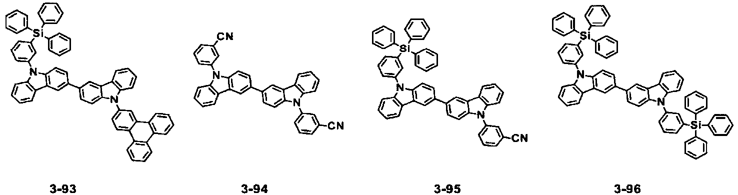

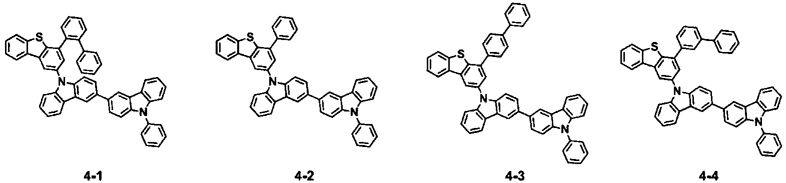

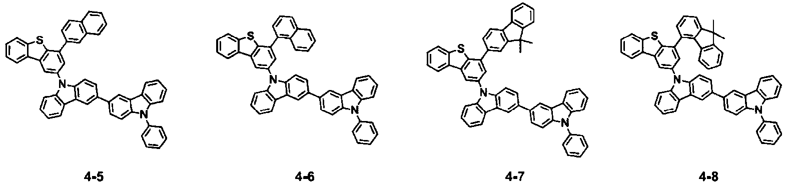

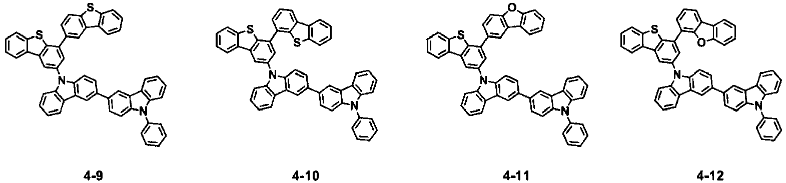

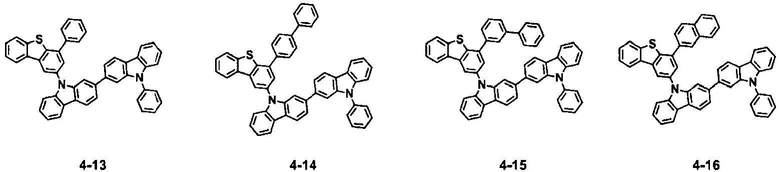

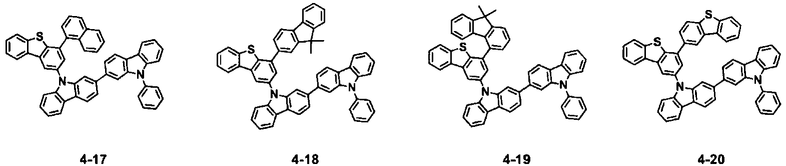

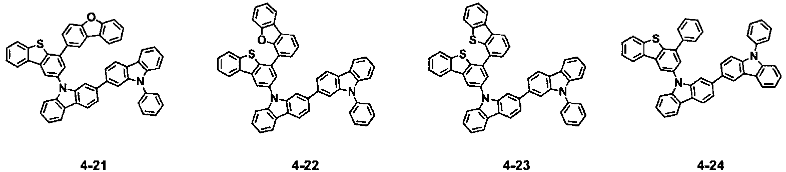

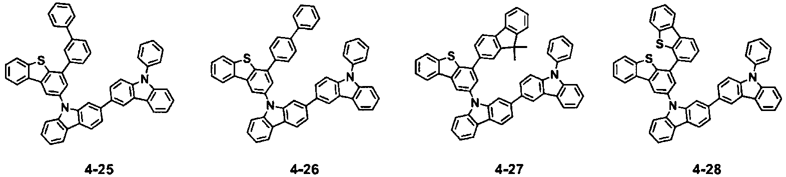

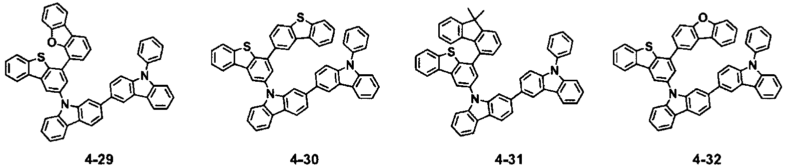

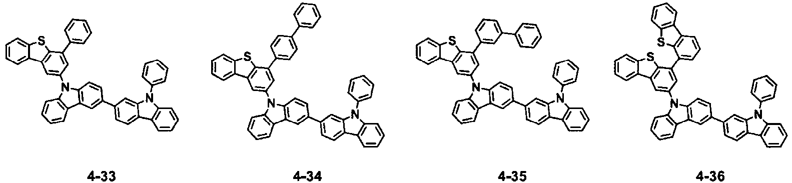

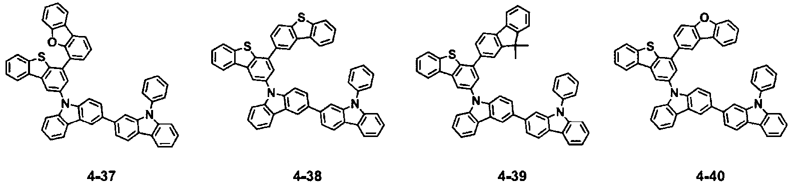

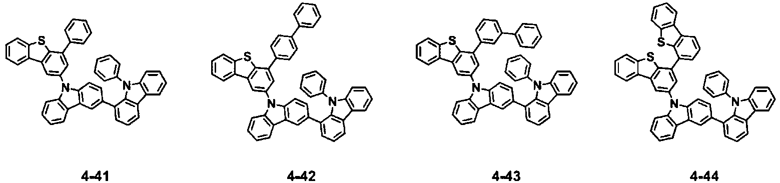

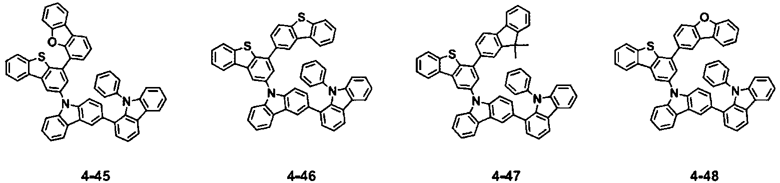

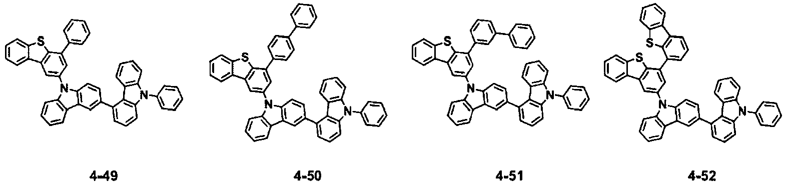

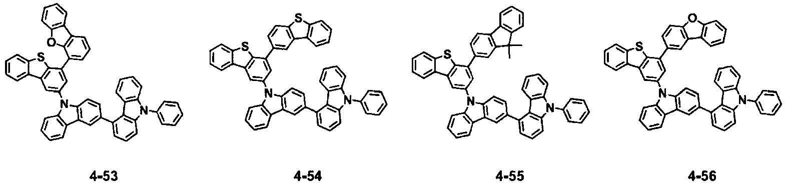

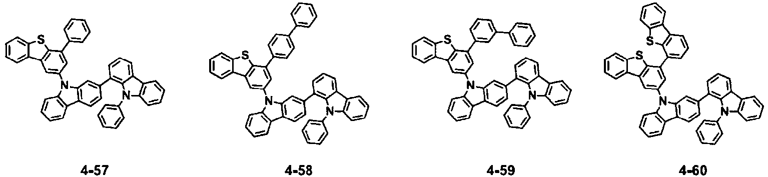

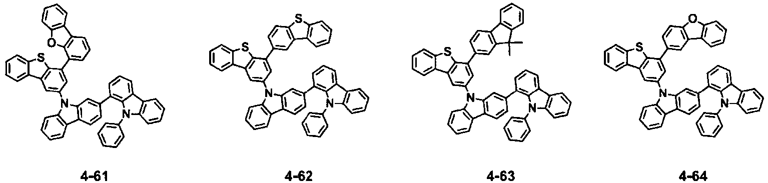

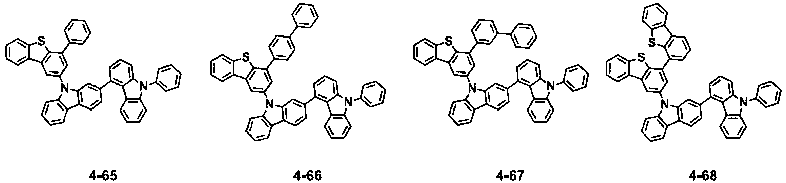

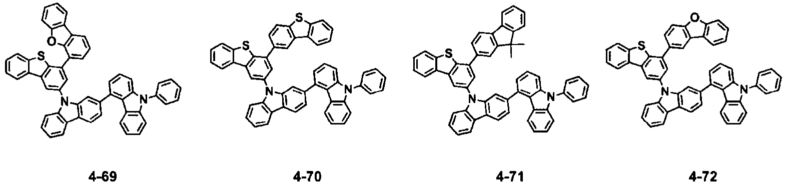

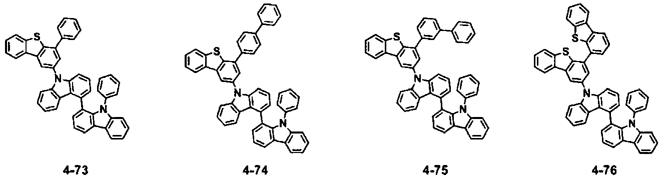

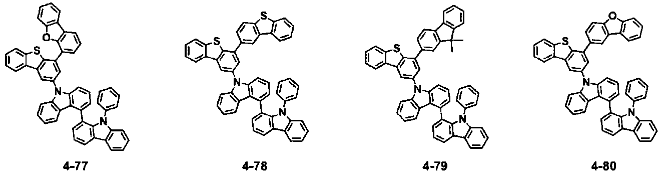

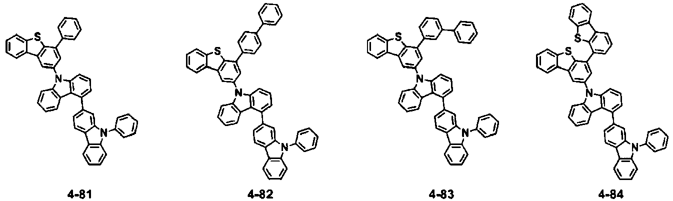

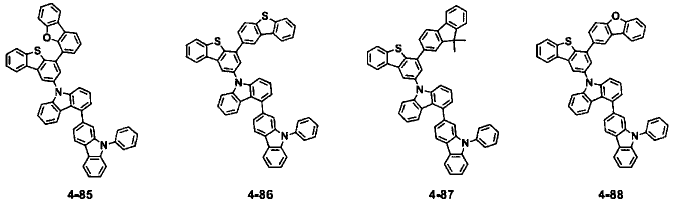

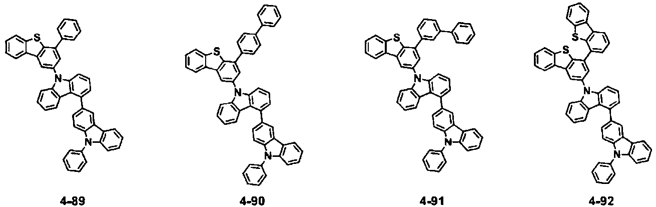

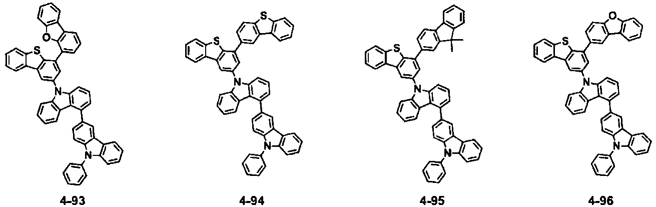

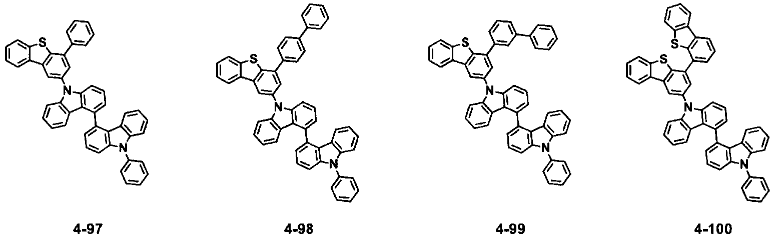

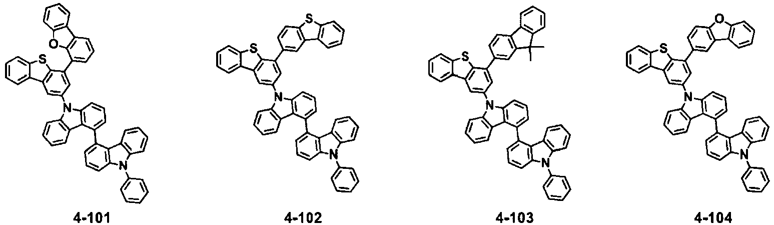

9. Zusammensetzung zum Bilden einer organischen Materialschicht, wobei die Zusammensetzung umfasst:
die heterocyclische Verbindung nach Anspruch 1; und
eine Verbindung der folgenden chemischen Formel 3: 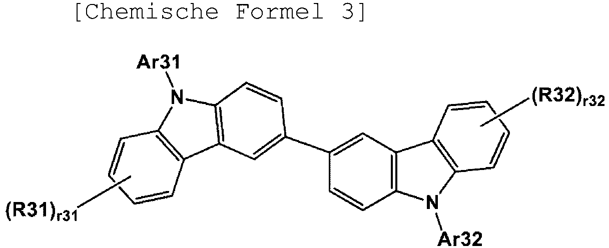
wobei in der chemischen Formel 3
R31 und R32 jeweils unabhängig Wasserstoff; Deuterium; eine Halogengruppe; eine substituierte oder unsubstituierte C1 - bis C60 -Alkylgruppe; eine substituierte oder unsubstituierte C3 - bis C60 -Cycloalkylgruppe; eine substituierte oder unsubstituierte C6 - bis C60 -Arylgruppe; oder eine substituierte oder unsubstituierte C2 - bis C60 - Heteroarylgruppe sind;
Ar31 und Ar32 jeweils unabhängig eine substituierte oder unsubstituierte C6 - bis C60 -Arylgruppe; oder eine substituierte oder unsubstituierte C2 - bis C60 - Heteroarylgruppe sind;
r31 eine ganze Zahl von 0 bis 4 ist, und wenn 2 oder größer, R31s gleich oder verschieden voneinander sind; und
r32 eine ganze Zahl von 0 bis 4 ist, und wenn 2 oder größer, R32s gleich oder verschieden voneinander sind.

10. Zusammensetzung zum Bilden einer organischen Materialschicht nach Anspruch 9, wobei die heterocyclische Verbindung und die Verbindung der chemischen Formel 3 ein Gewichtsverhältnis von 1:10 bis 10:1 aufweisen.

## Revendications

1. Composé hétérocyclique de la Formule chimique 1 ci-après : 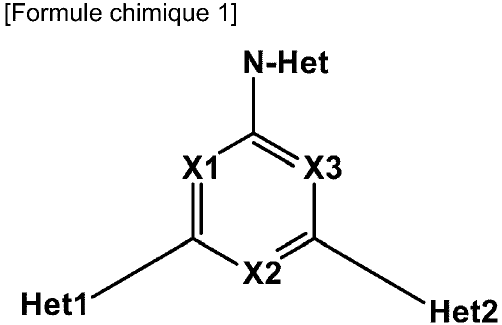 dans la Formule chimique 1,
X1 à X3 sont N,
N-Het est représenté par la Formule chimique N-1 ou N-2 ci-après, 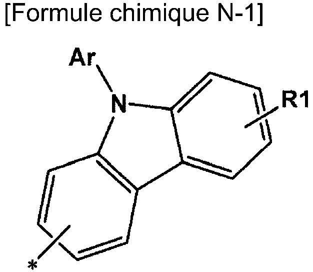 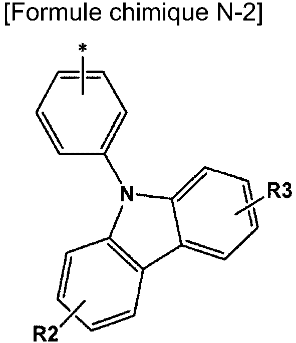
dans les Formules chimiques N-1 et N-2,
R1 est un hydrogène ; un deutérium ; un groupe halogène ; un groupe cyano ; un groupe silyle non substitué ou substitué ; un groupe oxyde de phosphine non substitué ou substitué ; un groupe alkyle en C1 à C60 non substitué ou substitué ; un groupe cycloalkyle en C3 à C60 non substitué ou substitué ; un groupe aryle bicyclique ou inférieur non substitué ou substitué ; un groupe terphényle non substitué ou substitué ; un groupe fluorényle non substitué ou substitué ; un groupe aryle tétracyclique ou supérieur non substitué ou substitué ; ou un groupe hétéroaryle en C2 à C60 non substitué ou substitué,
R2 et R3 sont chacun indépendamment un hydrogène ; un deutérium ; un groupe phényle non substitué ou substitué ; un groupe biphényle non substitué ou substitué ; ou un groupe terphényle non substitué ou substitué,
Ar est un groupe aryle en C6 à C60 non substitué ou substitué ; ou un groupe hétéroaryle en C2 à C60 non substitué ou substitué, et
Het1 est représenté par la Formule chimique H-1 ci-après et Het2 est représenté par la Formule chimique H-2 ci-après, 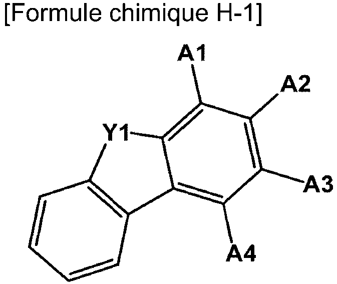 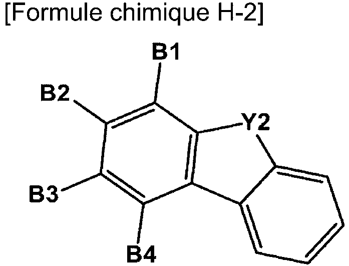
dans les Formules chimiques H-1 et H-2,
Y1 et Y2 sont chacun indépendamment O ou S,
A1 à A4 et B1 à B4 se lient chacun indépendamment à la Formule chimique 1, ou sont un hydrogène ; ou un deutérium, et
la Formule chimique 1 se lie à l'un quelconque de A1 à A4 de la Formule chimique H-1 et l'un quelconque de B1 à B4 de la Formule chimique H-2, qui est représenté par Am-Bn, m et n sont chacun 1, 2, 3 ou 4, et m et n sont différents.

2. Composé hétérocyclique selon la revendication 1, dans lequel Ar est un groupe phényle non substitué ou substitué ; un groupe biphényle non substitué ou substitué ; un groupe terphényle non substitué ou substitué ; un groupe naphtyle non substitué ou substitué ; un groupe phénanthrényle non substitué ou substitué ; un groupe triphénylényle non substitué ou substitué ; ou un groupe pyrényle non substitué ou substitué.

3. Composé hétérocyclique selon la revendication 1, dans lequel la Formule chimique 1 est représentée par l'un quelconque des composés suivants :

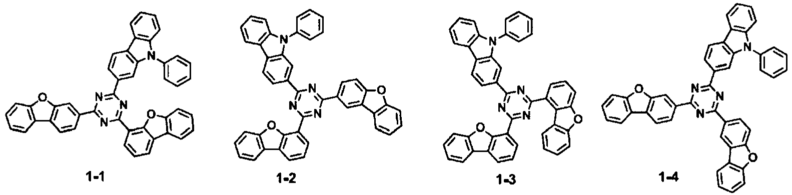

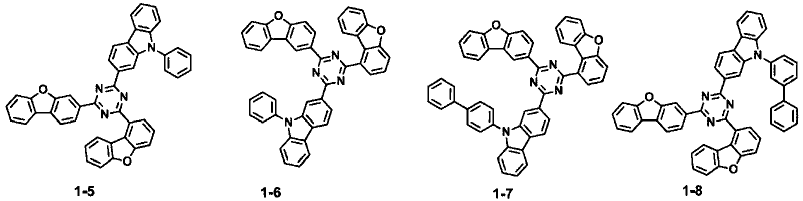

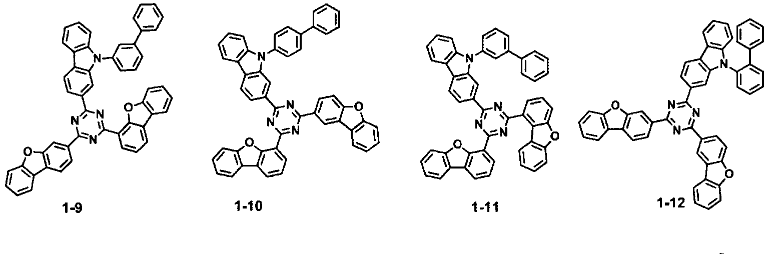

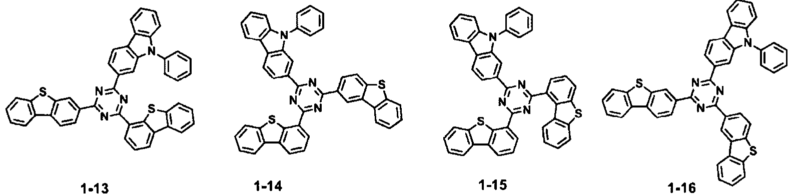

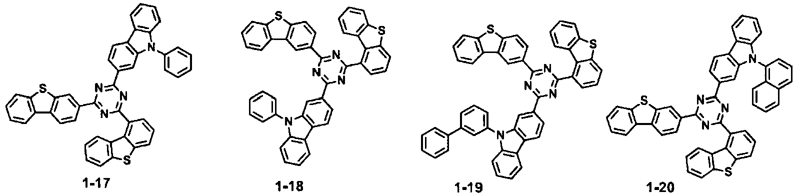

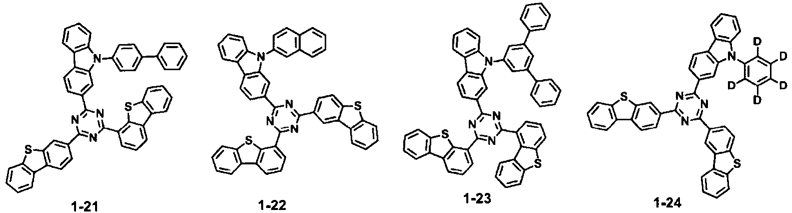

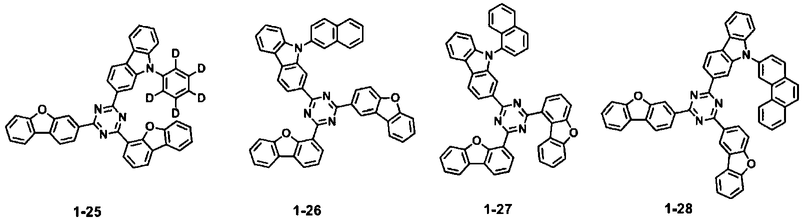

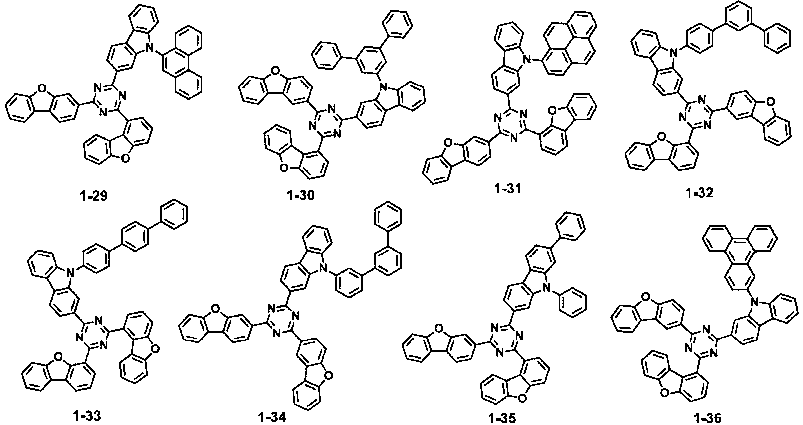

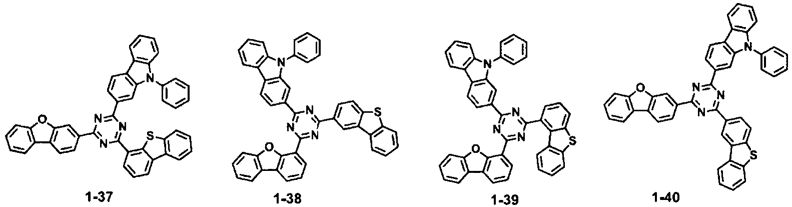

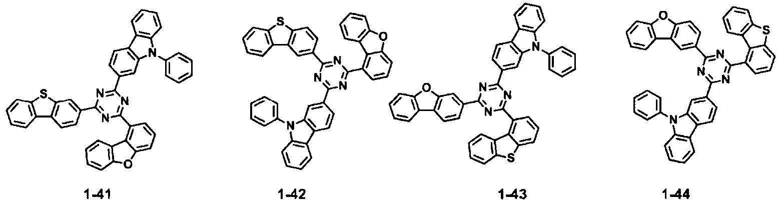

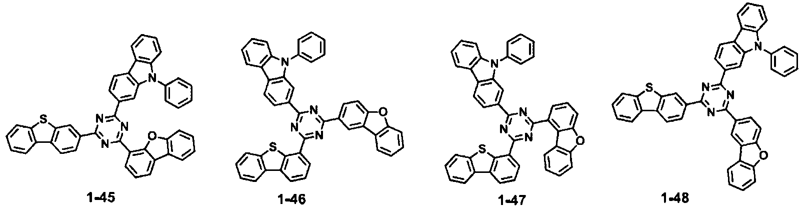

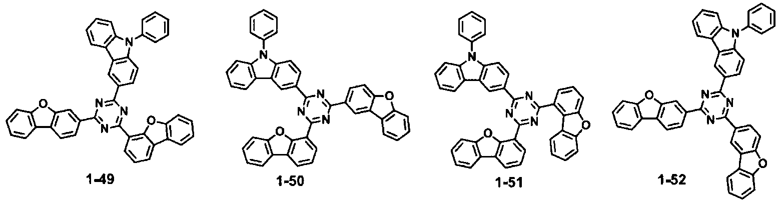

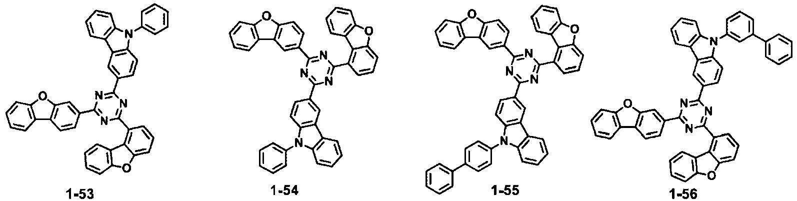

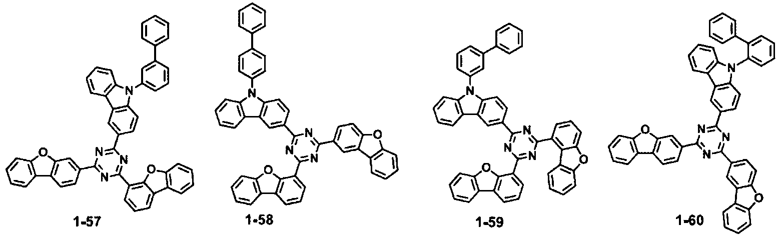

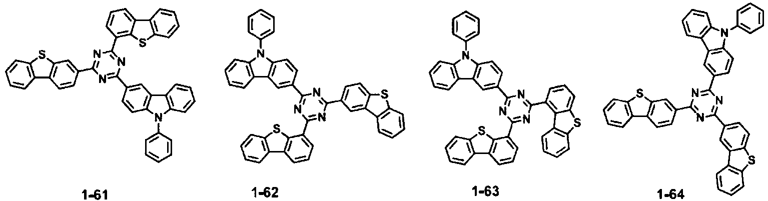

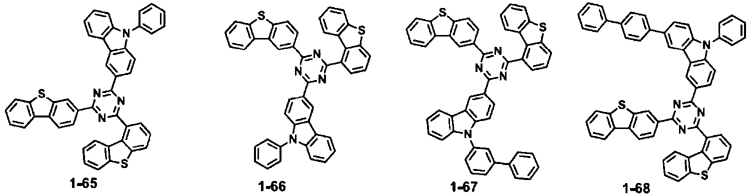

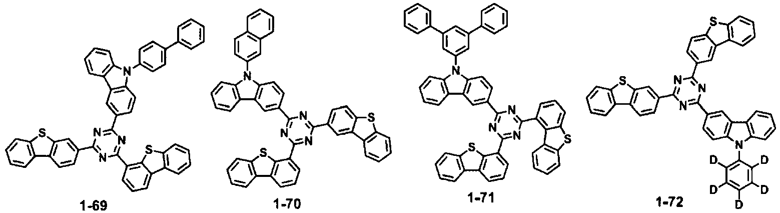

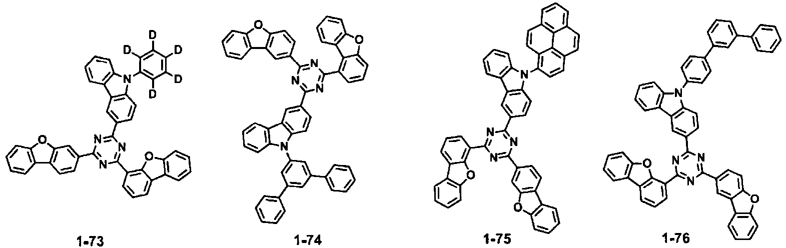

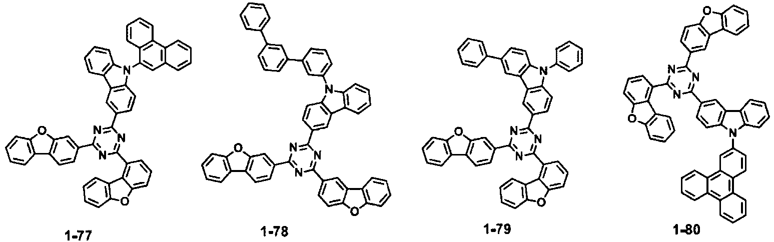

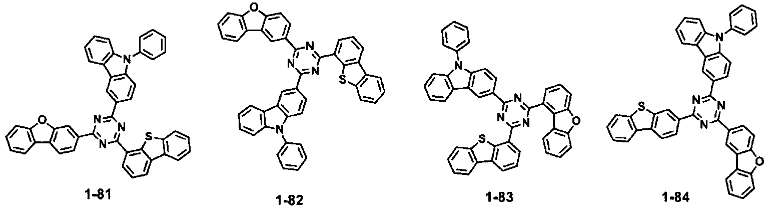

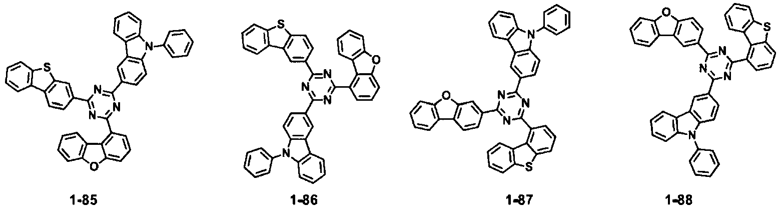

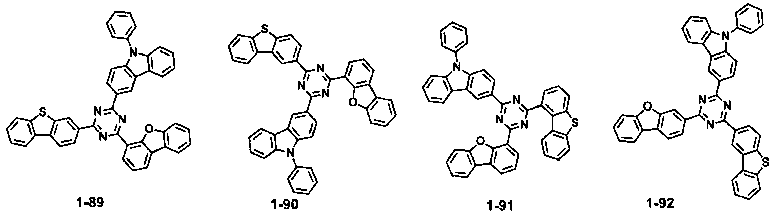

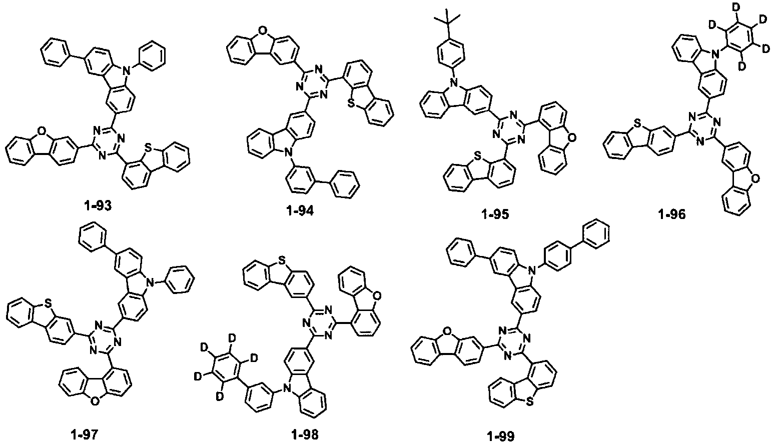

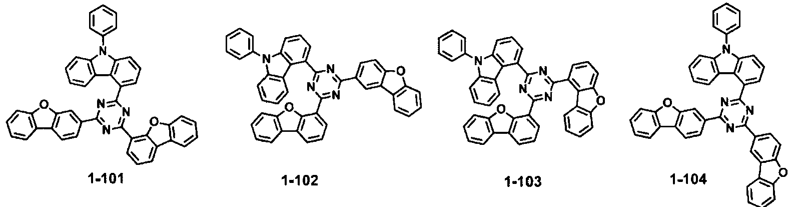

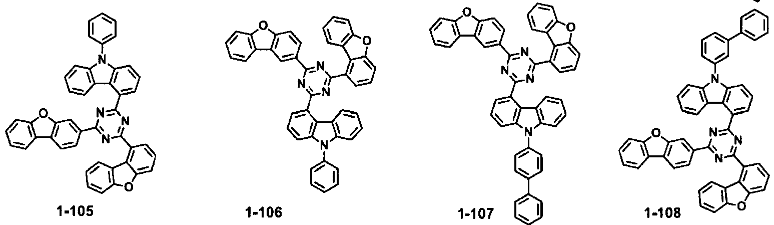

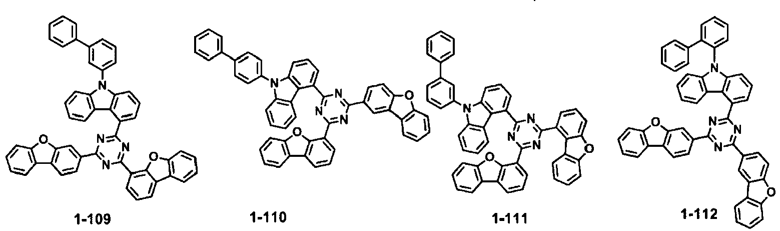

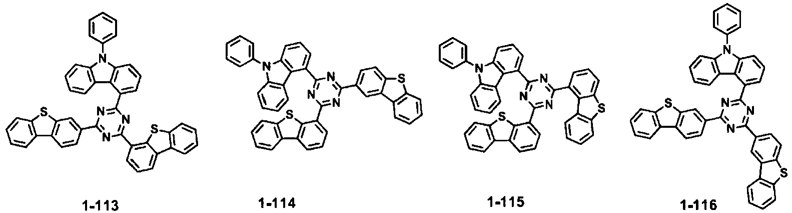

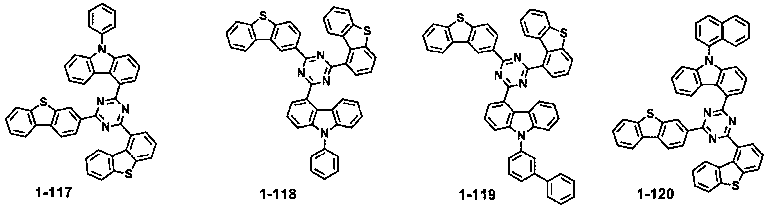

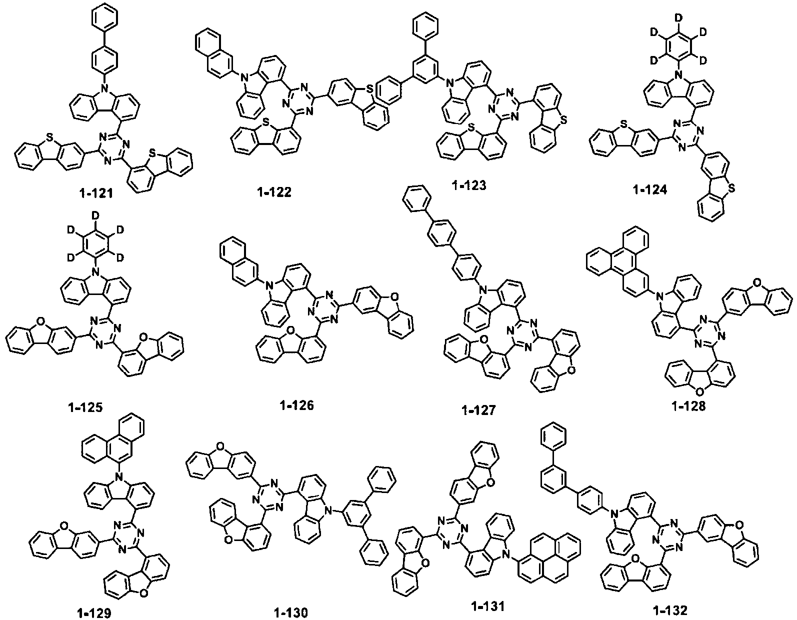

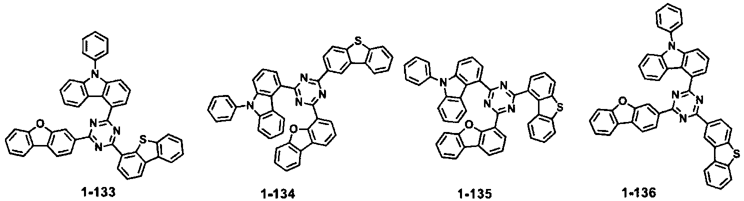

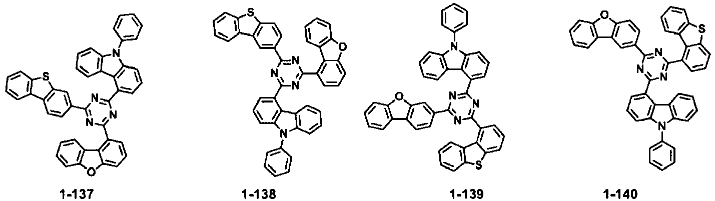

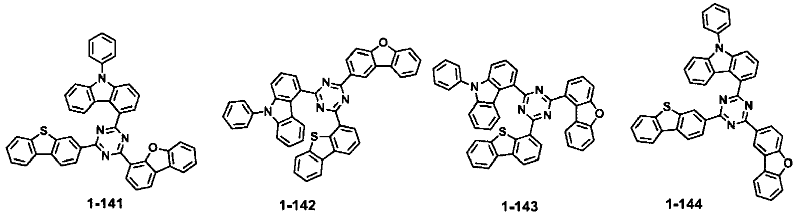

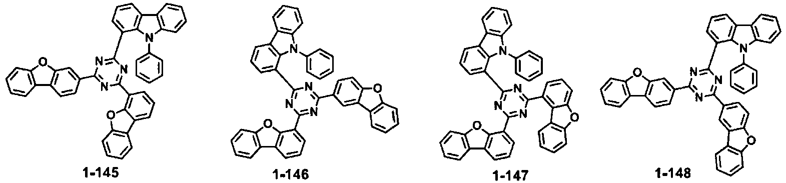

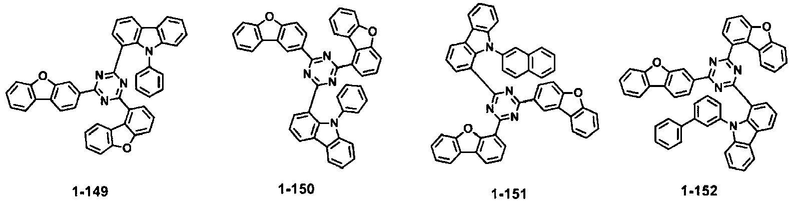

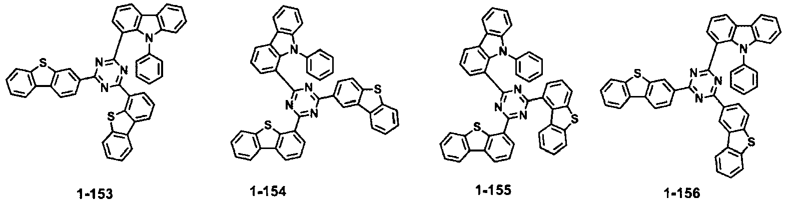

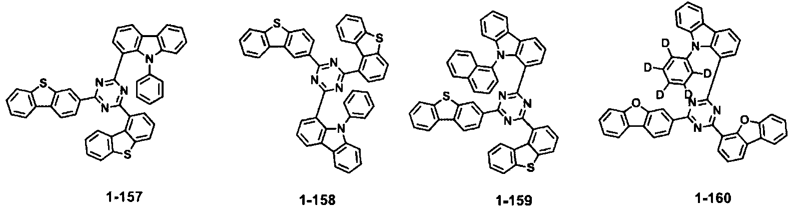

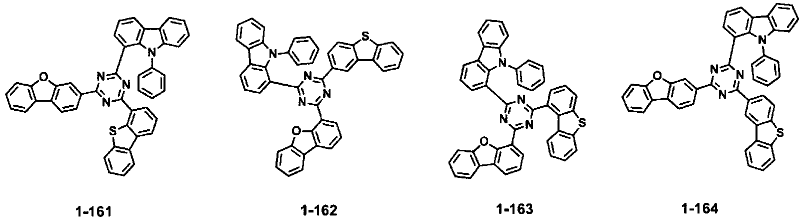

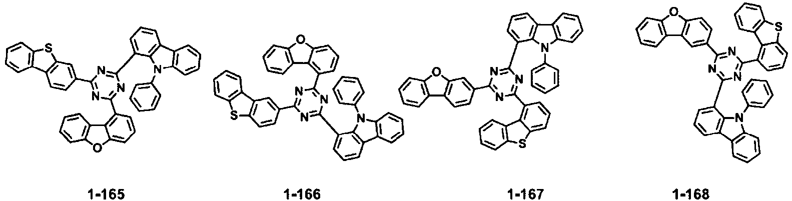

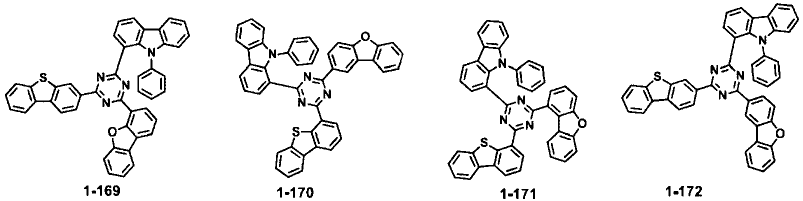

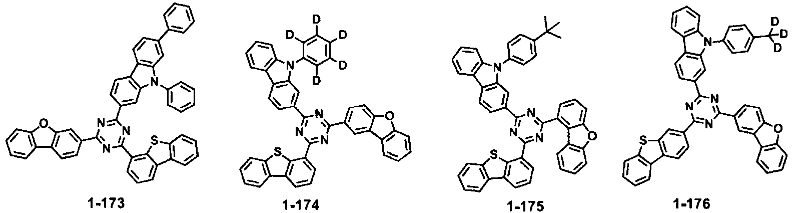

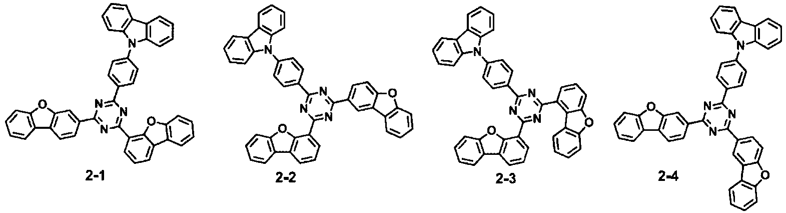

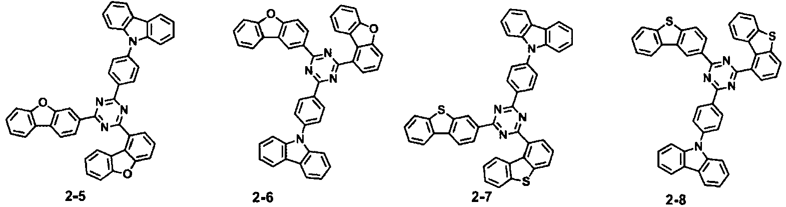

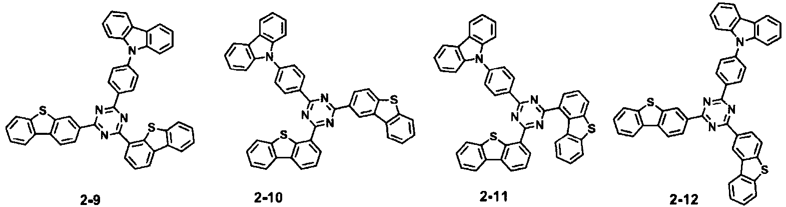

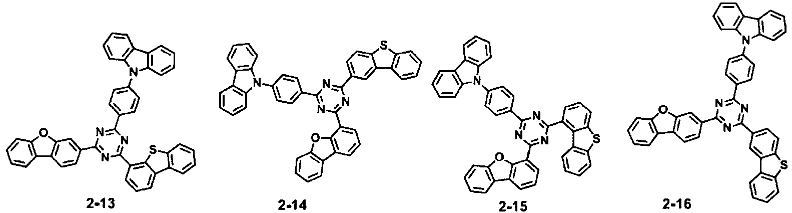

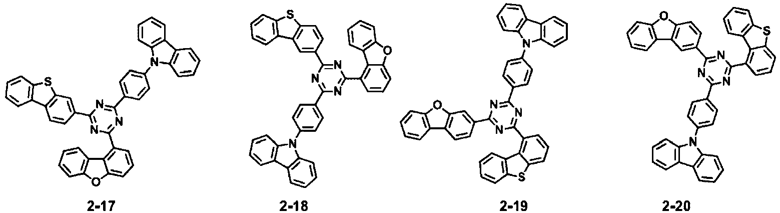

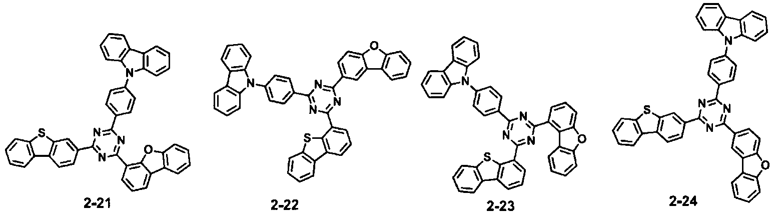

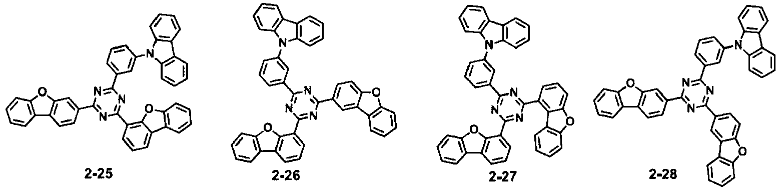

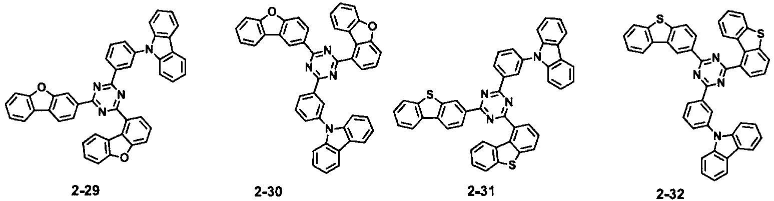

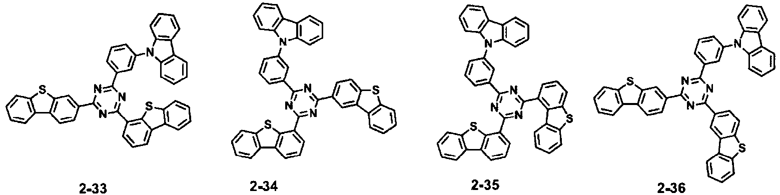

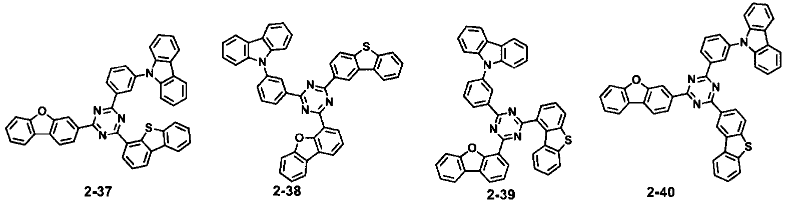

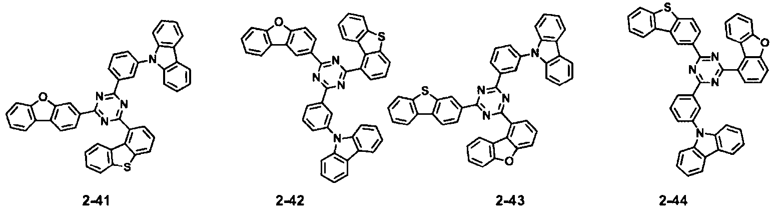

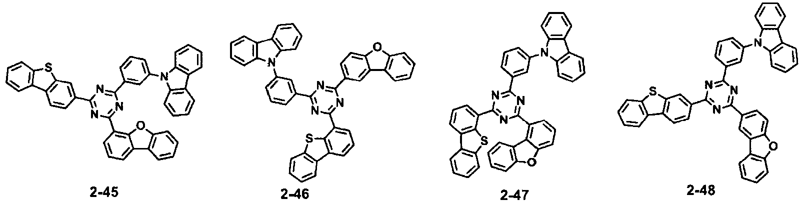

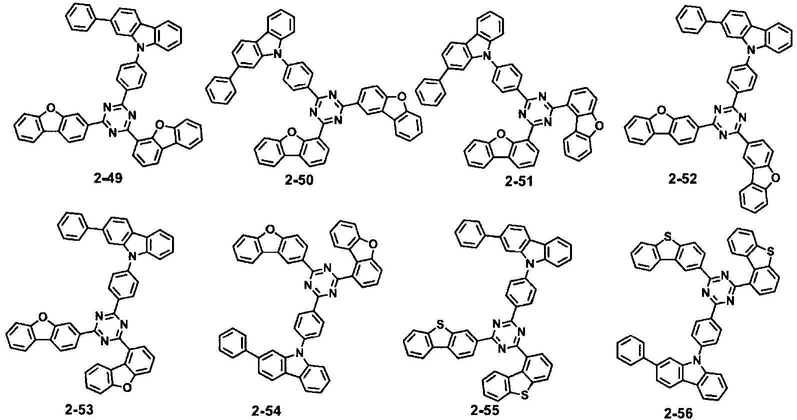

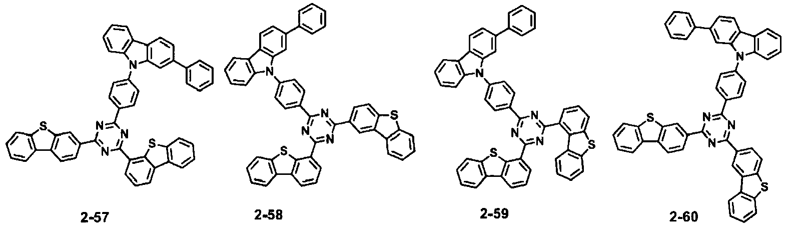

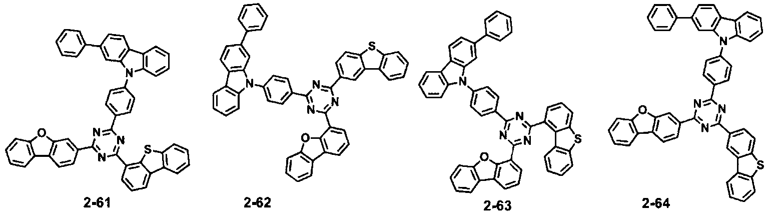

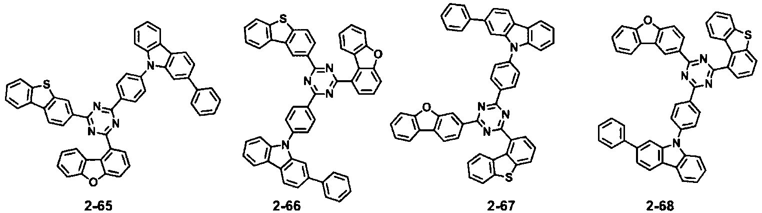

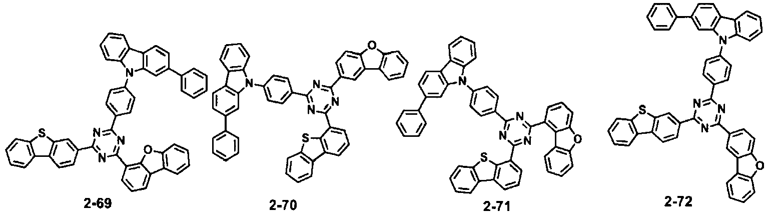

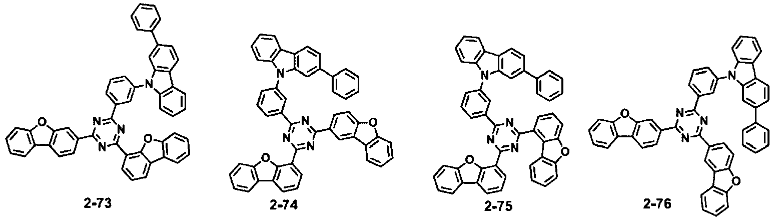

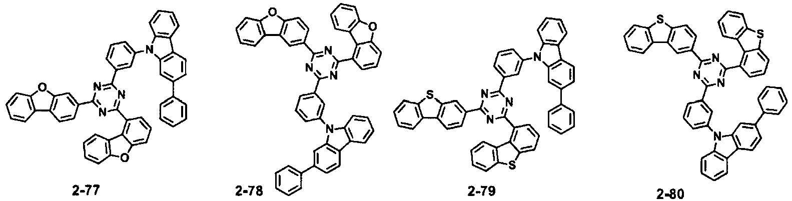

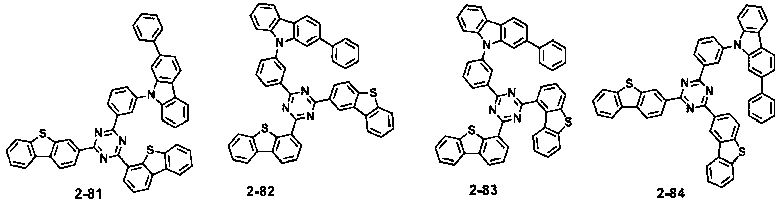

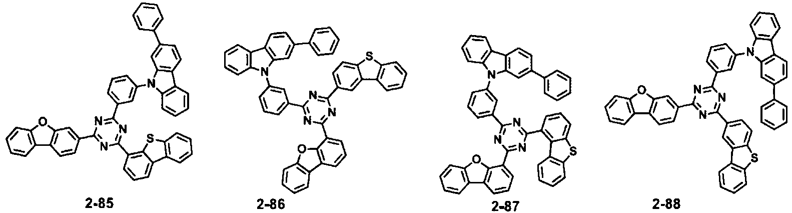

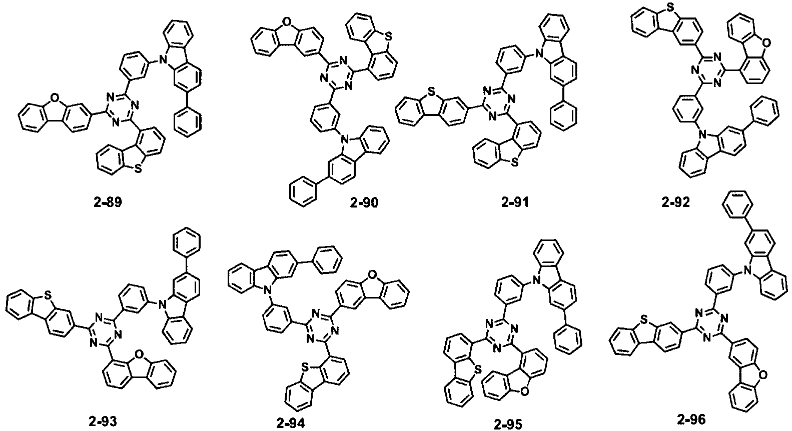

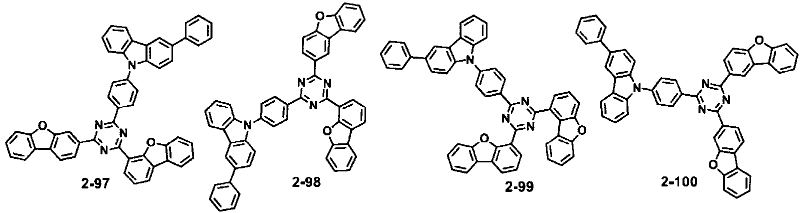

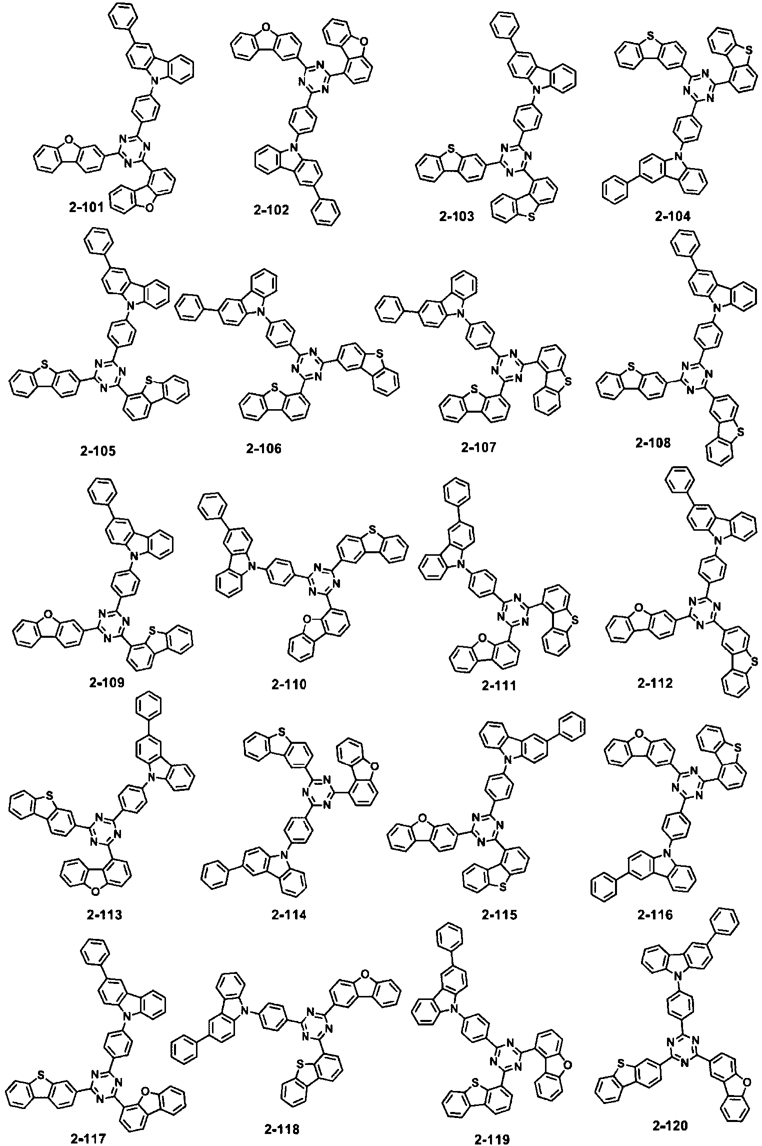

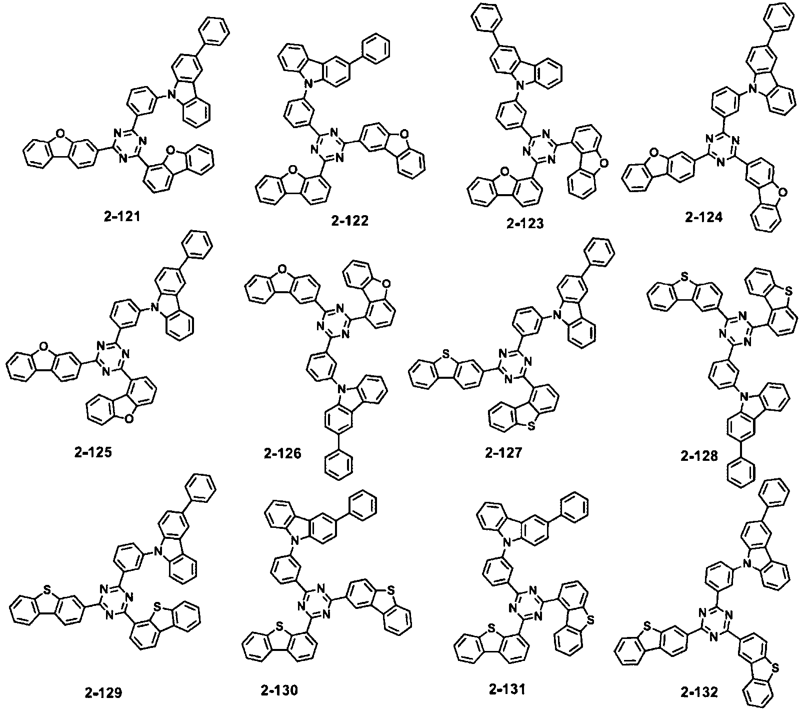

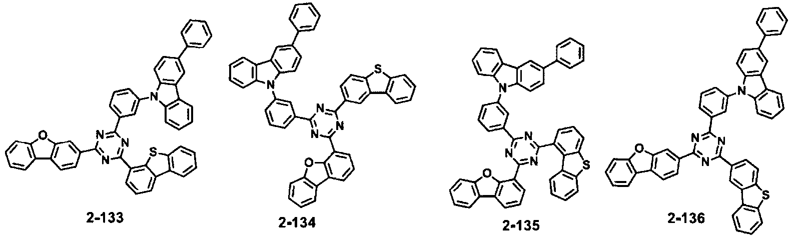

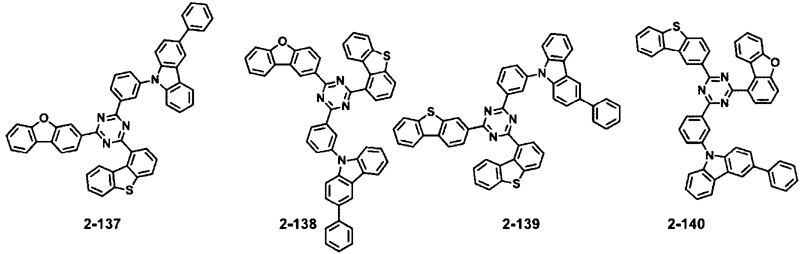

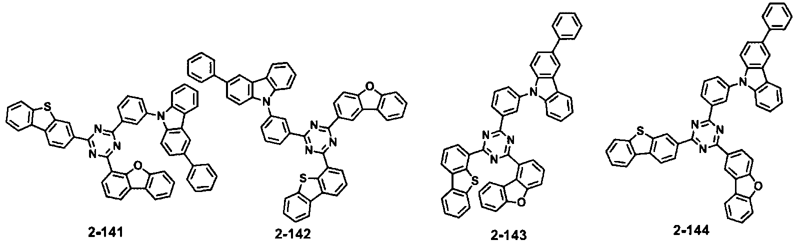

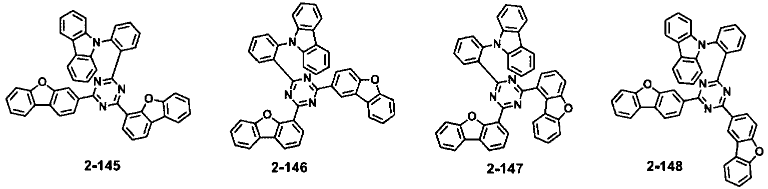

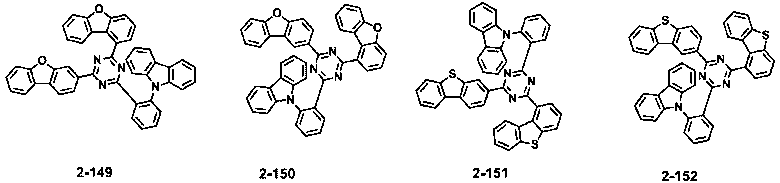

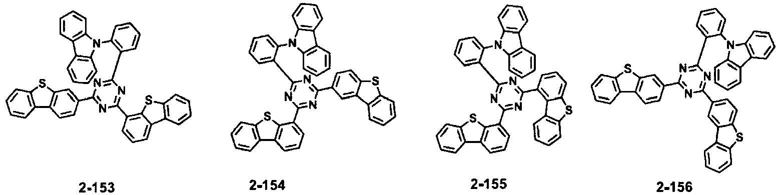

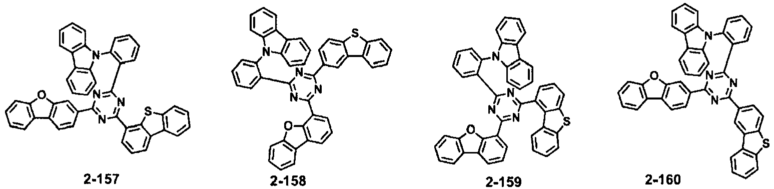

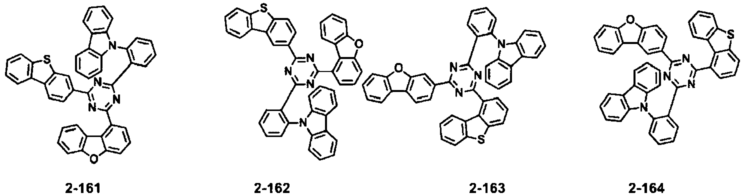

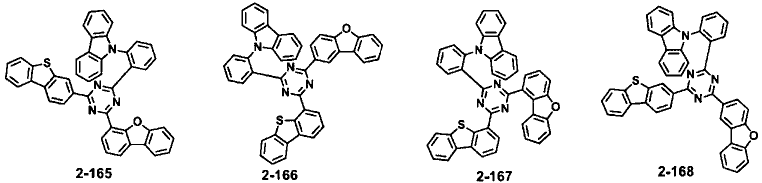

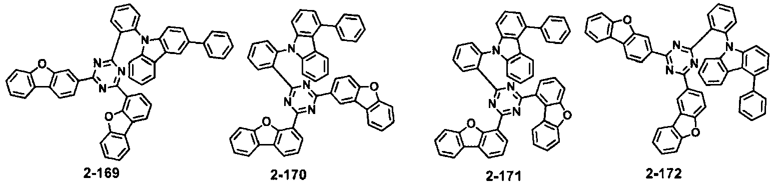

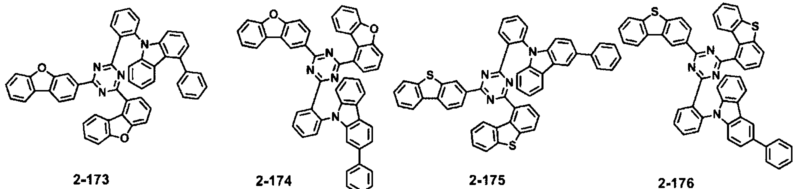

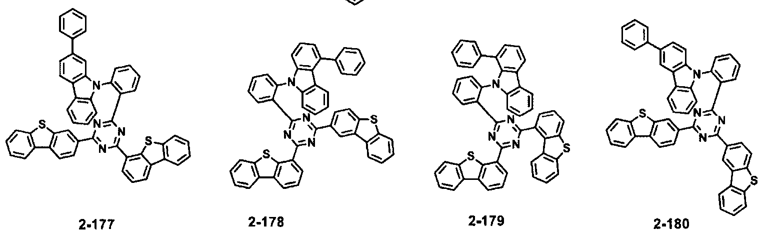

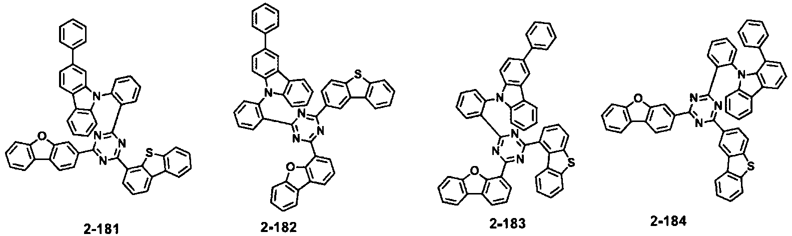

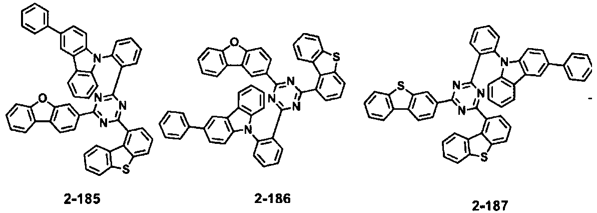

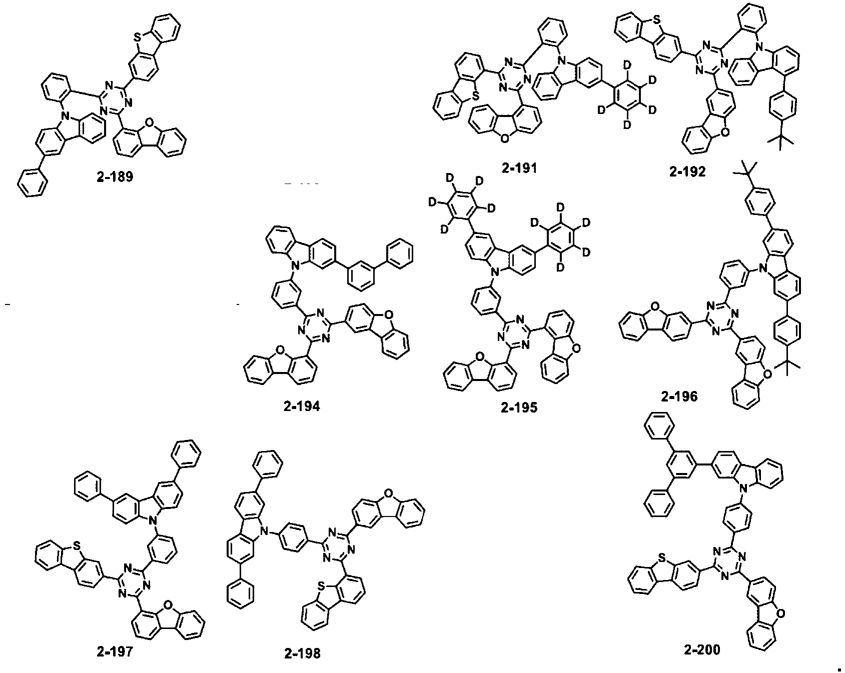

4. Dispositif électroluminescent organique comprenant :
une première électrode ;
une deuxième électrode ; et
une couche de matériau organique fournie entre la première électrode et la deuxième électrode,
dans lequel la couche de matériau organique inclut le composé hétérocyclique selon l'une quelconque des revendications 1 à 3.

5. Dispositif électroluminescent organique selon la revendication 4, dans lequel la couche de matériau organique inclut une couche électroluminescente, et la couche électroluminescente inclut le composé hétérocyclique.

6. Dispositif électroluminescent organique selon la revendication 4, dans lequel la couche de matériau organique inclut une couche électroluminescente, la couche électroluminescente inclut un hôte, et l'hôte inclut le composé hétérocyclique.

7. Dispositif électroluminescent organique selon la revendication 5, dans lequel la couche électroluminescente inclut en outre un composé de la Formule chimique 3 ci-après : 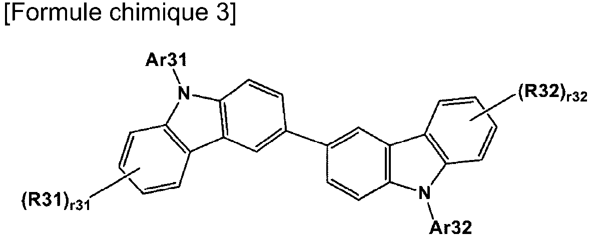 dans la Formule chimique 3,
R31 et R32 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué,
Ar31 et Ar32 sont chacun indépendamment un groupe aryle en C6 à C60 non substitué ou substitué ; ou un groupe hétéroaryle en C2 à C60 non substitué ou substitué,
r31 est un nombre entier de 0 à 4 et, quand il est 2 ou plus, les R31 sont identiques ou différents les uns des autres, et
r32 est un nombre entier de 0 à 4 et, quand il est 2 ou plus, les R32 sont identiques ou différents les uns des autres.

8. Dispositif électroluminescent organique selon la revendication 9, dans lequel la Formule chimique 3 est représentée par l'un quelconque des composés suivants :

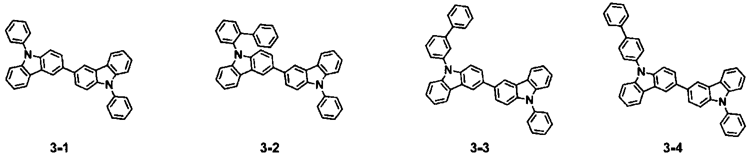

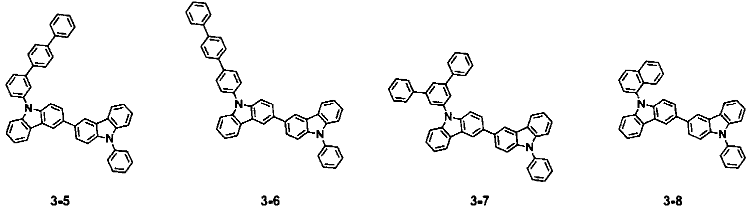

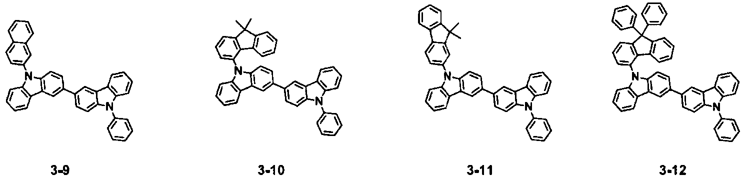

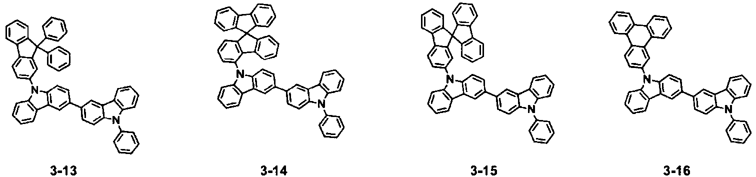

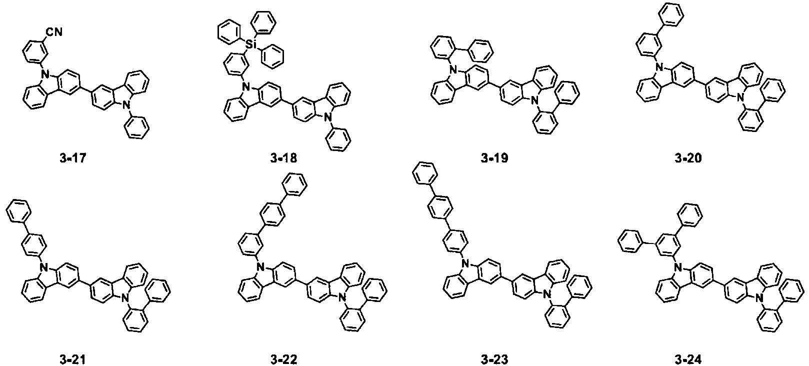

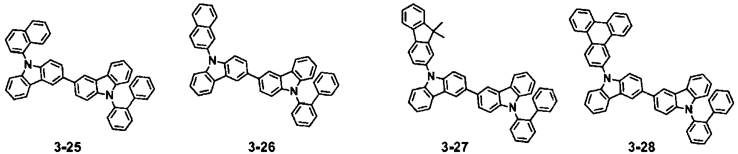

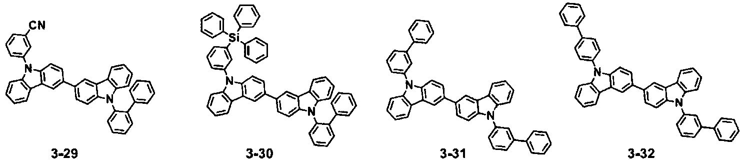

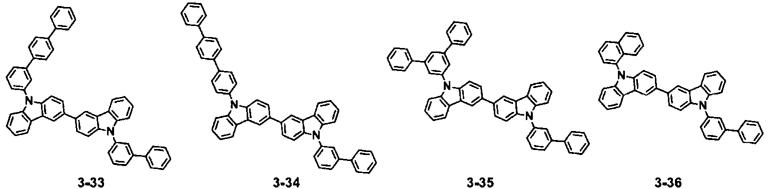

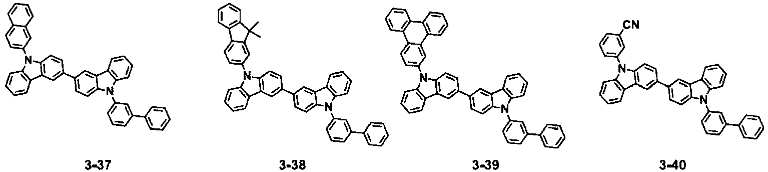

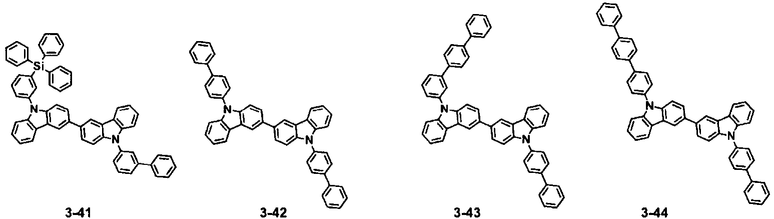

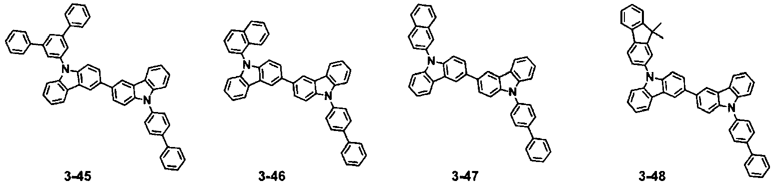

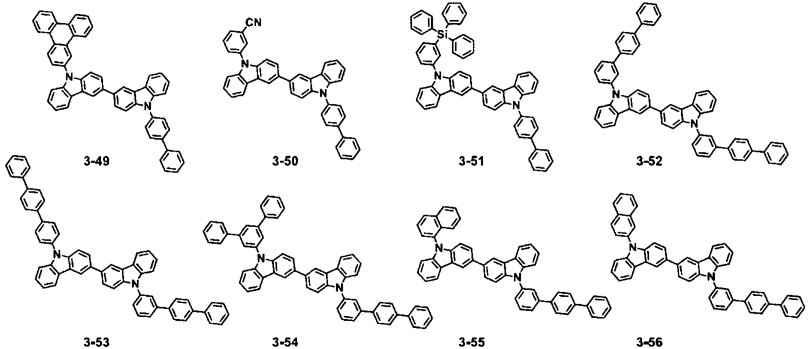

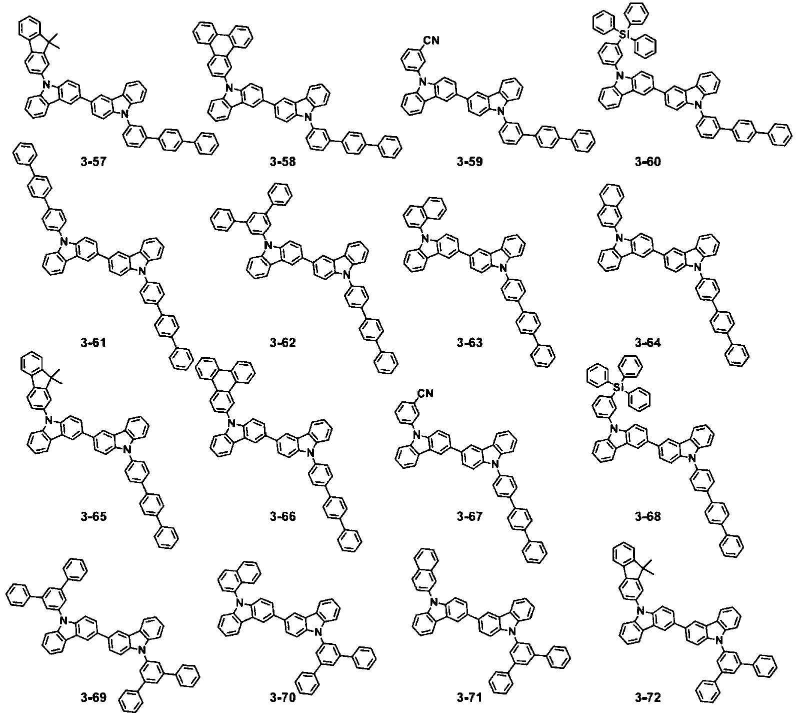

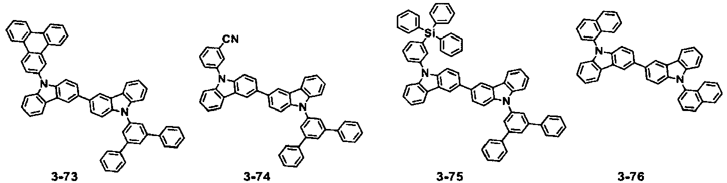

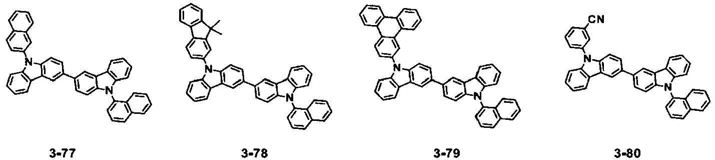

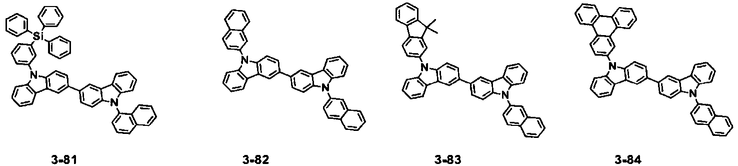

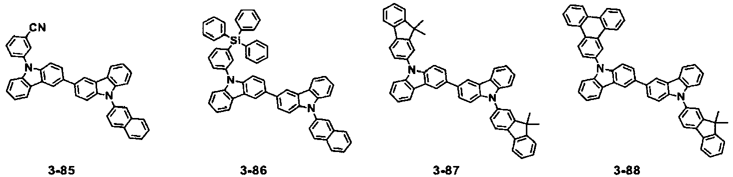

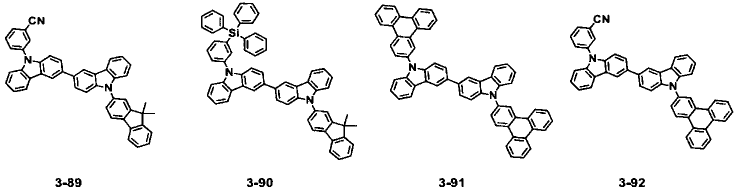

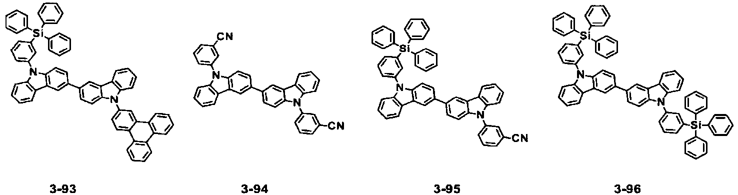

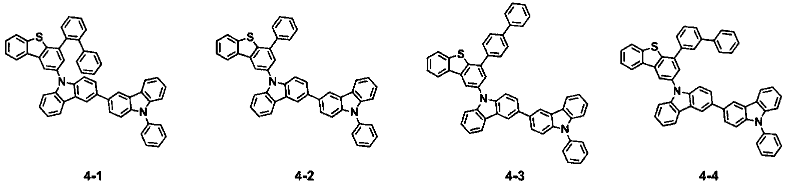

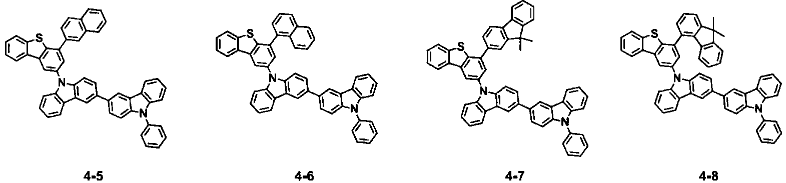

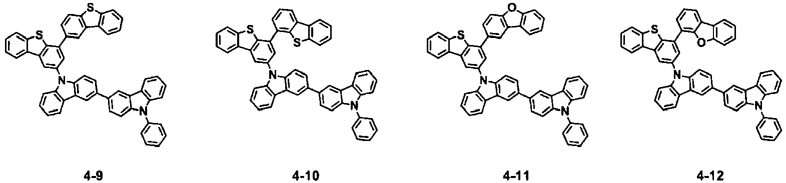

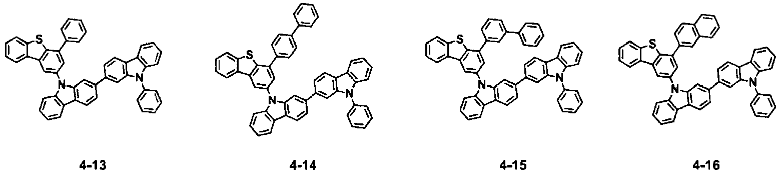

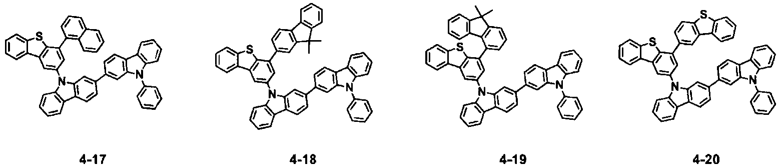

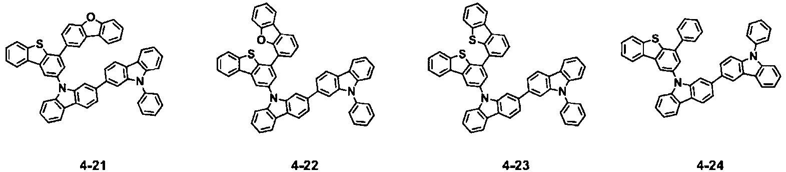

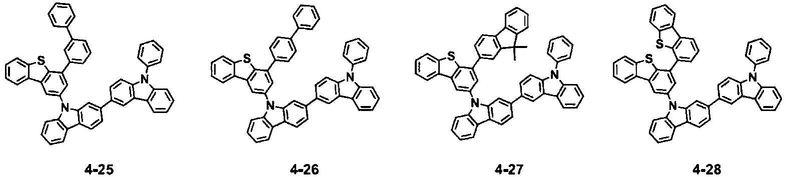

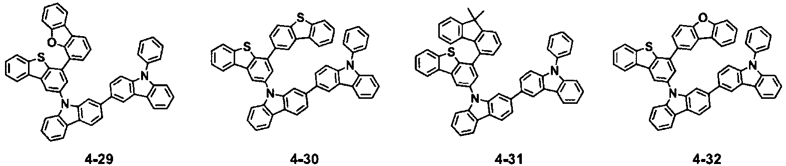

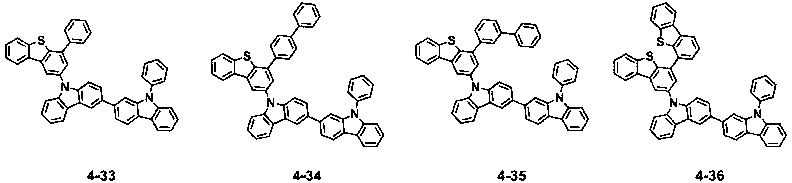

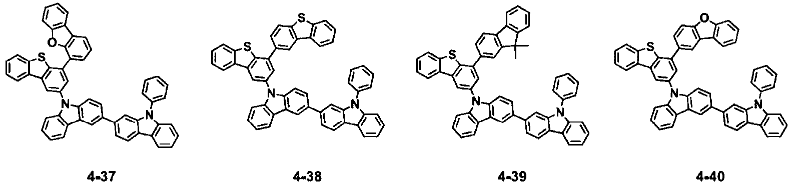

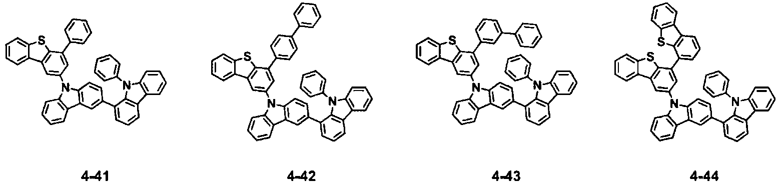

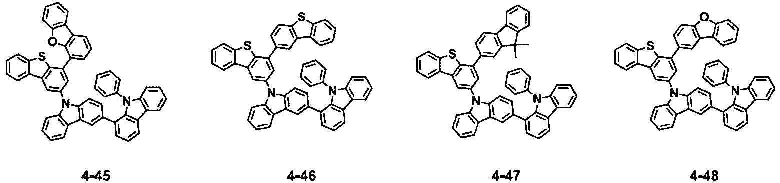

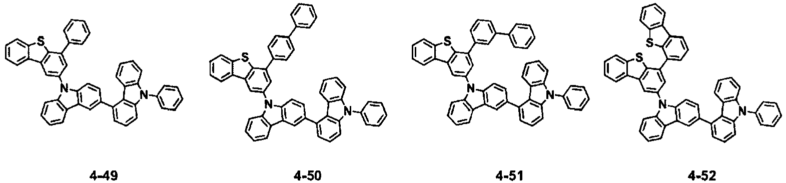

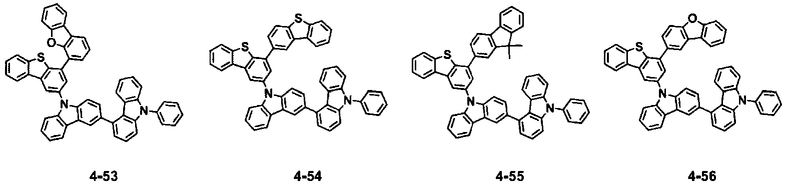

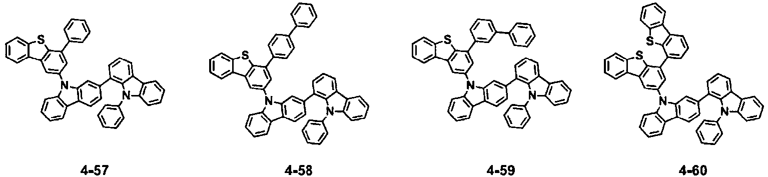

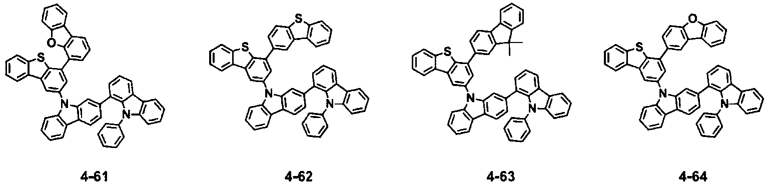

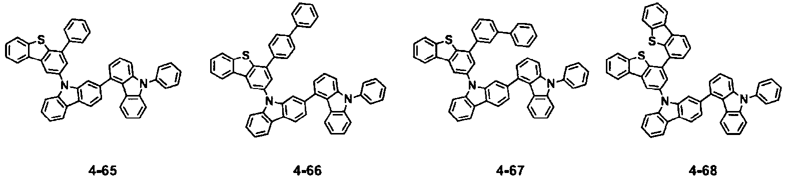

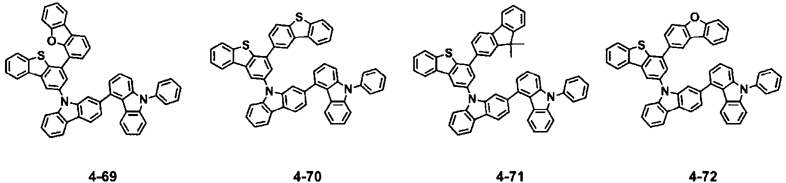

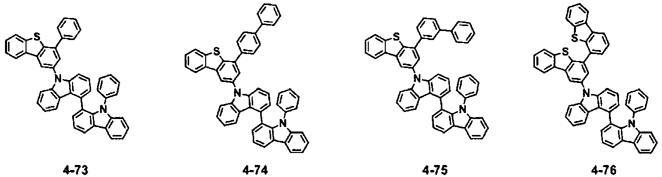

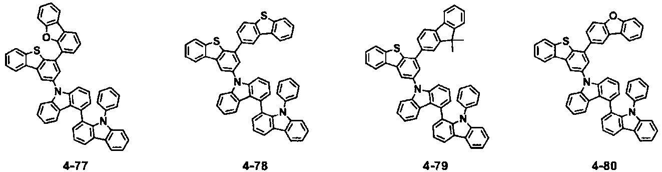

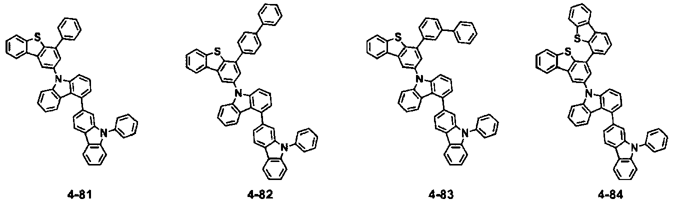

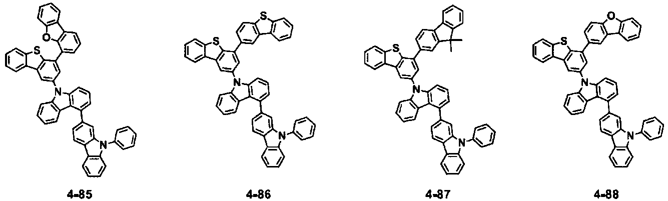

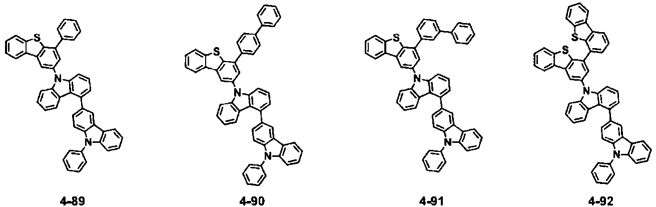

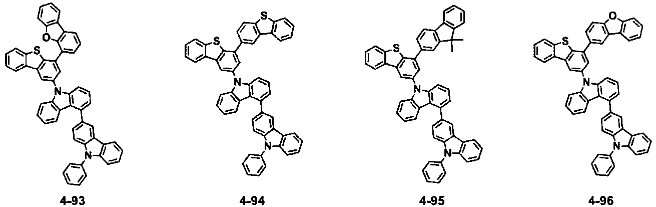

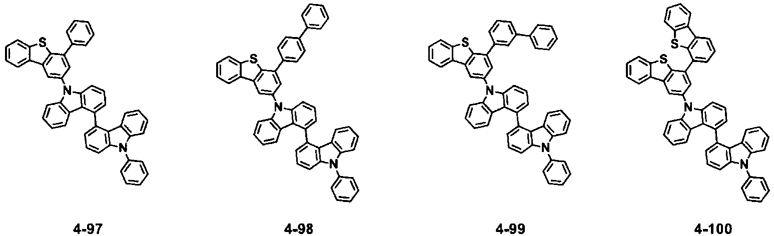

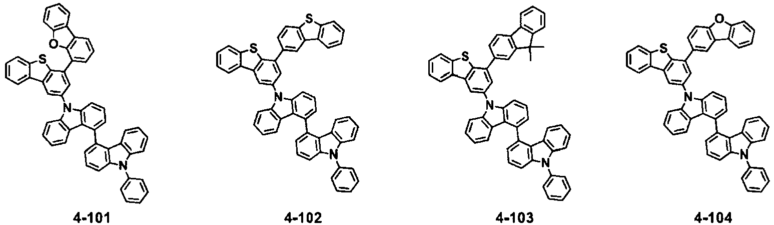

9. Composition pour former une couche de matériau organique, la composition comprenant :
un composé hétérocyclique selon la revendication 1 ; et
un composé de la Formule chimique 3 ci-après : 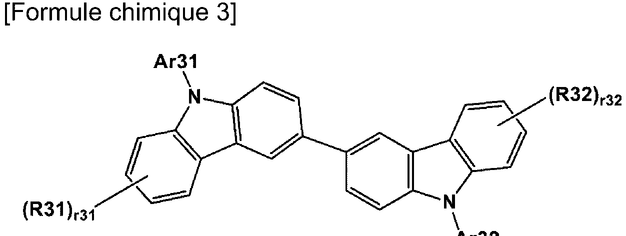
dans la Formule chimique 3,
R31 et R32 sont chacun indépendamment un hydrogène ; un deutérium ; un groupe halogène ; un groupe alkyle en C1 à C60 non substitué ou substitué ; un groupe cycloalkyle en C3 à C60 non substitué ou substitué ; un groupe aryle en C6 à C60 non substitué ou substitué ; ou un groupe hétéroaryle en C2 à C60 non substitué ou substitué ;
Ar31 et Ar32 sont chacun indépendamment un groupe aryle en C6 à C60 non substitué ou substitué ; ou un groupe hétéroaryle en C2 à C60 non substitué ou substitué ;
r31 est un nombre entier de 0 à 4 et, quand il est 2 ou plus, les R31 sont identiques ou différents les uns des autres ; et
r32 est un nombre entier de 0 à 4 et, quand il est 2 ou plus, les R32 sont identiques ou différents les uns des autres.

10. Composition pour former une couche de matériau organique selon la revendication 9, dans laquelle le composé hétérocyclique et le composé de la Formule chimique 3 ont un rapport en poids de 1:10 à 10:1.
